# EUROPEAN PATENT APPLICATION

(11) **EP 2 407 534 A1**
(43) Date of publication of application: **18.01.2012**
(21) Application number: 10382192.2
(22) Date of filing: 14.07.2010
(51) Int. Cl.: C12N 7/04

(54) **Methods and reagents for obtaining transcriptionally active virus-like particles and recombinant virions**

(71) Applicant: Neo Virnatech, S.L., 08110 Barcelona (ES)
(72) Inventor: Oña Blanco, Ana María, 28037 Madrid (ES)
(74) Representative: ABG Patentes, S.L.

(57) **Abstract**

The invention relates to a method for obtaining transcriptionally active virus-like particles, i.e., particles comprising a genetic material of interest and the polymerase necessary for transcribing said gene in the target cell. The invention also relates to a method for obtaining recombinant virions comprising additional genomic segments and wherein said additional segments comprise the sequence of a gene of interest. Finally, the invention relates to the virus-like particles and to the recombinant virions obtained using the methods described in the present invention as well as to the use in medicine of said virus-like particles and said recombinant virions.

SEQUENCE LISTING

<110>  Neo VirnaTech S.L.

<120>  MÉTODOS Y REACTIVOS PARA LA OBTENCIÓN DE PARTÍCULAS PSEUDOVIRALES
        TRANSCRIPCIONALEMTE ACTIVAS Y VIRIONES RECOMBINANTES

<130>  P3924EP00

<160>  26

<170>  PatentIn version 3.5

<210>  1
<211>  96
<212>  DNA
<213>  IBDV

<400>  1
ggatacgatc ggtctgaccc cgggggagtc acccggggac aggtcttcaa ggccttgttc      60

caggatggaa ctcctccttc tacaacgcta tcattg                                96


<210>  2
<211>  92
<212>  DNA
<213>  IBDV

<400>  2
ggctcctggg agtctcccga caccacccgc gcaggtgtgg acaccaattc ggccttacaa      60

catcccaaat tggatccgtt cgcgggtccc ct                                    92


<210>  3
<211>  17
<212>  DNA
<213>  Artificial sequence

<220>
<223>  Secuencia de reconocimiento por el promotor de la ARN polimerasa
       del fago T7

<400>  3
taatacgact cactata                                                     17


<210>  4
<211>  67
<212>  DNA
<213>  Artificial sequence

<220>
<223>  Terminador transcripcional de la ARN Polimerasa del fago T7

<400>  4
aaccccttgg ggcctctaaa cgggtcttga ggggtttttt gctgaaagga ggaactatat      60

ccggatc                                                                67

<210> 5
<211> 159
<212> DNA
<213> Virus de la hepatitis delta

<400> 5
gggtcggcat ggcatctcca cctcctcgcg gtccgacctg ggcatccgaa ggaggacgtc 60

gtccactcgg atggctaagg gagagctcgg atccggctgc taacaaagcc cgaaaggaag 120

ctgagttggc tgctgccacc gctgagcaat aactagcat 159


<210> 6
<211> 436
<212> DNA
<213> IBDV

<400> 6
atggtcagta gagatcagac aaacgatcgc agcgatgaca aacctgcaag atcaaaccca 60

acagattgtt ccgttcatac ggagccttct gatgccaaca accggaccgg cgtccattgt 120

gcatgcagag aagagccggt tggcatcaga agaacaaatc ctaagggcag ctacgtcgat 180

ctacggggct ccaggacagg cagagccacc ccaagctttc atagacgaag ttgccaaagt 240

ctatgaaatc aaccatggac gtggcccaaa ccaagaacag atgaaagatc tgctcttgac 300

tgcgatggag atgaagcatc gcaatcccag gcgggctcta ccaaagccca agccaaaacc 360

caatgctcca acacagagac ctcctggtcg gctgggccgc tggatcagga ccgtctctga 420

tgaggacctt gagtga 436


<210> 7
<211> 365
<212> DNA
<213> IBDV

<400> 7
atgagtgacg ttttcaatag tccacaggcg cgaagcacga tctcagcagc gttcggcata 60

aagcctactg ctggacaaga cgtggaagaa ctcttgatcc ctaaagtttg ggtgccacct 120

gaggatccgc ttgccagccc tagtcgactt ccgtgtacac ccccaagtac ccagaagtca 180

agaacccaca gaccgcctcc aaccccgttg ttgggctcca cctgcccgcc aagagagcca 240

ccggtgtcca ggccgctctt ctcggagcag gaacgagcag accaatgggg atggaggccc 300

caacacggtc caagaacgcc gtgaaaatgg ccaaacggcg gcaacgccaa aaggagagcc 360

gctaa 365


<210> 8
<211> 899
<212> DNA

<213>  Artificial sequence

<220>
<223>  Secuencia del  gen quimérico Cas1

<400>  8
gcggccgcat cgatatatga attctaatac gactcactat aggatacgat cggtctgacc      60

ccgggggagt cacccgggga caggtcttca aggccttgtt ccaggatgga actcctcctt     120

ctacaacgct atcattgatg gtcagtagag atcagacaaa cgatcgcagc gatgacaaac     180

ctgcaagatc aaacccaaca gattgttccg ttcatacgga gccttctgat gccaacaacc     240

ggaccggcgt ccattgtgca tgcagagaag agccggttgg catcagaaga acaaatccta     300

agggcagcta cgtcgatcta cggggctcca ggacaggcag agccacccca agctttcata     360

gacgaagttg ccaaagtcta tgaaatcaac catggacgtg gcccaaacca agaacagatg     420

aaagatctgc tcttgactgc gatggagatg aagcatcgca atcccaggcg ggctctacca     480

aagcccaagc caaaacccaa tgctccaaca cagagacctc ctggtcggct gggccgctgg     540

atcaggaccg tctctgatga ggaccttgag tgaggctcct gggagtctcc cgacaccacc     600

cgcgcaggtg tggacaccaa ttcggcctta caacatccca aattggatcc gttcgcgggt     660

cccctgggtc ggcatggcat ctccacctcc tcgcggtccg acctgggcat ccgaaggagg     720

acgtcgtcca ctcggatggc taagggagag ctcggatccg gctgctaaca aagcccgaaa     780

ggaagctgag ttggctgctg ccaccgctga gcaataacta gcataacccc ttggggcctc     840

taaacgggtc ttgaggggtt ttttgctgaa aggaggaact atatccggat cgcggccgc      899


<210>  9
<211>  3622
<212>  DNA
<213>  Artificial sequence

<220>
<223>  Secuencia del plásmido pCas1

<400>  9
tcgcgcgttt cggtgatgac ggtgaaaacc tctgacacat gcagctcccg gagacggtca      60

cagcttgtct gtaagcggat gccgggagca gacaagcccg tcagggcgcg tcagcgggtg     120

ttggcgggtg tcggggctgg cttaactatg cggcatcaga gcagattgta ctgagagtgc     180

accatatgcg gtgtgaaata ccgcacagat gcgtaaggag aaaataccgc atcaggcgcc     240

attcgccatt caggctgcgc aactgttggg aagggcgatc ggtgcgggcc tcttcgctat     300

tacgccagct ggcgaaaggg ggatgtgctg caaggcgatt aagttgggta acgccagggt     360

tttcccagtc acgacgttgt aaaacgacgg ccagtgaatt cgagctcggt acctcgcgaa     420

tgcatctaga tccaatgcgc gcggccgcat cgatatatga attctaatac gactcactat     480

aggatacgat cggtctgacc ccgggggagt cacccgggga caggtcttca aggccttgtt 540
ccaggatgga actcctcctt ctacaacgct atcattgatg gtcagtagag atcagacaaa 600
cgatcgcagc gatgacaaac ctgcaagatc aaacccaaca gattgttccg ttcatacgga 660
gccttctgat gccaacaacc ggaccggcgt ccattgtgca tgcagagaag agccggttgg 720
catcagaaga acaaatccta agggcagcta cgtcgatcta cggggctcca ggacaggcag 780
agccacccca agctttcata gacgaagttg ccaaagtcta tgaaatcaac catggacgtg 840
gcccaaacca agaacagatg aaagatctgc tcttgactgc gatggagatg aagcatcgca 900
atcccaggcg ggctctacca aagcccaagc caaaacccaa tgctccaaca cagagacctc 960
ctggtcggct gggccgctgg atcaggaccg tctctgatga ggaccttgag tgaggctcct 1020
gggagtctcc cgacaccacc cgcgcaggtg tggacaccaa ttcggcctta caacatccca 1080
aattggatcc gttcgcgggt cccctgggtc ggcatggcat ctccacctcc tcgcggtccg 1140
acctgggcat ccgaaggagg acgtcgtcca ctcggatggc taagggagag ctcggatccg 1200
gctgctaaca aagcccgaaa ggaagctgag ttggctgctg ccaccgctga gcaataacta 1260
gcataacccc ttggggcctc taaacgggtc ttgaggggtt ttttgctgaa aggaggaact 1320
atatccggat cgcggccgcg cgcatcggat cccgggcccg tcgactgcag aggcctgcat 1380
gcaagcttgg cgtaatcatg gtcatagctg tttcctgtgt gaaattgtta tccgctcaca 1440
attccacaca acatacgagc cggaagcata aagtgtaaag cctggggtgc ctaatgagtg 1500
agctaactca cattaattgc gttgcgctca ctgcccgctt tccagtcggg aaacctgtcg 1560
tgccagctgc attaatgaat cggccaacgc gcggggagag gcggtttgcg tattgggcgc 1620
tcttccgctt cctcgctcac tgactcgctg cgctcggtcg ttcggctgcg gcgagcggta 1680
tcagctcact caaaggcggt aatacggtta tccacagaat caggggataa cgcaggaaag 1740
aacatgtgag caaaaggcca gcaaaaggcc aggaaccgta aaaaggccgc gttgctggcg 1800
tttttccata ggctccgccc ccctgacgag catcacaaaa atcgacgctc aagtcagagg 1860
tggcgaaacc cgacaggact ataaagatac caggcgtttc cccctggaag ctccctcgtg 1920
cgctctcctg ttccgaccct gccgcttacc ggatacctgt ccgcctttct cccttcggga 1980
agcgtggcgc tttctcatag ctcacgctgt aggtatctca gttcggtgta ggtcgttcgc 2040
tccaagctgg gctgtgtgca cgaacccccc gttcagcccg accgctgcgc cttatccggt 2100
aactatcgtc ttgagtccaa cccggtaaga cacgacttat cgccactggc agcagccact 2160
ggtaacagga ttagcagagc gaggtatgta ggcggtgcta cagagttctt gaagtggtgg 2220
cctaactacg gctacactag aagaacagta tttggtatct gcgctctgct gaagccagtt 2280

accttcggaa aaagagttgg tagctcttga tccggcaaac aaaccaccgc tggtagcggt 2340

ggtttttttg tttgcaagca gcagattacg cgcagaaaaa aaggatctca agaagatcct 2400

ttgatctttt ctacggggtc tgacgctcag tggaacgaaa actcacgtta agggattttg 2460

gtcatgagat tatcaaaaag gatcttcacc tagatccttt taaattaaaa atgaagtttt 2520

aaatcaatct aaagtatata tgagtaaact tggtctgaca gttaccaatg cttaatcagt 2580

gaggcaccta tctcagcgat ctgtctattt cgttcatcca tagttgcctg actccccgtc 2640

gtgtagataa ctacgatacg ggagggctta ccatctggcc ccagtgctgc aatgataccg 2700

cgagacccac gctcaccggc tccagattta tcagcaataa accagccagc cggaagggcc 2760

gagcgcagaa gtggtcctgc aactttatcc gcctccatcc agtctattaa ttgttgccgg 2820

gaagctagag taagtagttc gccagttaat agtttgcgca acgttgttgc cattgctaca 2880

ggcatcgtgg tgtcacgctc gtcgtttggt atggcttcat tcagctccgg ttcccaacga 2940

tcaaggcgag ttacatgatc ccccatgttg tgcaaaaaag cggttagctc cttcggtcct 3000

ccgatcgttg tcagaagtaa gttggccgca gtgttatcac tcatggttat ggcagcactg 3060

cataattctc ttactgtcat gccatccgta agatgctttt ctgtgactgg tgagtactca 3120

accaagtcat tctgagaata gtgtatgcgg cgaccgagtt gctcttgccc ggcgtcaata 3180

cgggataata ccgcgccaca tagcagaact ttaaaagtgc tcatcattgg aaaacgttct 3240

tcggggcgaa aactctcaag gatcttaccg ctgttgagat ccagttcgat gtaacccact 3300

cgtgcaccca actgatcttc agcatctttt actttcacca gcgtttctgg gtgagcaaaa 3360

acaggaaggc aaaatgccgc aaaaaaggga ataagggcga cacggaaatg ttgaatactc 3420

atactcttcc tttttcaata ttattgaagc atttatcagg gttattgtct catgagcgga 3480

tacatatttg aatgtattta gaaaaataaa caaatagggg ttccgcgcac atttccccga 3540

aaagtgccac ctgacgtcta agaaaccatt attatcatga cattaaccta taaaaatagg 3600

cgtatcacga ggccctttcg tc 3622

<210> 10
<211> 3622
<212> DNA
<213> Artificial sequence

<220>
<223> Secuencia del plásmido pHA-Cas1

<400> 10
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgaatt cgagctcggt acctcgcgaa 420

tgcatctaga tccaatgcgc gcggccgcat cgatatatga attctaatac gactcactat 480

aggatacgat cggtctgacc ccgggggagt cacccgggga caggtcttca aggccttgtt 540

ccaggatgga actcctcctt ctacaacgct atcattgatg gtcagtagag atcagacaaa 600

cgatcgcagc gatgacaaac ctgcaagatc aaacccaaca gattgttccg ttcatacgga 660

gccttctgat gccaacaacc ggaccggcgt ccattgtgca tgcagagaag agccggttgg 720

catcagaaga acaaatccta agggcagcta cgtcgatcta cggggctcca ggacaggcag 780

agccacccca agctttcata gacgaagttg ccaaagtcta tgaaatcaac catggacgtg 840

gcccaaacca agaacagatg aaagatctgc tcttgactgc gatggagatg aagcatcgca 900

atcccaggcg ggctctacca aagcccaagc caaaacccaa tgctccaaca cagagacctc 960

ctggtcggct gggccgctgg atcaggaccg tctctgatga ggaccttgag tgaggctcct 1020

gggagtctcc cgacaccacc cgcgcaggtg tggacaccaa ttcggcctta caacatccca 1080

aattggatcc gttcgcgggt cccctgggtc ggcatggcat ctccacctcc tcgcggtccg 1140

acctgggcat ccgaaggagg acgtcgtcca ctcggatggc taagggagag ctcggatccg 1200

gctgctaaca aagcccgaaa ggaagctgag ttggctgctg ccaccgctga gcaataacta 1260

gcataacccc ttggggcctc taaacgggtc ttgaggggtt ttttgctgaa aggaggaact 1320

atatccggat cgcggccgcg cgcatcggat cccgggcccg tcgactgcag aggcctgcat 1380

gcaagcttgg cgtaatcatg gtcatagctg tttcctgtgt gaaattgtta tccgctcaca 1440

attccacaca acatacgagc cggaagcata aagtgtaaag cctggggtgc ctaatgagtg 1500

agctaactca cattaattgc gttgcgctca ctgcccgctt tccagtcggg aaacctgtcg 1560

tgccagctgc attaatgaat cggccaacgc gcggggagag gcggtttgcg tattgggcgc 1620

tcttccgctt cctcgctcac tgactcgctg cgctcggtcg ttcggctgcg gcgagcggta 1680

tcagctcact caaaggcggt aatacggtta tccacagaat caggggataa cgcaggaaag 1740

aacatgtgag caaaaggcca gcaaaaggcc aggaaccgta aaaaggccgc gttgctggcg 1800

tttttccata ggctccgccc ccctgacgag catcacaaaa atcgacgctc aagtcagagg 1860

tggcgaaacc cgacaggact ataaagatac caggcgtttc cccctggaag ctccctcgtg 1920

cgctctcctg ttccgaccct gccgcttacc ggatacctgt ccgcctttct cccttcggga 1980

agcgtggcgc tttctcatag ctcacgctgt aggtatctca gttcggtgta ggtcgttcgc 2040

tccaagctgg gctgtgtgca cgaacccccc gttcagcccg accgctgcgc cttatccggt 2100

aactatcgtc ttgagtccaa cccggtaaga cacgacttat cgccactggc agcagccact 2160

ggtaacagga ttagcagagc gaggtatgta ggcggtgcta cagagttctt gaagtggtgg 2220

cctaactacg gctacactag aagaacagta tttggtatct gcgctctgct gaagccagtt 2280

accttcggaa aaagagttgg tagctcttga tccggcaaac aaaccaccgc tggtagcggt 2340

ggtttttttg tttgcaagca gcagattacg cgcagaaaaa aaggatctca agaagatcct 2400

ttgatctttt ctacggggtc tgacgctcag tggaacgaaa actcacgtta agggattttg 2460

gtcatgagat tatcaaaaag gatcttcacc tagatccttt taaattaaaa atgaagtttt 2520

aaatcaatct aaagtatata tgagtaaact tggtctgaca gttaccaatg cttaatcagt 2580

gaggcaccta tctcagcgat ctgtctattt cgttcatcca tagttgcctg actccccgtc 2640

gtgtagataa ctacgatacg ggagggctta ccatctggcc ccagtgctgc aatgataccg 2700

cgagacccac gctcaccggc tccagattta tcagcaataa accagccagc cggaagggcc 2760

gagcgcagaa gtggtcctgc aactttatcc gcctccatcc agtctattaa ttgttgccgg 2820

gaagctagag taagtagttc gccagttaat agtttgcgca acgttgttgc cattgctaca 2880

ggcatcgtgg tgtcacgctc gtcgtttggt atggcttcat tcagctccgg ttcccaacga 2940

tcaaggcgag ttacatgatc ccccatgttg tgcaaaaaag cggttagctc cttcggtcct 3000

ccgatcgttg tcagaagtaa gttggccgca gtgttatcac tcatggttat ggcagcactg 3060

cataattctc ttactgtcat gccatccgta agatgctttt ctgtgactgg tgagtactca 3120

accaagtcat tctgagaata gtgtatgcgg cgaccgagtt gctcttgccc ggcgtcaata 3180

cgggataata ccgcgccaca tagcagaact ttaaaagtgc tcatcattgg aaaacgttct 3240

tcggggcgaa aactctcaag gatcttaccg ctgttgagat ccagttcgat gtaacccact 3300

cgtgcaccca actgatcttc agcatctttt actttcacca gcgtttctgg gtgagcaaaa 3360

acaggaaggc aaaatgccgc aaaaaaggga ataagggcga cacggaaatg ttgaatactc 3420

atactcttcc tttttcaata ttattgaagc atttatcagg gttattgtct catgagcgga 3480

tacatatttg aatgtattta gaaaaataaa caaatagggg ttccgcgcac atttccccga 3540

aaagtgccac ctgacgtcta agaaaccatt attatcatga cattaaccta taaaaatagg 3600

cgtatcacga ggccctttcg tc 3622

<210> 11
<211> 2653
<212> DNA
<213> Artificial sequence

<220>
<223>  Fragmento del vector pFastBac-Poly

<400>  11
gcgtccattc cggacgacac cctggagaag cacactctca ggtcagagac ctcgacctac        60

aatttgactg tgggggacac agggtcaggg ctaattgtct ttttccctgg attccctggc        120

tcaattgtgg gtgctcacta cacactgcag ggcaatggga actacaagtt cgatcagatg       180

ctcctgactg cccagaacct accggccagt tacaactact gcaggctagt gagtcggagt       240

ctcacagtga ggtcaagcac acttcctggt ggcgtttatg cactaaacgg caccataaac       300

gccgtgacct tccaaggaag cctgagtgaa ctgacagatg ttagctacaa tgggttgatg       360

tctgcaacag ccaacatcaa cgacaaaatt gggaacgtcc tagtagggga aggggtcacc       420

gtcctcagct tacccacatc atatgatctt gggtatgtga ggcttggtga ccccattccc       480

gcaatagggc ttgacccaaa aatggtagcc acatgtgaca gcagtgacag gcccagagtc       540

tacaccataa ctgcagccga tgattaccaa ttctcatcac agtaccaacc aggtggggta       600

acaatcacac tgttctcagc caacattgat gccatcacaa gcctcagcgt tgggggagag       660

ctcgtgtttc gaacaagcgt ccacggcctt gtactgggcg ccaccatcta cctcataggc       720

tttgatggga caacggtaat caccagggct gtggccgcaa acaatgggct gacgaccggc       780

accgacaacc ttatgccatt caatcttgtg attccaacaa acgagataac ccagccaatc       840

acatccatca aactggagat agtgacctcc aaaagtggtg gtcaggcagg ggatcagatg       900

tcatggtcgg caagagggag cctagcagtg acgatccatg gtggcaacta tccaggggcc       960

ctccgtcccg tcacgctagt ggcctacgaa agagtggcaa caggatccgt cgttacggtc      1020

gctggggtga gcaacttcga gctgatccca aatcctgaac tagcaaagaa cctggttaca      1080

gaatacggcc gatttgaccc aggagccatg aactacacaa aattgatact gagtgagagg      1140

gaccgtcttg gcatcaagac cgtctggcca acaagggagt acactgactt tcgtgaatac      1200

ttcatggagg tggccgacct caactctccc ctgaagattg caggagcatt cggcttcaaa      1260

gacataatcc gggccataag gaggatagct gtgccggtgg tctccacatt gttcccacct      1320

gccgctcccc tagcccatgc aattggggaa ggtgtagact acctgctggg cgatgaggcc      1380

caggccgctt caggaactgc tcgagccgcg tcaggaaaag caagagctgc ctcaggccgc      1440

ataaggcagc tgactctcgc cgccgacaag gggtacgagg tagtcgcgaa tctattccag      1500

gtgccccaga atcccgtagt cgacgggatt cttgcttcac ctggggtact ccgcggtgca      1560

cacaacctcg actgcgtgtt aagagagggt gccacgctat tccctgtggt tattacgaca      1620

gtggaagacg ccatgacacc caaagcattg aacagcaaaa tgtttgctgt cattgaaggc      1680

gtgcgagaag acctccaacc tccatctcaa agaggatcct tcatacgaac tctctctgga 1740

cacagagtct atggatatgc tccagatggg gtacttccac tggagactgg gagagactac 1800

accgttgtcc caatagatga tgtctgggac gacagcatta tgctgtccaa agatcccata 1860

cctcctattg tgggaaacag tggaaatcta gccatagctt acatggatgt gtttcgaccc 1920

aaagtcccaa tccatgtggc tatgacggga gccctcaatg cttgtggcga gattgagaaa 1980

gtaagcttta gaagcaccaa gctcgctact gcacaccgac ttggccttag gttggctggt 2040

cccggagcat tcgatgtaaa caccgggccc aactgggcaa cgttcatcaa acgtttccct 2100

cacaatccac gcgactggga caggctcccc tacctcaacc taccatacct tccacccaat 2160

gcaggacgcc agtaccacct tgccatggct gcatcagagt tcaaagagac ccccgaactc 2220

gagagtgccg tcagagcaat ggaagcagca gccaacgtgg acccactatt ccaatctgca 2280

ctcagtgtgt tcatgtggct ggaagagaat gggattgtga ctgacatggc caacttcgca 2340

ctcagcgacc cgaacgccca tcggatgcga aattttcttg caaacgcacc acaagcaggc 2400

agcaagtcgc aaagggccaa gtacgggaca gcaggctacg gagtggaggc tcggggcccc 2460

acaccagagg aagcacagag ggaaaaagac acacggatct caaagaagat ggagaccatg 2520

ggcatctact ttgcaacacc agaatgggta gcactcaatg ggcaccgagg gccaagccca 2580

ggccaggtaa agtactggca gaacaaacga gaaataccgg acccaaacga ggactatcta 2640

gactacgtgc atg 2653

<210> 12
<211> 31
<212> DNA
<213> Artificial sequence

<220>
<223> Cebador directo utilizado para amplificar el gen GPT junto con el
      promotor E/L de vaccina flanqueado por dianas ClaI

<400> 12
gcgcatcgat catttcgcgg gatcgagatc c 31

<210> 13
<211> 34
<212> DNA
<213> Artificial sequence

<220>
<223> Cebador reverso utilizado para amplificar el gen GPT junto con el
      promotor E/L de vaccina flanqueado por dianas ClaI

<400> 13
gcgcatcgat agaaaaatta gcgaccggag attg 34

<210>  14
<211>  642
<212>  DNA
<213>  Artificial sequence

<220>
<223>  Secuencia del gen GPT precedido del promotor e/l de vaccina
       flanqueado por dianas ClaI

<400>  14
gcgcatcgat catttcgcgg gatcgagatc catgtcgatc gacgatcact aattccaaac      60

ccacccgctt tttatagtaa gtttttcacc cataaataat aaatacaata attaatttct     120

cgtaaaagta gaaaatatat tctaatttat tgcacggtaa ggaagtatga gcgaaaaata     180

catcgtcacc tgggacatgt tgcagatcca tgcacgtaaa ctcgcaagcc gactgatgcc     240

ttctgaacaa tggaaaggca ttattgccgt aagccgtggc ggtctggtac cgggtgcgtt     300

actggcgcgt gaactgggta ttcgtcatgt cgataccgtt tgtatttcca gctacgatca     360

cgacaaccag cgcgagctta aagtgctgaa acgcgcagaa ggcgatggcg aaggcttcat     420

cgttattgat gacctggtgg ataccggtgg tactgcggtt gcgattcgtg aaatgtatcc     480

aaaagcgcac tttgtcacca tcttcgcaaa accggctggt cgtccgctgg gtgatgacta     540

tgttgttgat atcccgcaag atacctggat tgaacagccg tgggatatgg gcgtcgtatt     600

cgtcccgcca atctccggtc gctaattttt ctatcgatgc gc                        642


<210>  15
<211>  8610
<212>  DNA
<213>  Artificial sequence

<220>
<223>  Secuencia del plásmido pT-ReGS A

<400>  15
cgatatatga attctaatac gactcactat aggatacgat cggtctgacc ccgggggagt      60

cacccgggga caggtcttca aggccttgtt ccaggatgga actcctcctt ctacaacgct     120

atcattgatg gtcagtagag atcagacaaa cgatcgcagc gatgacaaac ctgcaagatc     180

aaacccaaca gattgttccg ttcatacgga gccttctgat gccaacaacc ggaccggcgt     240

ccattccgga cgacaccctg gagaagcaca ctctcaggtc agagacctcg acctacaatt     300

tgactgtggg ggacacaggg tcagggctaa ttgtcttttt ccctggattc cctggctcaa     360

ttgtgggtgc tcactacaca ctgcagggca atgggaacta caagttcgat cagatgctcc     420

tgactgccca gaacctaccg gccagttaca actactgcag gctagtgagt cggagtctca     480

cagtgaggtc aagcacactt cctggtggcg tttatgcact aaacggcacc ataaacgccg     540

tgaccttcca aggaagcctg agtgaactga cagatgttag ctacaatggg ttgatgtctg     600

caacagccaa catcaacgac aaaattggga acgtcctagt aggggaaggg gtcaccgtcc 660

tcagcttacc cacatcatat gatcttgggt atgtgaggct tggtgacccc attcccgcaa 720

tagggcttga cccaaaaatg gtagccacat gtgacagcag tgacaggccc agagtctaca 780

ccataactgc agccgatgat taccaattct catcacagta ccaaccaggt ggggtaacaa 840

tcacactgtt ctcagccaac attgatgcca tcacaagcct cagcgttggg ggagagctcg 900

tgtttcgaac aagcgtccac ggccttgtac tgggcgccac catctacctc ataggctttg 960

atgggacaac ggtaatcacc agggctgtgg ccgcaaacaa tgggctgacg accggcaccg 1020

acaaccttat gccattcaat cttgtgattc caacaaacga gataacccag ccaatcacat 1080

ccatcaaact ggagatagtg acctccaaaa gtggtggtca ggcaggggat cagatgtcat 1140

ggtcggcaag agggagccta gcagtgacga tccatggtgg caactatcca ggggccctcc 1200

gtcccgtcac gctagtggcc tacgaaagag tggcaacagg atccgtcgtt acggtcgctg 1260

gggtgagcaa cttcgagctg atcccaaatc ctgaactagc aaagaacctg gttacagaat 1320

acggccgatt tgacccagga gccatgaact acacaaaatt gatactgagt gagagggacc 1380

gtcttggcat caagaccgtc tggccaacaa gggagtacac tgactttcgt gaatacttca 1440

tggaggtggc cgacctcaac tctcccctga agattgcagg agcattcggc ttcaaagaca 1500

taatccgggc cataaggagg atagctgtgc cggtggtctc cacattgttc ccacctgccg 1560

ctcccctagc ccatgcaatt ggggaaggtg tagactacct gctgggcgat gaggcccagg 1620

ccgcttcagg aactgctcga gccgcgtcag gaaaagcaag agctgcctca ggccgcataa 1680

ggcagctgac tctcgccgcc gacaaggggt acgaggtagt cgcgaatcta ttccaggtgc 1740

cccagaatcc cgtagtcgac gggattcttg cttcacctgg ggtactccgc ggtgcacaca 1800

acctcgactg cgtgttaaga gagggtgcca cgctattccc tgtggttatt acgacagtgg 1860

aagacgccat gacacccaaa gcattgaaca gcaaaatgtt tgctgtcatt gaaggcgtgc 1920

gagaagacct ccaacctcca tctcaaagag gatccttcat acgaactctc tctggacaca 1980

gagtctatgg atatgctcca gatggggtac ttccactgga gactgggaga gactacaccg 2040

ttgtcccaat agatgatgtc tgggacgaca gcattatgct gtccaaagat cccatacctc 2100

ctattgtggg aaacagtgga aatctagcca tagcttacat ggatgtgttt cgacccaaag 2160

tcccaatcca tgtggctatg acgggagccc tcaatgcttg tggcgagatt gagaaagtaa 2220

gctttagaag caccaagctc gctactgcac accgacttgg ccttaggttg gctggtcccg 2280

gagcattcga tgtaaacacc gggcccaact gggcaacgtt catcaaacgt ttccctcaca 2340

atccacgcga ctgggacagg ctcccctacc tcaacctacc ataccttcca cccaatgcag 2400

gacgccagta ccaccttgcc atggctgcat cagagttcaa agagaccccc gaactcgaga 2460

gtgccgtcag agcaatggaa gcagcagcca acgtggaccc actattccaa tctgcactca 2520

gtgtgttcat gtggctggaa gagaatggga ttgtgactga catggccaac ttcgcactca 2580

gcgacccgaa cgcccatcgg atgcgaaatt ttcttgcaaa cgcaccacaa gcaggcagca 2640

agtcgcaaag ggccaagtac gggacagcag gctacggagt ggaggctcgg ggccccacac 2700

cagaggaagc acagagggaa aaagacacac ggatctcaaa gaagatggag accatgggca 2760

tctactttgc aacaccagaa tgggtagcac tcaatgggca ccgagggcca agcccaggcc 2820

aggtaaagta ctggcagaac aaacgagaaa taccggaccc aaacgaggac tatctagact 2880

acgtgcatgc agagaagagc cggttggcat cagaagaaca aatcctaagg gcagctacgt 2940

cgatctacgg ggctccagga caggcagagc caccccaagc tttcatagac gaagttgcca 3000

aagtctatga aatcaaccat ggacgtggcc caaaccaaga acagatgaaa gatctgctct 3060

tgactgcgat ggagatgaag catcgcaatc ccaggcgggc tctaccaaag cccaagccaa 3120

aacccaatgc tccaacacag agacctcctg gtcggctggg ccgctggatc aggaccgtct 3180

ctgatgagga ccttgagtga ggctcctggg agtctcccga caccacccgc gcaggtgtgg 3240

acaccaattc ggccttacaa catcccaaat tggatccgtt cgcgggtccc ctgggtcggc 3300

atggcatctc cacctcctcg cggtccgacc tgggcatccg aaggaggacg tcgtccactc 3360

ggatggctaa gggagagctc ggatccggct gctaacaaag cccgaaagga agctgagttg 3420

gctgctgcca ccgctgagca ataactagca taaccccttg gggcctctaa acgggtcttg 3480

aggggttttt tgctgaaagg aggaactata tccggatcgc ggccgcccaa ttccgactcc 3540

ggaaccaatt actgataatg tagaagatca tacagacacc gtcacataca ctagtgatag 3600

cattaataca gtaagtgcat catctggaga atccacaaca gacgagactc cggaaccaat 3660

tactgataaa gaagatcata cagttacaga cactgtctca tacactacag taagtacatc 3720

atctggaatt gtcactacta aatcaaccac cgatgatgcg gatctttatg atacgtacaa 3780

tgataatgat acagtaccac caactactgt aggcggtagt acaacctcta ttagcaatta 3840

taaaaccaag gactttgtag aaatatttgg tattaccgca ttaattatat tgtcggccgt 3900

ggcaattttc tgtattacat attatatata taataaacgt tcacgtaaat acaaaacaga 3960

gaacaaagtc tagatttttg acttacataa atgtctggga tagtaaaatc tatcatattg 4020

agcggaccat ctggtttagg aaagacagcc atagccaaaa gactatggga atatatttgg 4080

atttgtggtg tcccatacca ctagatttcc tcgtcctatg gaacgagaag gtgttgatta 4140

ccattacgtt aacagagagg ccatctggaa gggaatagcc gccggaaact ttctagaaca 4200

tactgagttt ttaggaaata tttacggaac ttctaaaaca gctgtgaata cagcggctat 4260

taataatcgt atttgtgtga tggatctaaa catcgacggt gttagaagtc ttaaaaatac 4320

gtacctaatg ccttactcgg tgtatataag acctacctct cttaaaatgg ttgagaccaa 4380

gcttggcact ggccgtcgtt ttacaacgtc gtgactggga aaaccctggc gttacccaac 4440

ttaatcgcct tgcagcacat ccccctttcg ccagctggcg taatagcgaa gaggcccgca 4500

ccgatcgccc ttcccaacag ttgcgcagcc tgaatggcga atggcgcctg atgcggtatt 4560

ttctccttac gcatctgtgc ggtatttcac accgcatata tggtgcactc tcagtacaat 4620

ctgctctgat gccgcatagt taagccagcc ccgacacccg ccaacacccg ctgacgcgcc 4680

ctgacgggct tgtctgctcc cggcatccgc ttacagacaa gctgtgaccg tctccgggag 4740

ctgcatgtgt cagaggtttt caccgtcatc accgaaacgc gcgagacgaa agggcctcgt 4800

gatacgccta tttttatagg ttaatgtcat gataataatg gtttcttaga cgtcaggtgg 4860

cacttttcgg ggaaatgtgc gcggaacccc tatttgttta tttttctaaa tacattcaaa 4920

tatgtatccg ctcatgagac aataaccctg ataaatgctt caataatatt gaaaaaggaa 4980

gagtatgagt attcaacatt tccgtgtcgc ccttattccc ttttttgcgg cattttgcct 5040

tcctgttttt gctcacccag aaacgctggt gaaagtaaaa gatgctgaag atcagttggg 5100

tgcacgagtg ggttacatcg aactggatct caacagcggt aagatccttg agagttttcg 5160

ccccgaagaa cgttttccaa tgatgagcac ttttaaagtt ctgctatgtg gcgcggtatt 5220

atcccgtatt gacgccgggc aagagcaact cggtcgccgc atacactatt ctcagaatga 5280

cttggttgag tactcaccag tcacagaaaa gcatcttacg gatggcatga cagtaagaga 5340

attatgcagt gctgccataa ccatgagtga taacactgcg gccaacttac ttctgacaac 5400

gatcggagga ccgaaggagc taaccgcttt tttgcacaac atgggggatc atgtaactcg 5460

ccttgatcgt tgggaaccgg agctgaatga agccatacca aacgacgagc gtgacaccac 5520

gatgcctgta gcaatggcaa caacgttgcg caaactatta actggcgaac tacttactct 5580

agcttcccgg caacaattaa tagactggat ggaggcggat aaagttgcag gaccacttct 5640

gcgctcggcc cttccggctg gctggtttat tgctgataaa tctggagccg gtgagcgtgg 5700

gtctcgcggt atcattgcag cactggggcc agatggtaag ccctcccgta tcgtagttat 5760

ctacacgacg gggagtcagg caactatgga tgaacgaaat agacagatcg ctgagatagg 5820

tgcctcactg attaagcatt ggtaactgtc agaccaagtt tactcatata tactttagat 5880

tgatttaaaa cttcattttt aatttaaaag gatctaggtg aagatccttt ttgataatct 5940

catgaccaaa atcccttaac gtgagttttc gttccactga gcgtcagacc ccgtagaaaa 6000

gatcaaagga tcttcttgag atcctttttt tctgcgcgta atctgctgct tgcaaacaaa 6060

aaaaccaccg ctaccagcgg tggtttgttt gccggatcaa gagctaccaa ctctttttcc 6120

gaaggtaact ggcttcagca gagcgcagat accaaatact gtccttctag tgtagccgta 6180

gttaggccac cacttcaaga actctgtagc accgcctaca tacctcgctc tgctaatcct 6240

gttaccagtg gctgctgcca gtggcgataa gtcgtgtctt accgggttgg actcaagacg 6300

atagttaccg gataaggcgc agcggtcggg ctgaacgggg ggttcgtgca cacagcccag 6360

cttggagcga acgacctaca ccgaactgag atacctacag cgtgagctat gagaaagcgc 6420

cacgcttccc gaagggagaa aggcggacag gtatccggta agcggcaggg tcggaacagg 6480

agagcgcacg agggagcttc cagggggaaa cgcctggtat ctttatagtc ctgtcgggtt 6540

tcgccacctc tgacttgagc gtcgattttt gtgatgctcg tcaggggggc ggagcctatg 6600

gaaaaacgcc agcaacgcgg cctttttacg gttcctggcc ttttgctggc cttttgctca 6660

catgttcttt cctgcgttat cccctgattc tgtggataac cgtattaccg cctttgagtg 6720

agctgatacc gctcgccgca gccgaacgac cgagcgcagc gagtcagtga gcgaggaagc 6780

ggaagagcgc ccaatacgca aaccgcctct ccccgcgcgt tggccgattc attaatgcag 6840

ctggcacgac aggtttcccg actggaaagc gggcagtgag cgcaacgcaa ttaatgtgag 6900

ttagctcact cattaggcac cccaggcttt acactttatg cttccggctc gtatgttgtg 6960

tggaattgtg agcggataac aatttcacac aggaaacagc tatgaccatg attacgaatt 7020

tcgacgattg ttcatgatgg caagatttat atatctggag gttacaacaa tagtagtgta 7080

gttaatgtaa tatcgaatct agtccttagc tataatccga tatatgatga atggaccaaa 7140

ttatcatcat taaacattcc tagaattaat cccgctctat ggtcagcgca taataaatta 7200

tatgtaggag gaggaatatc tgatgatgtt cgaactaata catctgaaac atacgataaa 7260

gaaaaagatt gttggacatt ggataatggt cacgtgttac cacgcaatta tataatgtat 7320

aaatgcgaac cgattaaaca taaatatcca ttggaaaaaa cacagtacac gaatgatttt 7380

ctaaagtatt tggaaagttt tataggtagt tgatagaaca aaatacataa ttttgtaaaa 7440

ataaatcact ttttatacta atatgacacg attaccaata cttttgttac taatatcatt 7500

agtatacgct acaccttttc ctcagacatc taaaaaaata ggtgatgatg caactctatc 7560

atgtaatcga aataatacaa atgactacgt tgttatgagt gcttggtata aggagcccaa 7620

ttccattatt cttttagctg ctaaaagcga cgtcttgtat tttgataatt ataccaagga 7680

taaaatatct tacgactctc catacgatga tctagttaca actatcacaa ttaaatcatt 7740

gactgctaga gatgccggta cttatgtatg tgcattcttt atgacatcaa ctacaaatga 7800

cactgataaa gtagattatg aagaatactc cacagagttg attgtaaata cagatagtga 7860

atcgactata gacataatac tatctggatc tacacattca ccggaaacta gttctaagaa 7920

acctgattat atagataatt ctaattgctc gtcggtattc gaaatcggag ctgcggccgc 7980

atcgatagaa aaattagcga ccggagattg gcgggacgaa tacgacgccc atatcccacg 8040

gctgttcaat ccaggtatct tgcgggatat caacaacata gtcatcaccc agcggacgac 8100

cagccggttt tgcgaagatg gtgacaaagt gcgcttttgg atacatttca cgaatcgcaa 8160

ccgcagtacc accggtatcc accaggtcat caataacgat gaagccttcg ccatcgcctt 8220

ctgcgcgttt cagcacttta agctcgcgct ggttgtcgtg atcgtagctg gaaatacaaa 8280

cggtatcgac atgacgaata cccagttcac gcgccagtaa cgcacccggt accagaccgc 8340

cacggcttac ggcaataatg cctttccatt gttcagaagg catcagtcgg cttgcgagtt 8400

tacgtgcatg gatctgcaac atgtcccagg tgacgatgta tttttcgctc atacttcctt 8460

accgtgcaat aaattagaat atattttcta cttttacgag aaattaatta ttgtatttat 8520

tatttatggg tgaaaaactt actataaaaa gcgggtgggt ttggaattag tgatcgtcga 8580

tcgacatgga tctcgatccc gcgaaatgat 8610

<210> 16
<211> 830
<212> DNA
<213> Artificial sequence

<220>
<223> Secuencia del gen quimérico Cas2

<400> 16
gcggccgcat cgatatatga attctaatac gactcactat aggatacgat gggtctgacc 60

ctctgggagt cacgaattaa cgtggctact aggggcgata cccgccgctg gccgccacgt 120

tagtggctcc tcttcttgat gattctgcca ccatgagtga cgttttcaat agtccacagg 180

cgcgaagcac gatctcagca gcgttcggca taaagcctac tgctggacaa gacgtggaag 240

aactcttgat ccctaaagtt tgggtgccac ctgaggatcc gcttgccagc cctagtcgac 300

ttccgtgtac acccccaagt acccagaagt caagaaccca cagaccgcct ccaaccccgt 360

tgttgggctc cacctgcccg ccaagagagc caccggtgtc caggccgctc ttctcggagc 420

aggaacgagc agaccaatgg ggatggaggc cccaacacgg tccaagaacg ccgtgaaaat 480

ggccaaacgg cggcaacgcc aaaaggagag ccgctaacag ccatgatggg aaccactcaa 540

gaagaggaca ctaatcccag accccgtatc cccggccttc gcctgcgggg gcccccgggt 600

cggcatggca tctccacctc ctcgcggtcc gacctgggca tccgaaggag gacgtcgtcc 660

actcggatgg ctaaggagag ctcggatcc ggctgctaac aaagcccgaa aggaagctga 720

gttggctgct gccaccgctg agcaataact agcataaccc cttggggcct ctaaacgggt 780

cttgaggggt tttttgctga aaggaggaac tatatccgga tcgcggccgc 830

<210> 17
<211> 3549
<212> DNA
<213> Artificial sequence

<220>
<223> Secuencia del gen plásmido pCas2

<400> 17
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgaatt cgagctcggt acctcgcgaa 420

tgcatctaga tccaatgcgc gcggccgcat cgatatatga attctaatac gactcactat 480

aggatacgat gggtctgacc ctctgggagt cacgaattaa cgtggctact aggggcgata 540

cccgccgctg gccgccacgt tagtggctcc tcttcttgat gattctgcca ccatgagtga 600

cgttttcaat agtccacagg cgcgaagcac gatctcagca gcgttcggca taaagcctac 660

tgctggacaa gacgtggaag aactcttgat ccctaaagtt tgggtgccac ctgaggatcc 720

gcttgccagc cctagtcgac ttccgtgtac acccccaagt acccagaagt caagaaccca 780

cagaccgcct ccaaccccgt tgttgggctc cacctgcccg ccaagagagc caccggtgtc 840

caggccgctc ttctcggagc aggaacgagc agaccaatgg ggatggaggc cccaacacgg 900

tccaagaacg ccgtgaaaat ggccaaacgg cggcaacgcc aaaaggagag ccgctaacag 960

ccatgatggg aaccactcaa gaagaggaca ctaatcccag accccgtatc cccggccttc 1020

gcctgcgggg gcccccgggt cggcatggca tctccacctc ctcgcggtcc gacctgggca 1080

tccgaaggag gacgtcgtcc actcggatgg ctaagggaga gctcggatcc ggctgctaac 1140

aaagcccgaa aggaagctga gttggctgct gccaccgctg agcaataact agcataaccc 1200

cttggggcct ctaaacgggt cttgaggggt tttttgctga aaggaggaac tatatccgga 1260

tcgcggccgc atcggatccc gggcccgtcg actgcagagg cctgcatgca agcttggcgt 1320

aatcatggtc atagctgttt cctgtgtgaa attgttatcc gctcacaatt ccacacaaca 1380

tacgagccgg aagcataaag tgtaaagcct ggggtgccta atgagtgagc taactcacat 1440

taattgcgtt gcgctcactg cccgctttcc agtcgggaaa cctgtcgtgc cagctgcatt 1500

aatgaatcgg ccaacgcgcg gggagaggcg gtttgcgtat tgggcgctct tccgcttcct 1560

cgctcactga ctcgctgcgc tcggtcgttc ggctgcggcg agcggtatca gctcactcaa 1620

aggcggtaat acggttatcc acagaatcag gggataacgc aggaaagaac atgtgagcaa 1680

aaggccagca aaaggccagg aaccgtaaaa aggccgcgtt gctggcgttt ttccataggc 1740

tccgcccccc tgacgagcat cacaaaaatc gacgctcaag tcagaggtgg cgaaacccga 1800

caggactata aagataccag gcgtttcccc ctggaagctc cctcgtgcgc tctcctgttc 1860

cgaccctgcc gcttaccgga tacctgtccg cctttctccc ttcgggaagc gtggcgcttt 1920

ctcatagctc acgctgtagg tatctcagtt cggtgtaggt cgttcgctcc aagctgggct 1980

gtgtgcacga accccccgtt cagcccgacc gctgcgcctt atccggtaac tatcgtcttg 2040

agtccaaccc ggtaagacac gacttatcgc cactggcagc agccactggt aacaggatta 2100

gcagagcgag gtatgtaggc ggtgctacag agttcttgaa gtggtggcct aactacggct 2160

acactagaag aacagtattt ggtatctgcg ctctgctgaa gccagttacc ttcggaaaaa 2220

gagttggtag ctcttgatcc ggcaaacaaa ccaccgctgg tagcggtggt ttttttgttt 2280

gcaagcagca gattacgcgc agaaaaaaag gatctcaaga agatcctttg atcttttcta 2340

cggggtctga cgctcagtgg aacgaaaact cacgttaagg gattttggtc atgagattat 2400

caaaaaggat cttcacctag atccttttaa attaaaaatg aagttttaaa tcaatctaaa 2460

gtatatatga gtaaacttgg tctgacagtt accaatgctt aatcagtgag gcacctatct 2520

cagcgatctg tctatttcgt tcatccatag ttgcctgact ccccgtcgtg tagataacta 2580

cgatacggga gggcttacca tctggcccca gtgctgcaat gataccgcga gacccacgct 2640

caccggctcc agatttatca gcaataaacc agccagccgg aagggccgag cgcagaagtg 2700

gtcctgcaac tttatccgcc tccatccagt ctattaattg ttgccgggaa gctagagtaa 2760

gtagttcgcc agttaatagt ttgcgcaacg ttgttgccat tgctacaggc atcgtggtgt 2820

cacgctcgtc gtttggtatg gcttcattca gctccggttc ccaacgatca aggcgagtta 2880

catgatcccc catgttgtgc aaaaaagcgg ttagctcctt cggtcctccg atcgttgtca 2940

gaagtaagtt ggccgcagtg ttatcactca tggttatggc agcactgcat aattctctta 3000

ctgtcatgcc atccgtaaga tgcttttctg tgactggtga gtactcaacc aagtcattct 3060

gagaatagtg tatgcggcga ccgagttgct cttgcccggc gtcaatacgg gataataccg 3120

cgccacatag cagaacttta aaagtgctca tcattggaaa acgttcttcg gggcgaaaac 3180

tctcaaggat cttaccgctg ttgagatcca gttcgatgta acccactcgt gcacccaact 3240

gatcttcagc atcttttact ttcaccagcg tttctgggtg agcaaaaaca ggaaggcaaa 3300

atgccgcaaa aaagggaata agggcgacac ggaaatgttg aatactcata ctcttccttt 3360

ttcaatatta ttgaagcatt tatcagggtt attgtctcat gagcggatac atatttgaat 3420

gtatttagaa aaataaacaa ataggggttc cgcgcacatt tccccgaaaa gtgccacctg 3480

acgtctaaga aaccattatt atcatgacat taacctataa aaataggcgt atcacgaggc 3540

cctttcgtc 3549

<210> 18
<211> 5276
<212> DNA
<213> Artificial sequence

<220>
<223> Secuencia del plásmido pHA_Cas2

<400> 18
ggccgcatcg atatatgaat tctaatacga ctcactatag gatacgatgg gtctgaccct 60

ctgggagtca cgaattaacg tggctactag ggcgataccc cgccgctggc cgccacgtta 120

gtggctcctc ttcttgatga ttctgccacc atgagtgacg ttttcaatag tccacaggcg 180

cgaagcacga tctcagcagc gttcggcata aagcctactg ctggacaaga cgtggaagaa 240

ctcttgatcc ctaaagtttg ggtgccacct gaggatccgc ttgccagccc tagtcgactt 300

ccgtgtacac ccccaagtac ccagaagtca agaacccaca gaccgcctcc aaccccgttg 360

ttgggctcca cctgcccgcc aagagagcca ccggtgtcca ggccgctctt ctcggagcag 420

gaacgagcag accaatgggg atggaggccc caacacggtc caagaacgcc gtgaaaatgg 480

ccaaacggcg gcaacgccaa aaggagagcc gctaacagcc atgatgggaa ccactcaaga 540

agaggacact aatcccagac cccgtatccc cggccttcgc ctgcgggggc ccccgggtcg 600

gcatggcatc tccacctcct cgcggtccga cctgggcatc cgaaggagga cgtcgtccac 660

tcggatggct aagggagagc tcggatccgg ctgctaacaa agcccgaaag gaagctgagt 720

tggctgctgc caccgctgag caataactag cataacccct tggggcctct aaacgggtct 780

tgaggggttt tttgctgaaa ggaggaacta tatccggatc gcggccgcag ctccgatttc 840

gaataccgac gagcaattag aattatctat ataatcaggt ttcttagaac tagtttccgg 900

tgaatgtgta gatccagata gtattatgtc tatagtcgat tcactatctg tatttacaat 960

caactctgtg gagtattctt cataatctac tttatcagtg tcatttgtag ttgatgtcat 1020

aaagaatgca catacataag taccggcatc tctagcagtc aatgatttaa ttgtgatagt 1080

tgtaactaga tcatcgtatg gagagtcgta agatatttta tccttggtat aattatcaaa 1140

atacaagacg tcgcttttag cagctaaaag aataatggaa ttgggctcct tataccaagc 1200

actcataaca acgtagtcat ttgtattatt tcgattacat gatagagttg catcatcacc 1260

tattttttta gatgtctgag gaaaaggtgt agcgtatact aatgatatta gtaacaaaag 1320

tattggtaat cgtgtcatat tagtataaaa agtgatttat ttttacaaaa ttatgtattt 1380

tgttctatca actacctata aaactttcca aatactttag aaaatcattc gtgtactgtg 1440

ttttttccaa tggatattta tgtttaatcg gttcgcattt atacattata taattgcgtg 1500

gtaacacgtg accattatcc aatgtccaac aatctttttc tttatcgtat gtttcagatg 1560

tattagttcg aacatcatca gatattcctc ctcctacata taatttatta tgcgctgacc 1620

atagagcggg attaattcta ggaatgttta atgatgataa tttggtccat tcatcatata 1680

tcggattata gctaaggact agattcgata ttacattaac tacactacta ttgttgtaac 1740

ctccagatat ataaatcttg ccatcatgaa caatcgtcga aattcgtaat catggtcata 1800

gctgtttcct gtgtgaaatt gttatccgct cacaattcca cacaacatac gagccggaag 1860

cataaagtgt aaagcctggg gtgcctaatg agtgagctaa ctcacattaa ttgcgttgcg 1920

ctcactgccc gctttccagt cgggaaacct gtcgtgccag ctgcattaat gaatcggcca 1980

acgcgcgggg agaggcggtt tgcgtattgg gcgctcttcc gcttcctcgc tcactgactc 2040

gctgcgctcg gtcgttcggc tgcggcgagc ggtatcagct cactcaaagg cggtaatacg 2100

gttatccaca gaatcagggg ataacgcagg aaagaacatg tgagcaaaag gccagcaaaa 2160

ggccaggaac cgtaaaaagg ccgcgttgct ggcgtttttc cataggctcc gcccccctga 2220

cgagcatcac aaaaatcgac gctcaagtca gaggtggcga aacccgacag gactataaag 2280

ataccaggcg tttccccctg gaagctccct cgtgcgctct cctgttccga ccctgccgct 2340

taccggatac ctgtccgcct ttctcccttc gggaagcgtg gcgctttctc atagctcacg 2400

ctgtaggtat ctcagttcgg tgtaggtcgt tcgctccaag ctgggctgtg tgcacgaacc 2460

ccccgttcag cccgaccgct gcgccttatc cggtaactat cgtcttgagt ccaacccggt 2520

aagacacgac ttatcgccac tggcagcagc cactggtaac aggattagca gagcgaggta 2580

tgtaggcggt gctacagagt tcttgaagtg gtggcctaac tacggctaca ctagaaggac 2640

agtatttggt atctgcgctc tgctgaagcc agttaccttc ggaaaaagag ttggtagctc 2700

ttgatccggc aaacaaacca ccgctggtag cggtggtttt tttgtttgca agcagcagat 2760

tacgcgcaga aaaaaaggat ctcaagaaga tcctttgatc ttttctacgg ggtctgacgc 2820

tcagtggaac gaaaactcac gttaagggat tttggtcatg agattatcaa aaaggatctt 2880

cacctagatc cttttaaatt aaaaatgaag ttttaaatca atctaaagta tatatgagta 2940

aacttggtct gacagttacc aatgcttaat cagtgaggca cctatctcag cgatctgtct 3000

atttcgttca tccatagttg cctgactccc cgtcgtgtag ataactacga tacgggaggg 3060

cttaccatct ggccccagtg ctgcaatgat accgcgagac ccacgctcac cggctccaga 3120

tttatcagca ataaaccagc cagccggaag ggccgagcgc agaagtggtc ctgcaacttt 3180

atccgcctcc atccagtcta ttaattgttg ccgggaagct agagtaagta gttcgccagt 3240

taatagtttg cgcaacgttg ttgccattgc tacaggcatc gtggtgtcac gctcgtcgtt 3300

tggtatggct tcattcagct ccggttccca acgatcaagg cgagttacat gatcccccat 3360

gttgtgcaaa aaagcggtta gctccttcgg tcctccgatc gttgtcagaa gtaagttggc 3420

cgcagtgtta tcactcatgg ttatggcagc actgcataat tctcttactg tcatgccatc 3480

cgtaagatgc ttttctgtga ctggtgagta ctcaaccaag tcattctgag aatagtgtat 3540

gcggcgaccg agttgctctt gcccggcgtc aatacgggat aataccgcgc cacatagcag 3600

aactttaaaa gtgctcatca ttggaaaacg ttcttcgggg cgaaaactct caaggatctt 3660

accgctgttg agatccagtt cgatgtaacc cactcgtgca cccaactgat cttcagcatc 3720

ttttactttc accagcgttt ctgggtgagc aaaaacagga aggcaaaatg ccgcaaaaaa 3780

gggaataagg gcgacacgga aatgttgaat actcatactc ttcctttttc aatattattg 3840

aagcatttat cagggttatt gtctcatgag cggatacata tttgaatgta tttagaaaaa 3900

taaacaaata ggggttccgc gcacatttcc ccgaaaagtg ccacctgacg tctaagaaac 3960

cattattatc atgacattaa cctataaaaa taggcgtatc acgaggccct ttcgtctcgc 4020

gcgtttcggt gatgacggtg aaaacctctg acacatgcag ctcccggaga cggtcacagc 4080

ttgtctgtaa gcggatgccg ggagcagaca agcccgtcag ggcgcgtcag cgggtgttgg 4140

cgggtgtcgg ggctggctta actatgcggc atcagagcag attgtactga gagtgcacca 4200

tatatgcggt gtgaaatacc gcacagatgc gtaaggagaa aataccgcat caggcgccat 4260

tcgccattca ggctgcgcaa ctgttgggaa gggcgatcgg tgcgggcctc ttcgctatta 4320

cgccagctgg cgaaaggggg atgtgctgca aggcgattaa gttgggtaac gccagggttt 4380

tcccagtcac gacgttgtaa aacgacggcc agtgccaagc ttggtctcaa ccattttaag 4440

agaggtaggt cttatataca ccgagtaagg cattaggtac gtatttttaa gacttctaac 4500

accgtcgatg tttagatcca tcacacaaat acgattatta atagccgctg tattcacagc 4560

tgttttagaa gttccgtaaa tatttcctaa aaactcagta tgttctagaa agtttccggc 4620

ggctattccc ttccagatgg cctctctgtt aacgtaatgg taatcaacac cttctcgttc 4680

cataggacga ggaaatctag tggtatggga caccacaaat ccaaatatat tcccatagtc 4740

ttttggctat ggctgtcttt cctaaaccag atggtccgct caatatgata gattttacta 4800

tcccagacat ttatgtaagt caaaaatcta gactttgttc tctgttttgt atttacgtga 4860

acgtttatta tatatataat atgtaataca gaaaattgcc acggccgaca atataattaa 4920

tgcggtaata ccaaatattt ctacaaagtc cttggtttta taattgctaa tagaggttgt 4980

actaccgcct acagtagttg gtggtactgt atcattatca ttgtacgtat cataaagatc 5040

cgcatcatcg gtggttgatt tagtagtgac aattccagat gatgtactta ctgtagtgta 5100

tgagacagtg tctgtaactg tatgatcttc tttatcagta attggttccg gagtctcgtc 5160

tgttgtggat tctccagatg atgcacttac tgtattaatg ctatcactag tgtatgtgac 5220

ggtgtctgta tgatcttcta cattatcagt aattggttcc ggagtcggaa ttgggc 5276

<210> 19
<211> 2272
<212> DNA
<213> Artificial sequence

<220>
<223> Secuencia extraído del plásmido pFastBac-VP1, por restricción con
      el enzima Sal I.

<400> 19
tcgactggca aagttcctca gagagaacgg ctacaaagtt ttgcagccgc ggtctctgcc 60

cgagaatgag gagtatgaga ccgaccaaat actcccagac ttagcatgga tgcgacagat 120

agaaggggct gttttaaaac ccactctatc tctccctatt ggagatcagg agtacttccc 180

aaagtactac ccaacacatc gccctagcaa ggagaagccc aatgcgtacc caccagacat 240

cgcactactc aagcagatga tttacctgtt tctccaggtt ccagaggcca acgagggcct 300

aaaggatgaa gtaaccctct tgacccaaaa cataagggac aaggcctatg gaagtgggac 360

ctacatggga caagcaaatc gacttgtggc catgaaggag gtcgccactg gaagaaaccc 420

aaacaaggat cctctaaagc ttgggtacac ttttgagagc atcgcgcagc tacttgacat 480

cacactaccg gtaggcccac ccggtgagga tgacaagccc tgggtgccac tcacaagagt 540

gccgtcacgg atgttggtgc tgacgggaga cgtagatggc gactttgagg ttgaagatta 600

ccttcccaaa atcaacctca agtcatcaag tggactacca tatgtaggtc gcaccaaagg 660

agagacaatt ggcgagatga tagctatatc aaaccagttt ctcagagagc tatcaacact 720

gttgaagcaa ggtgcaggga caaaggggtc aaacaagaag aagctactca gcatgttaag 780

tgactattgg tacttatcat gcgggctttt gtttccaaag gctgaaaggt acgacaaaag 840

tacatggctc accaagaccc ggaacatatg gtcagctcca tccccaacac acctcatgat 900

ctccatgatc acctggcccg tgatgtccaa cagcccaaat aacgtgttga acattgaagg 960

gtgtccatca ctctacaaat tcaacccgtt cagaggaggg ttgaacagga tcgtcgagtg 1020

gatattggcc ccggaagaac ccaaggctct tgtatatgcg gacaacatat acattgtcca 1080

ctcaaacacg tggtactcaa ttgacctaga gaagggtgag gcaaactgca ctcgccaaca 1140

catgcaagcc gcaatgtact acatactcac cagagggtgg tcagacaacg gcgacccaat 1200

gttcaatcaa acatgggcca cctttgccat gaacattgcc cctgctctag tggtggactc 1260

atcgtgcctg ataatgaacc tgcaaattaa gacctatggt caaggcagcg ggaatgcagc 1320

cacgttcatc aacaaccacc tcttgagcac gctagtgctt gaccagtgga acttgatgag 1380

acagcccaga ccagacagcg aggagttcaa atcaattgag gacaagctag gtatcaactt 1440

taagattgag aggtccattg atgatatcag gggcaagctg agacagcttg tcctccttgc 1500

acaaccaggg tacctgagtg gggggttgga accagaacaa tccagcccaa ctgttgagct 1560

tgacctacta gggtggtcag ctacatacag caaagatctc gggatctatg tgccggtgct 1620

tgacaaggaa cgcctatttt gttctgctgc gtatcccaag ggagtagaga acaagagtct 1680

caagtccaaa gtcgggatcg agcaggcata caaggtagtc aggtatgagg cgttaaggtt 1740

ggtaggtggt tggaactacc cactcctgaa caaagcctgc aagaataacg caggcgccgc 1800

tcggcggcat ctggaggcca aggggttccc actcgacgag ttcctagccg agtggtctga 1860

gctgtcagag ttcggtgagg ccttcgaagg cttcaatatc aagctgaccg taacatctga 1920

gagcctagcc gaactgaaca agccagtacc ccccaagccc ccaaatgtca acagaccagt 1980

caacactggg ggactcaagg cagtcagcaa cgccctcaag accggtcggt acaggaacga 2040

agccggactg agtggtctcg tccttctagc cacagcaaga agccgtctgc aagatgcagt 2100

taaggccaag gcagaagccg agaaactcca caagtccaag ccagacgacc ccgatgcaga 2160

ctggttcgaa agatcagaaa ctctgtcaga ccttctggag aaagccgaca tcgccagcaa 2220

ggtcgcccac tcagcactcg tggaaacaag cgacgccctt gaagcagttc ag 2272

<210> 20
<211> 8176
<212> DNA
<213> Artificial sequence

<220>
<223> Secuencia del plásmido pT-ReGS-B

<400> 20
cgatatatga attctaatac gactcactat aggatacgat gggtctgacc ctctgggagt 60

cacgaattaa cgtggctact aggggcgata cccgccgctg gccgccacgt tagtggctcc 120

tcttcttgat gattctgcca ccatgagtga cgttttcaat agtccacagg cgcgaagcac 180

gatctcagca gcgttcggca taaagcctac tgctggacaa gacgtggaag aactcttgat 240

ccctaaagtt tgggtgccac ctgaggatcc gcttgccagc cctagtcgac tggcaaagtt 300

cctcagagag aacggctaca aagttttgca gccgcggtct ctgcccgaga atgaggagta 360

tgagaccgac caaatactcc cagacttagc atggatgcga cagatagaag gggctgtttt 420
aaaacccact ctatctctcc ctattggaga tcaggagtac ttcccaaagt actacccaac 480
acatcgccct agcaaggaga agcccaatgc gtacccacca gacatcgcac tactcaagca 540
gatgatttac ctgtttctcc aggttccaga ggccaacgag ggcctaaagg atgaagtaac 600
cctcttgacc caaaacataa gggacaaggc ctatggaagt gggacctaca tgggacaagc 660
aaatcgactt gtggccatga aggaggtcgc cactggaaga aacccaaaca aggatcctct 720
aaagcttggg tacacttttg agagcatcgc gcagctactt gacatcacac taccggtagg 780
cccacccggt gaggatgaca agccctgggt gccactcaca agagtgccgt cacggatgtt 840
ggtgctgacg ggagacgtag atggcgactt tgaggttgaa gattaccttc ccaaaatcaa 900
cctcaagtca tcaagtggac taccatatgt aggtcgcacc aaaggagaga caattggcga 960
gatgatagct atatcaaacc agtttctcag agagctatca acactgttga agcaaggtgc 1020
agggacaaag gggtcaaaca agaagaagct actcagcatg ttaagtgact attggtactt 1080
atcatgcggg cttttgtttc caaaggctga aaggtacgac aaaagtacat ggctcaccaa 1140
gacccggaac atatggtcag ctccatcccc aacacacctc atgatctcca tgatcacctg 1200
gcccgtgatg tccaacagcc caaataacgt gttgaacatt gaagggtgtc catcactcta 1260
caaattcaac ccgttcagag gagggttgaa caggatcgtc gagtggatat tggccccgga 1320
agaacccaag gctcttgtat atgcggacaa catatacatt gtccactcaa acacgtggta 1380
ctcaattgac ctagagaagg gtgaggcaaa ctgcactcgc caacacatgc aagccgcaat 1440
gtactacata ctcaccagag ggtggtcaga caacggcgac ccaatgttca atcaaacatg 1500
ggccaccttt gccatgaaca ttgcccctgc tctagtggtg gactcatcgt gcctgataat 1560
gaacctgcaa attaagacct atggtcaagg cagcgggaat gcagccacgt tcatcaacaa 1620
ccacctcttg agcacgctag tgcttgacca gtggaacttg atgagacagc ccagaccaga 1680
cagcgaggag ttcaaatcaa ttgaggacaa gctaggtatc aactttaaga ttgagaggtc 1740
cattgatgat atcaggggca agctgagaca gcttgtcctc cttgcacaac cagggtacct 1800
gagtgggggg gttgaaccag aacaatccag cccaactgtt gagcttgacc tactagggtg 1860
gtcagctaca tacagcaaag atctcgggat ctatgtgccg gtgcttgaca aggaacgcct 1920
attttgttct gctgcgtatc ccaagggagt agagaacaag agtctcaagt ccaaagtcgg 1980
gatcgagcag gcatacaagg tagtcaggta tgaggcgtta aggttggtag gtggttggaa 2040
ctacccactc ctgaacaaag cctgcaagaa taacgcaggc gccgctcggc ggcatctgga 2100
ggccaagggg ttcccactcg acgagttcct agccgagtgg tctgagctgt cagagttcgg 2160
tgaggccttc gaaggcttca atatcaagct gaccgtaaca tctgagagcc tagccgaact 2220

gaacaagcca gtacccccca agcccccaaa tgtcaacaga ccagtcaaca ctgggggact 2280

caaggcagtc agcaacgccc tcaagaccgg tcggtacagg aacgaagccg gactgagtgg 2340

tctcgtcctt ctagccacag caagaagccg tctgcaagat gcagttaagg ccaaggcaga 2400

agccgagaaa ctccacaagt ccaagccaga cgaccccgat gcagactggt tcgaaagatc 2460

agaaactctg tcagaccttc tggagaaagc cgacatcgcc agcaaggtcg cccactcagc 2520

actcgtggaa acaagcgacg cccttgaagc agttcagtcg acttccgtgt acacccccaa 2580

gtacccagaa gtcaagaacc cacagaccgc ctccaacccc gttgttgggc tccacctgcc 2640

cgccaagaga gccaccggtg tccaggccgc tcttctcgga gcaggaacga gcagaccaat 2700

ggggatggag gccccaacac ggtccaagaa cgccgtgaaa atggccaaac ggcggcaacg 2760

ccaaaaggag agccgctaac agccatgatg ggaaccactc aagaagagga cactaatccc 2820

agaccccgta tccccggcct tcgcctgcgg gggcccccgg gtcggcatgg catctccacc 2880

tcctcgcggt ccgacctggg catccgaagg aggacgtcgt ccactcggat ggctaaggga 2940

gagctcggat ccggctgcta acaaagcccg aaaggaagct gagttggctg ctgccaccgc 3000

tgagcaataa ctagcataac cccttggggc ctctaaacgg gtcttgaggg gttttttgct 3060

gaaaggagga actatatccg gatcgcggcc gcagctccga tttcgaatac cgacgagcaa 3120

ttagaattat ctatataatc aggtttctta gaactagttt ccggtgaatg tgtagatcca 3180

gatagtatta tgtctatagt cgattcacta tctgtattta caatcaactc tgtggagtat 3240

tcttcataat ctactttatc agtgtcattt gtagttgatg tcataaagaa tgcacataca 3300

taagtaccgg catctctagc agtcaatgat ttaattgtga tagttgtaac tagatcatcg 3360

tatggagagt cgtaagatat tttatccttg gtataattat caaaatacaa gacgtcgctt 3420

ttagcagcta aaagaataat ggaattgggc tccttatacc aagcactcat aacaacgtag 3480

tcatttgtat tatttcgatt acatgataga gttgcatcat cacctatttt tttagatgtc 3540

tgaggaaaag gtgtagcgta tactaatgat attagtaaca aaagtattgg taatcgtgtc 3600

atattagtat aaaaagtgat ttatttttac aaaattatgt attttgttct atcaactacc 3660

tataaaactt tccaaatact ttagaaaatc attcgtgtac tgtgtttttt ccaatggata 3720

tttatgttta atcggttcgc atttatacat tatataattg cgtggtaaca cgtgaccatt 3780

atccaatgtc caacaatctt tttctttatc gtatgtttca gatgtattag ttcgaacatc 3840

atcagatatt cctcctccta catataattt attatgcgct gaccatagag cgggattaat 3900

tctaggaatg tttaatgatg ataatttggt ccattcatca tatatcggat tatagctaag 3960

gactagattc gatattacat taactacact actattgttg taacctccag atatataaat 4020

cttgccatca tgaacaatcg tcgaaattcg taatcatggt catagctgtt tcctgtgtga 4080

aattgttatc cgctcacaat tccacacaac atacgagccg gaagcataaa gtgtaaagcc 4140

tggggtgcct aatgagtgag ctaactcaca ttaattgcgt tgcgctcact gcccgctttc 4200

cagtcgggaa acctgtcgtg ccagctgcat taatgaatcg gccaacgcgc ggggagaggc 4260

ggtttgcgta ttgggcgctc ttccgcttcc tcgctcactg actcgctgcg ctcggtcgtt 4320

cggctgcggc gagcggtatc agctcactca aaggcggtaa tacggttatc cacagaatca 4380

ggggataacg caggaaagaa catgtgagca aaaggccagc aaaaggccag gaaccgtaaa 4440

aaggccgcgt tgctggcgtt tttccatagg ctccgccccc ctgacgagca tcacaaaaat 4500

cgacgctcaa gtcagaggtg gcgaaacccg acaggactat aaagatacca ggcgtttccc 4560

cctggaagct ccctcgtgcg ctctcctgtt ccgaccctgc cgcttaccgg atacctgtcc 4620

gcctttctcc cttcgggaag cgtggcgctt tctcatagct cacgctgtag gtatctcagt 4680

tcggtgtagg tcgttcgctc caagctgggc tgtgtgcacg aaccccccgt tcagcccgac 4740

cgctgcgcct tatccggtaa ctatcgtctt gagtccaacc cggtaagaca cgacttatcg 4800

ccactggcag cagccactgg taacaggatt agcagagcga ggtatgtagg cggtgctaca 4860

gagttcttga agtggtggcc taactacggc tacactagaa ggacagtatt tggtatctgc 4920

gctctgctga agccagttac cttcggaaaa agagttggta gctcttgatc cggcaaacaa 4980

accaccgctg gtagcggtgg tttttttgtt tgcaagcagc agattacgcg cagaaaaaaa 5040

ggatctcaag aagatccttt gatcttttct acggggtctg acgctcagtg gaacgaaaac 5100

tcacgttaag ggattttggt catgagatta tcaaaaagga tcttcaccta gatcctttta 5160

aattaaaaat gaagttttaa atcaatctaa agtatatatg agtaaacttg gtctgacagt 5220

taccaatgct taatcagtga ggcacctatc tcagcgatct gtctatttcg ttcatccata 5280

gttgcctgac tccccgtcgt gtagataact acgatacggg agggcttacc atctggcccc 5340

agtgctgcaa tgataccgcg agacccacgc tcaccggctc cagatttatc agcaataaac 5400

cagccagccg gaagggccga gcgcagaagt ggtcctgcaa ctttatccgc ctccatccag 5460

tctattaatt gttgccggga agctagagta agtagttcgc cagttaatag tttgcgcaac 5520

gttgttgcca ttgctacagg catcgtggtg tcacgctcgt cgtttggtat ggcttcattc 5580

agctccggtt cccaacgatc aaggcgagtt acatgatccc ccatgttgtg caaaaaagcg 5640

gttagctcct tcggtcctcc gatcgttgtc agaagtaagt tggccgcagt gttatcactc 5700

atggttatgg cagcactgca taattctctt actgtcatgc catccgtaag atgcttttct 5760

gtgactggtg agtactcaac caagtcattc tgagaatagt gtatgcggcg accgagttgc 5820

tcttgcccgg cgtcaatacg ggataatacc gcgccacata gcagaacttt aaaagtgctc 5880

atcattggaa aacgttcttc ggggcgaaaa ctctcaagga tcttaccgct gttgagatcc 5940

agttcgatgt aacccactcg tgcacccaac tgatcttcag catcttttac tttcaccagc 6000

gtttctgggt gagcaaaaac aggaaggcaa aatgccgcaa aaaagggaat aagggcgaca 6060

cggaaatgtt gaatactcat actcttcctt tttcaatatt attgaagcat ttatcagggt 6120

tattgtctca tgagcggata catatttgaa tgtatttaga aaaataaaca aataggggtt 6180

ccgcgcacat ttccccgaaa agtgccacct gacgtctaag aaaccattat tatcatgaca 6240

ttaacctata aaaataggcg tatcacgagg ccctttcgtc tcgcgcgttt cggtgatgac 6300

ggtgaaaacc tctgacacat gcagctcccg gagacggtca cagcttgtct gtaagcggat 6360

gccgggagca gacaagcccg tcagggcgcg tcagcgggtg ttggcgggtg tcggggctgg 6420

cttaactatg cggcatcaga gcagattgta ctgagagtgc accatatatg cggtgtgaaa 6480

taccgcacag atgcgtaagg agaaaatacc gcatcaggcg ccattcgcca ttcaggctgc 6540

gcaactgttg ggaagggcga tcggtgcggg cctcttcgct attacgccag ctggcgaaag 6600

ggggatgtgc tgcaaggcga ttaagttggg taacgccagg gttttcccag tcacgacgtt 6660

gtaaaacgac ggccagtgcc aagcttggtc tcaaccattt taagagaggt aggtcttata 6720

tacaccgagt aaggcattag gtacgtattt ttaagacttc taacaccgtc gatgtttaga 6780

tccatcacac aaatacgatt attaatagcc gctgtattca cagctgtttt agaagttccg 6840

taaatatttc ctaaaaactc agtatgttct agaaagtttc cggcggctat tcccttccag 6900

atggcctctc tgttaacgta atggtaatca acaccttctc gttccatagg acgaggaaat 6960

ctagtggtat gggacaccac aaatccaaat atattcccat agtcttttgg ctatggctgt 7020

ctttcctaaa ccagatggtc cgctcaatat gatagatttt actatcccag acatttatgt 7080

aagtcaaaaa tctagacttt gttctctgtt ttgtatttac gtgaacgttt attatatata 7140

taatatgtaa tacagaaaat tgccacggcc gacaatataa ttaatgcggt aataccaaat 7200

atttctacaa agtccttggt tttataattg ctaatagagg ttgtactacc gcctacagta 7260

gttggtggta ctgtatcatt atcattgtac gtatcataaa gatccgcatc atcggtggtt 7320

gatttagtag tgacaattcc agatgatgta cttactgtag tgtatgagac agtgtctgta 7380

actgtatgat cttctttatc agtaattggt tccggagtct cgtctgttgt ggattctcca 7440

gatgatgcac ttactgtatt aatgctatca ctagtgtatg tgacggtgtc tgtatgatct 7500

tctacattat cagtaattgg ttccggagtc ggaattgggc ggccgcatcg atagaaaaat 7560

tagcgaccgg agattggcgg gacgaatacg acgcccatat cccacggctg ttcaatccag 7620

gtatcttgcg ggatatcaac aacatagtca tcacccagcg gacgaccagc cggttttgcg 7680

aagatggtga caaagtgcgc ttttggatac atttcacgaa tcgcaaccgc agtaccaccg 7740

gtatccacca ggtcatcaat aacgatgaag ccttcgccat cgccttctgc gcgtttcagc 7800

actttaagct cgcgctggtt gtcgtgatcg tagctggaaa tacaaacggt atcgacatga 7860

cgaataccca gttcacgcgc cagtaacgca cccggtacca gaccgccacg gcttacggca 7920

ataatgcctt tccattgttc agaaggcatc agtcggcttg cgagtttacg tgcatggatc 7980

tgcaacatgt cccaggtgac gatgtatttt tcgctcatac ttccttaccg tgcaataaat 8040

tagaatatat tttctacttt tacgagaaat taattattgt atttattatt tatgggtgaa 8100

aaacttacta taaaaagcgg gtgggtttgg aattagtgat cgtcgatcga catggatctc 8160

gatcccgcga aatgat 8176

<210> 21
<211> 266
<212> PRT
<213> Artificial sequence

<220>
<223> Proteina de fusion GFP-Poliproteina IBDV

<400> 21

Met Thr Asn Leu Gln Asp Gln Thr Gln Gln Ile Val Pro Phe Ile Arg
1               5                   10                  15

Ser Leu Leu Met Pro Thr Thr Gly Pro Gly Met Ser Lys Gly Glu Glu
            20                  25                  30

Leu Phe Thr Gly Val Val Pro Ile Leu Val Glu Leu Asp Gly Asp Val
        35                  40                  45

Asn Gly His Lys Phe Ser Val Ser Gly Glu Gly Glu Gly Asp Ala Thr
    50                  55                  60

Tyr Gly Lys Leu Thr Leu Lys Phe Ile Cys Thr Thr Gly Lys Leu Pro
65                  70                  75                  80

Val Pro Trp Pro Thr Leu Val Thr Thr Leu Thr Tyr Gly Val Gln Cys
                85                  90                  95

Phe Ser Arg Tyr Pro Asp His Met Lys Gln His Asp Phe Phe Lys Ser
            100                 105                 110

Ala Met Pro Glu Gly Tyr Val Gln Glu Arg Thr Ile Phe Phe Lys Asp
        115                 120                 125

Asp Gly Asn Tyr Lys Thr Arg Ala Glu Val Lys Phe Glu Gly Asp Thr
    130                 135                 140

Leu Val Asn Arg Ile Glu Leu Lys Gly Ile Asp Phe Lys Glu Asp Gly
145                 150                 155                 160

Asn Ile Leu Gly His Lys Leu Glu Tyr Asn Tyr Asn Ser His Asn Val
                165                 170                 175

Tyr Ile Met Ala Asp Lys Gln Lys Asn Gly Ile Lys Val Asn Phe Lys
            180                 185                 190

Ile Arg His Asn Ile Glu Asp Gly Ser Val Gln Leu Ala Asp His Tyr
        195                 200                 205

Gln Gln Asn Thr Pro Ile Gly Asp Gly Pro Val Leu Leu Pro Asp Asn
    210                 215                 220

His Tyr Leu Ser Thr Gln Ser Ala Leu Ser Lys Asp Pro Asn Glu Lys
225                 230                 235                 240

Arg Asp His Met Val Leu Leu Glu Phe Val Thr Ala Ala Gly Ile Thr
                245                 250                 255

Leu Gly Met Asp Glu Leu Tyr Lys Ser Gly
            260                 265

<210>  22
<211>  282
<212>  PRT
<213>  Artificial sequence

<220>
<223>  Proteina de fusión ORF1A-ORF2 de IBDV

<400>  22

Met Val Ser Arg Asp Gln Thr Asn Asp Arg Ser Asp Asp Lys Pro Ala
1               5                   10                  15

Arg Ser Asn Pro Thr Asp Cys Ser Val His Thr Glu Pro Ser Asp Ala
            20                  25                  30

Asn Asn Arg Thr Gly Asp Glu Gln Gly Arg Gly Ala Val His Arg Gly
        35                  40                  45

Gly Ala His Pro Gly Arg Ala Gly Arg Arg Arg Lys Arg Pro Gln Val
    50                  55                  60

Gln Arg Val Arg Arg Gly Arg Gly Arg Cys His Leu Arg Gln Ala Asp
65                  70                  75                  80

Pro Glu Val His Leu His His Arg Gln Ala Ala Arg Ala Leu Ala His
                85                  90                  95

Pro Arg Asp His Pro Asp Leu Arg Arg Ala Val Leu Gln Pro Leu Pro
            100                 105                 110

Arg Pro His Glu Ala Ala Arg Leu Leu Gln Val Arg His Ala Arg Arg
        115                 120                 125

Leu Arg Pro Gly Ala His His Leu Leu Gln Gly Arg Arg Gln Leu Gln
    130                 135                 140

Asp Pro Arg Arg Gly Glu Val Arg Gly Arg His Pro Gly Glu Pro His
145                 150                 155                 160

Arg Ala Glu Gly His Arg Leu Gln Gly Gly Arg Gln His Pro Gly Ala
                165                 170                 175

Gln Ala Gly Val Gln Leu Gln Gln Pro Gln Arg Leu Tyr His Gly Arg
            180                 185                 190

Gln Ala Glu Glu Arg His Gln Gly Glu Leu Gln Asp Pro Pro Gln His
        195                 200                 205

Arg Gly Arg Gln Arg Ala Ala Arg Arg Pro Leu Pro Ala Glu His Pro
    210                 215                 220

His Arg Arg Arg Pro Arg Ala Ala Ala Arg Gln Pro Leu Pro Glu His
225                 230                 235                 240

Pro Val Arg Pro Glu Gln Arg Pro Gln Arg Glu Ala Arg Ser His Gly
                245                 250                 255

Pro Ala Gly Val Arg Asp Arg Arg Arg Asp His Ser Arg His Gly Arg
            260                 265                 270

Ala Val Gln Val Arg Ile Lys Ile Cys Ser
        275                 280

<210>  23
<211>  31
<212>  DNA
<213>  Artificial sequence

<220>
<223>  Cebador directo para amplificar la secuencia que codifida EGFP a
      partir de pEGFP-C1

<400>  23
cgcgcccggg atgagcaagg gcgaggagct g                                    31


<210>  24
<211>  36
<212>  DNA
<213>  Artificial sequence

<220>
<223>  Cebador reverso para amplificar EGFP a partir de pEGFP-C1

<400>  24
gcgcagatct gattatccgg acttgtacag ctcgtc                               36


<210>  25
<211>  739
<212>  DNA
<213>  Artificial sequence

<220>
<223>  Secuencia que codifica EGFP flanqueada por sitios SmaI y BglII

<400>  25
cgcgcccggg atgagcaagg gcgaggagct gttcaccggg gtggtgccca tcctggtcga      60

gctggacggc gacgtaaacg gccacaagtt cagcgtgtcc ggcgagggcg agggcgatgc     120

cacctacggc aagctgaccc tgaagttcat ctgcaccacc ggcaagctgc ccgtgccctg     180

gcccaccctc gtgaccaccc tgacctacgg cgtgcagtgc ttcagccgct accccgacca     240

catgaagcag cacgacttct tcaagtccgc catgcccgaa ggctacgtcc aggagcgcac     300

catcttcttc aaggacgacg gcaactacaa gacccgcgcc gaggtgaagt tcgagggcga     360

caccctggtg aaccgcatcg agctgaaggg catcgacttc aaggaggacg gcaacatcct     420

ggggcacaag ctggagtaca actacaacag ccacaacgtc tatatcatgg ccgacaagca     480

gaagaacggc atcaaggtga acttcaagat ccgccacaac atcgaggacg gcagcgtgca     540

gctcgccgac cactaccagc agaacacccc catcggcgac ggccccgtgc tgctgcccga     600

caaccactac ctgagcaccc agtccgccct gagcaaagac cccaacgaga agcgcgatca     660

catggtcctg ctggagttcg tgaccgccgc cgggatcact ctcggcatgg acgagctgta     720

caagtccgga taaagatct                                                  739


<210>  26
<211>  6523
<212>  DNA
<213>  Artificial sequence

<220>
<223>  Secuencia del plasmido pT-ReGS-AGFP

<400>  26
cgatatatga attctaatac gactcactat aggatacgat cggtctgacc ccgggggagt      60

cacccgggga caggtcttca aggccttgtt ccaggatgga actcctcctt ctacaacgct     120

atcattgatg gtcagtagag atcagacaaa cgatcgcagc gatgacaaac ctgcaagatc     180

aaacccaaca gattgttccg ttcatacgga gccttctgat gccaacaacc ggaccgggga     240

tgagcaaggg cgaggagctg ttcaccgggg tggtgcccat cctggtcgag ctggacggcg     300

acgtaaacgg ccacaagttc agcgtgtccg gcgagggcga gggcgatgcc acctacggca     360

agctgaccct gaagttcatc tgcaccaccg gcaagctgcc cgtgccctgg cccaccctcg     420

tgaccaccct gacctacggc gtgcagtgct tcagccgcta ccccgaccac atgaagcagc     480

acgacttctt caagtccgcc atgcccgaag gctacgtcca ggagcgcacc atcttcttca     540

aggacgacgg caactacaag acccgcgccg aggtgaagtt cgagggcgac accctggtga     600

accgcatcga gctgaagggc atcgacttca aggaggacgg caacatcctg gggcacaagc     660

tggagtacaa ctacaacagc cacaacgtct atatcatggc cgacaagcag aagaacggca     720

tcaaggtgaa cttcaagatc cgccacaaca tcgaggacgg cagcgtgcag ctcgccgacc     780

actaccagca gaacaccccc atcggcgacg gccccgtgct gctgcccgac aaccactacc     840

tgagcaccca gtccgccctg agcaaagacc ccaacgagaa gcgcgatcac atggtcctgc     900

tggagttcgt gaccgccgcc gggatcactc tcggcatgga cgagctgtac aagtccggat     960

aaagatctgc tcttgactgc gatggagatg aagcatcgca atcccaggcg ggctctacca    1020

aagcccaagc caaaacccaa tgctccaaca cagagacctc ctggtcggct gggccgctgg    1080

atcaggaccg tctctgatga ggaccttgag tgaggctcct gggagtctcc cgacaccacc    1140

cgcgcaggtg tggacaccaa ttcggcctta caacatccca aattggatcc gttcgcgggt    1200

cccctgggtc ggcatggcat ctccacctcc tcgcggtccg acctgggcat ccgaaggagg    1260

acgtcgtcca ctcggatggc taagggagag ctcggatccg gctgctaaca aagcccgaaa    1320

ggaagctgag ttggctgctg ccaccgctga gcaataacta gcataacccc ttggggcctc    1380

taaacgggtc ttgaggggtt ttttgctgaa aggaggaact atatccggat cgcggccgcc    1440

caattccgac tccggaacca attactgata atgtagaaga tcatacagac accgtcacat    1500

acactagtga tagcattaat acagtaagtg catcatctgg agaatccaca acagacgaga    1560

ctccggaacc aattactgat aaagaagatc atacagttac agacactgtc tcatacacta    1620

cagtaagtac atcatctgga attgtcacta ctaaatcaac caccgatgat gcggatcttt    1680

atgatacgta caatgataat gatacagtac caccaactac tgtaggcggt agtacaacct 1740

ctattagcaa ttataaaacc aaggactttg tagaaatatt tggtattacc gcattaatta 1800

tattgtcggc cgtggcaatt ttctgtatta catattatat atataataaa cgttcacgta 1860

aatacaaaac agagaacaaa gtctagattt ttgacttaca taaatgtctg ggatagtaaa 1920

atctatcata ttgagcggac catctggttt aggaaagaca gccatagcca aaagactatg 1980

ggaatatatt tggatttgtg gtgtcccata ccactagatt tcctcgtcct atggaacgag 2040

aaggtgttga ttaccattac gttaacagag aggccatctg gaagggaata gccgccggaa 2100

actttctaga acatactgag tttttaggaa atatttacgg aacttctaaa acagctgtga 2160

atacagcggc tattaataat cgtatttgtg tgatggatct aaacatcgac ggtgttagaa 2220

gtcttaaaaa tacgtaccta atgccttact cggtgtatat aagacctacc tctcttaaaa 2280

tggttgagac caagcttggc actggccgtc gttttacaac gtcgtgactg ggaaaaccct 2340

ggcgttaccc aacttaatcg ccttgcagca catccccctt tcgccagctg gcgtaatagc 2400

gaagaggccc gcaccgatcg cccttcccaa cagttgcgca gcctgaatgg cgaatggcgc 2460

ctgatgcggt attttctcct tacgcatctg tgcggtattt cacaccgcat atatggtgca 2520

ctctcagtac aatctgctct gatgccgcat agttaagcca gccccgacac ccgccaacac 2580

ccgctgacgc gccctgacgg gcttgtctgc tcccggcatc cgcttacaga caagctgtga 2640

ccgtctccgg gagctgcatg tgtcagaggt tttcaccgtc atcaccgaaa cgcgcgagac 2700

gaaagggcct cgtgatacgc ctatttttat aggttaatgt catgataata atggtttctt 2760

agacgtcagg tggcactttt cggggaaatg tgcgcggaac ccctatttgt ttatttttct 2820

aaatacattc aaatatgtat ccgctcatga gacaataacc ctgataaatg cttcaataat 2880

attgaaaaag gaagagtatg agtattcaac atttccgtgt cgcccttatt cccttttttg 2940

cggcattttg ccttcctgtt tttgctcacc cagaaacgct ggtgaaagta aaagatgctg 3000

aagatcagtt gggtgcacga gtgggttaca tcgaactgga tctcaacagc ggtaagatcc 3060

ttgagagttt tcgccccgaa gaacgttttc caatgatgag cacttttaaa gttctgctat 3120

gtggcgcggt attatcccgt attgacgccg ggcaagagca actcggtcgc cgcatacact 3180

attctcagaa tgacttggtt gagtactcac cagtcacaga aaagcatctt acggatggca 3240

tgacagtaag agaattatgc agtgctgcca taaccatgag tgataacact gcggccaact 3300

tacttctgac aacgatcgga ggaccgaagg agctaaccgc ttttttgcac aacatggggg 3360

atcatgtaac tcgccttgat cgttgggaac cggagctgaa tgaagccata ccaaacgacg 3420

agcgtgacac cacgatgcct gtagcaatgg caacaacgtt gcgcaaacta ttaactggcg 3480

aactacttac tctagcttcc cggcaacaat taatagactg gatggaggcg gataaagttg 3540

caggaccact tctgcgctcg gcccttccgg ctggctggtt tattgctgat aaatctggag 3600

ccggtgagcg tgggtctcgc ggtatcattg cagcactggg gccagatggt aagccctccc 3660

gtatcgtagt tatctacacg acggggagtc aggcaactat ggatgaacga aatagacaga 3720

tcgctgagat aggtgcctca ctgattaagc attggtaact gtcagaccaa gtttactcat 3780

atatacttta gattgattta aaacttcatt tttaatttaa aaggatctag gtgaagatcc 3840

tttttgataa tctcatgacc aaaatccctt aacgtgagtt ttcgttccac tgagcgtcag 3900

accccgtaga aaagatcaaa ggatcttctt gagatccttt ttttctgcgc gtaatctgct 3960

gcttgcaaac aaaaaaacca ccgctaccag cggtggtttg tttgccggat caagagctac 4020

caactctttt tccgaaggta actggcttca gcagagcgca gataccaaat actgtccttc 4080

tagtgtagcc gtagttaggc caccacttca agaactctgt agcaccgcct acatacctcg 4140

ctctgctaat cctgttacca gtggctgctg ccagtggcga taagtcgtgt cttaccgggt 4200

tggactcaag acgatagtta ccggataagg cgcagcggtc gggctgaacg gggggttcgt 4260

gcacacagcc cagcttggag cgaacgacct acaccgaact gagataccta cagcgtgagc 4320

tatgagaaag cgccacgctt cccgaaggga gaaaggcgga caggtatccg gtaagcggca 4380

gggtcggaac aggagagcgc acgagggagc ttccaggggg aaacgcctgg tatctttata 4440

gtcctgtcgg gtttcgccac ctctgacttg agcgtcgatt tttgtgatgc tcgtcagggg 4500

ggcggagcct atggaaaaac gccagcaacg cggccttttt acggttcctg gccttttgct 4560

ggccttttgc tcacatgttc tttcctgcgt tatcccctga ttctgtggat aaccgtatta 4620

ccgcctttga gtgagctgat accgctcgcc gcagccgaac gaccgagcgc agcgagtcag 4680

tgagcgagga agcggaagag cgcccaatac gcaaaccgcc tctccccgcg cgttggccga 4740

ttcattaatg cagctggcac gacaggtttc ccgactggaa agcgggcagt gagcgcaacg 4800

caattaatgt gagttagctc actcattagg caccccaggc tttacacttt atgcttccgg 4860

ctcgtatgtt gtgtggaatt gtgagcggat aacaatttca cacaggaaac agctatgacc 4920

atgattacga atttcgacga ttgttcatga tggcaagatt tatatatctg gaggttacaa 4980

caatagtagt gtagttaatg taatatcgaa tctagtcctt agctataatc cgatatatga 5040

tgaatggacc aaattatcat cattaaacat tcctagaatt aatcccgctc tatggtcagc 5100

gcataataaa ttatatgtag gaggaggaat atctgatgat gttcgaacta atacatctga 5160

aacatacgat aaagaaaaag attgttggac attggataat ggtcacgtgt taccacgcaa 5220

ttatataatg tataaatgcg aaccgattaa acataaatat ccattggaaa aaacacagta 5280

cacgaatgat tttctaaagt atttggaaag ttttataggt agttgataga acaaaataca 5340

taattttgta aaaataaatc actttttata ctaatatgac acgattacca atacttttgt 5400

tactaatatc attagtatac gctacacctt ttcctcagac atctaaaaaa ataggtgatg 5460

atgcaactct atcatgtaat cgaaataata caaatgacta cgttgttatg agtgcttggt 5520

ataaggagcc caattccatt attcttttag ctgctaaaag cgacgtcttg tattttgata 5580

attataccaa ggataaaata tcttacgact ctccatacga tgatctagtt acaactatca 5640

caattaaatc attgactgct agagatgccg gtacttatgt atgtgcattc tttatgacat 5700

caactacaaa tgacactgat aaagtagatt atgaagaata ctccacagag ttgattgtaa 5760

atacagatag tgaatcgact atagacataa tactatctgg atctacacat tcaccggaaa 5820

ctagttctaa gaaacctgat tatatagata attctaattg ctcgtcggta ttcgaaatcg 5880

gagctgcggc cgcatcgata gaaaaattag cgaccggaga ttggcgggac gaatacgacg 5940

cccatatccc acggctgttc aatccaggta tcttgcggga tatcaacaac atagtcatca 6000

cccagcggac gaccagccgg ttttgcgaag atggtgacaa agtgcgcttt tggatacatt 6060

tcacgaatcg caaccgcagt accaccggta tccaccaggt catcaataac gatgaagcct 6120

tcgccatcgc cttctgcgcg tttcagcact ttaagctcgc gctggttgtc gtgatcgtag 6180

ctggaaatac aaacggtatc gacatgacga atacccagtt cacgcgccag taacgcaccc 6240

ggtaccagac cgccacggct tacggcaata atgcctttcc attgttcaga aggcatcagt 6300

cggcttgcga gtttacgtgc atggatctgc aacatgtccc aggtgacgat gtatttttcg 6360

ctcatacttc cttaccgtgc aataaattag aatatatttt ctacttttac gagaaattaa 6420

ttattgtatt tattatttat gggtgaaaaa cttactataa aaagcgggtg ggtttggaat 6480

tagtgatcgt cgatcgacat ggatctcgat cccgcgaaat gat 6523

## Description

### Technical Field of the Invention

The invention belongs to the biotechnology field and, more specifically, to methods for carrying a gene of interest to a target cell by means of using genetically modified viral capsids and virions based on RNA viruses which require an RNA polymerase for their replication and which include a gene of interest. The modified viral capsids and virions can infect a target cell, giving rise to the expression of said gene in said target cell, so they can be used as a vaccine or for the treatment of diseases in which there is a deficiency in the expression of a gene.

### Background of the Invention

The development of molecular biology techniques has allowed conducting systematic studies aimed at characterizing at the molecular level the mechanisms which direct and control the assembly process of different types of macromolecules. Studies of this type have been especially successful in the characterization of macromolecular assemblies essential for the correct functioning of the replicative cycle of a large number of viruses. The study of the assembly processes of viral capsids has given rise to the isolation of proteins capable of self-assembling to give rise to the formation of stable structures generically referred to as empty viral particles or virus-like particles (VLPs) (Kingsman and Kingsman, 1988; Murray and Eaton, 1996, Casal et *al.,* 1999; Maranga et *al.,* 2002). VLPs obtained by means of the expression of a large number of viral proteins have been successfully used in the development of non-infective vaccines (subunit) against various diseases (Yao et *al.,* 2003; Maclean et *al.,* 2005; Hammonds et *al.,* 2007).

However, a limitation of the systems based on VLPs known to date is that they do not allow including heterologous genetic material therein in a transcriptionally active form, i.e., such that said genetic material can be expressed once it is inside the infected cells.

Thus, US6528063 describes virus-like particles formed from the expression in transgenic plants of the infectious bursal disease virus (IBDV) VP2, VP3 and VP4 proteins. However, this document does not describe the possibility of the virus-like particles being able to encapsidate genetic material.

WO980964 describes a method for generating non-pathogenic infective IBDV particles by means of the introduction in permissive cells of synthetic transcripts obtained *in vitro* from cDNAs corresponding to segments A and B of the IBDV genome. However, this document does not describe the possibility of introducing heterologous genetic material in said IBDV particles.

WO0507106 describes the formation of virus-like particles from the expression in insect cells of the infectious bursal disease virus (IBDV) VP2 and VP3 proteins. However, these virus-like particles are empty so they are not useful for the administration of genetic material.

WO0408790 describes a method for generating virus-like particles from the expression in insect cells of the IBDV polyprotein and of the VP1 protein. However, this document does not describe the possibility of the virus-like particles being able to encapsidate genetic material.

In this sense, the availability of particles capable of housing genetic material and of methods for obtaining them in *vivo* or *in vitro* would enormously facilitate the treatments based on gene therapies since there would be no limitation in relation to the type of agent which could be administered in the particles. Different methods for obtaining virus-like particles containing genetic material have been described to date.

For example, WO08024427 describes virus-like particles based on an RNA bacteriophage which encapsidates a transcriptional unit therein. However, these particles are based on structural proteins of a bacteriophage so they can only be used for the administration of said genetic material to prokaryotic cells, unless the capsid proteins are modified by means of the insertion of an epitope allowing the interaction of the virus-like particles with surface proteins present in the target cells.

WO0408493 describes virus-like particles having the capacity to incorporate an immunomodulator compound such as oligonucleotides. However, this system requires the oligonucleotides to be encapsidated *in vitro* by means of contacting said oligonucleotides with the virus-like particles, so it is only suitable for the encapsidation of low molecular weight compounds.

WO991563 describes virus-like particles obtained by means of the expression in insect cells of the HPV-16 L1 and L2 proteins. These particles can be dissociated and reassembled in *vitro* in the presence of a genetic material, giving rise to particles comprising genetic material and which can be used to transfer a gene of interest to a target cell. However, the expression of the genetic material included in the microparticles in the target cell requires cell transcription machinery, so in those cases in which the cell is altered or its transcription machinery is damaged, it is possible that the encapsulated material will not be expressed.

Therefore, there is a need in the art for methods for preparing virus-like particles which comprise a heterologous genetic material and which are transcriptionally active, i.e., which allow the expression of said genetic material in the target cell independently from the transcriptional capacity of the cell.

### Summary of the Invention

In a first aspect, the invention relates to a polynucleotide comprising in the 5' to 3' direction:
(a) a first sequence comprising a non-translated region of a segment of the genomic RNA of a birnavirus or a functionally equivalent variant thereof,
(b) a second sequence encoding a heterologous protein and
(c) a third sequence comprising a non-translated region of a segment of the genomic RNA of said birnavirus
   or a functionally equivalent variant thereof wherein the first and/or the third sequence allow or allows the encapsidation of said polynucleotide inside a viral particle.

In successive aspects, the invention relates to a vector comprising a polynucleotide of the invention and to a host cell comprising a polynucleotide of the invention or a vector according to the invention.

In another aspect, the invention relates to a method for obtaining transcriptionally active virus-like particles which comprises the steps of:
(i) contacting a cell with a first polynucleotide under conditions suitable for the entry of said polynucleotide in the cell, wherein said polynucleotide comprises in the 5' to 3' direction
   (a) a first sequence comprising a non-translated region of a segment of the genomic RNA of an RNA virus requiring an RNA polymerase for its replication or a functionally equivalent variant thereof,
   (b) a second sequence encoding a heterologous product of therapeutic interest and
   (c) a third sequence comprising a non-translated region of a segment of the genomic RNA of said RNA virus or a functionally equivalent variant thereof
(ii) contacting said cell with one or several additional polynucleotides under conditions suitable for the entry of said polynucleotide or said polynucleotides in the cell and wherein said polynucleotide or said polynucleotides comprise sequences encoding the RNA polymerase of said RNA virus from which the non-translated sequence forming part of the first polynucleotide is derived sequences encoding all the proteins necessary for the encapsidation of said RNA virus,
(iii) maintaining the cells under conditions suitable for the expression of the polynucleotides introduced in the cell in steps (i) and (ii) and
(iv) recovering the virus-like particles from the culture
obtained in step (iii)
wherein steps (i) and (ii) are carried out in any order or simultaneously.

In another aspect, the invention relates to a virus-like particle obtained by means of a method according to the invention.

In another aspect, the invention relates to a method for obtaining recombinant virions containing a heterologous genomic segment which comprises the steps of:
(i) contacting a cell with a polynucleotide under conditions suitable for the entry of said polynucleotide in the cell, wherein said polynucleotide comprises in the 5' to 3' direction
   (a) a first sequence comprising a non-translated region of a segment of the genomic RNA of an RNA virus requiring an RNA polymerase for its replication or a functionally equivalent variant thereof,
   (b) a second sequence encoding a heterologous product and
   (c) a third sequence comprising a non-translated region of a segment of the genomic RNA of said RNA virus or a functionally equivalent variant thereof
(ii) contacting said cell with an RNA virus from which the non-translated regions used in the polynucleotide used in step (i) are derived,
(iii) maintaining the cells under conditions suitable for the expression of the polynucleotide introduced in the cell in step (i) and for the expression of the genes of the RNA virus introduced in step (ii) and
(iv) recovering the recombinant virions from the culture obtained in step (iii)
wherein steps (i) and (ii) are carried out in any order or simultaneously.

In another aspect, the invention relates to a recombinant virion obtained by means of a method of the invention.

In another aspect, the invention relates to an *in vitro* method for expressing a protein of therapeutic interest in a cell which comprises contacting said cell with a virus-like particle or with a recombinant virion according to the invention.

Additional aspects of the invention include a virus-like particle or a recombinant virion of the invention for its use in medicine; a vaccine comprising a virus-like particle or a recombinant virion according to the invention, or a virus-like particle or a recombinant virion according to the invention for its use in the treatment of an infectious disease caused by the pathogenic organism from which the antigen is derived.

### Brief Description of the Drawings

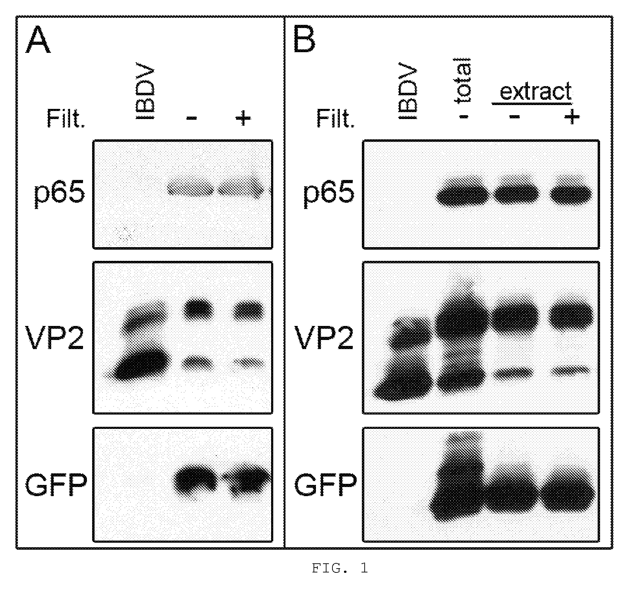

Figure 1. Biochemical characterization of the expression of proteins in cells coinfected with VT7/Poly, VT7/VP1, VT7/TReGS-A-GFP. Analysis by means of SDS-PAGE and WB of the expression in (A) extracellular extracts and (B) intracellular extracts before (-) and after (+) being filtered through cellulose membranes with a pore size of 0.1 µm. Furthermore, in the case of the characterization of intracellular extracts, the cell pellets were loaded without lysing (total). Finally, purified IBDV particles were loaded as a control of the pVP2/VP2 processing. The gels were electrotransferred to nitrocellulose membranes and immunodetected with anti-p65 (top), anti-VP2 (middle) or anti-GFP (bottom) antibodies.

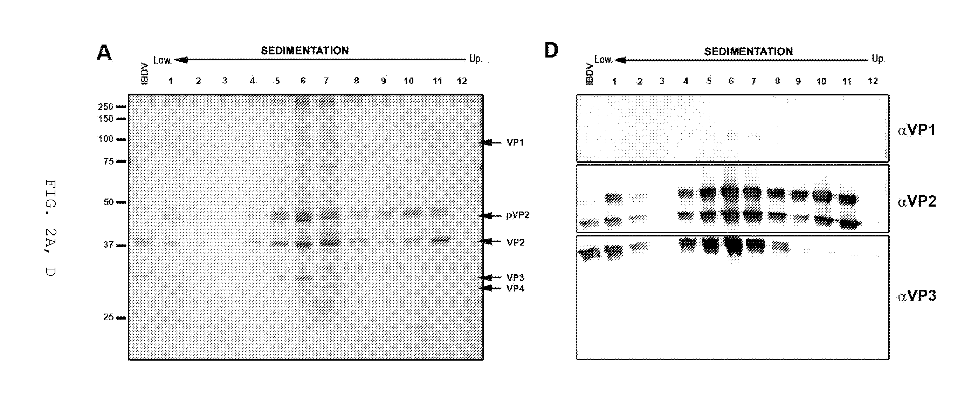

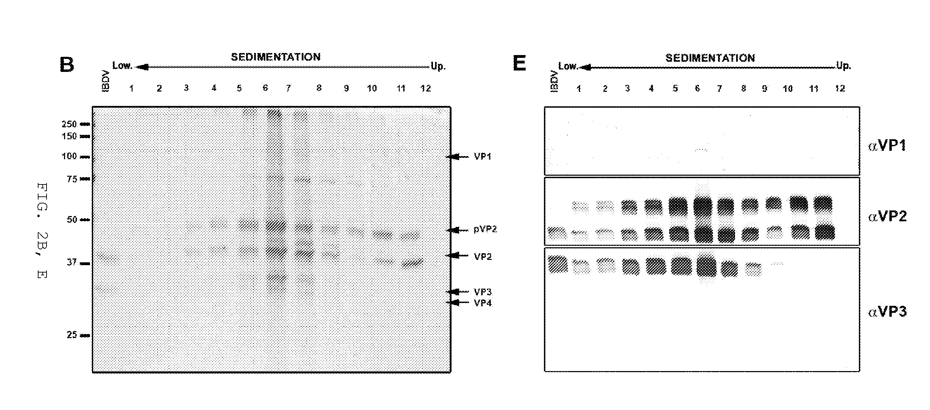

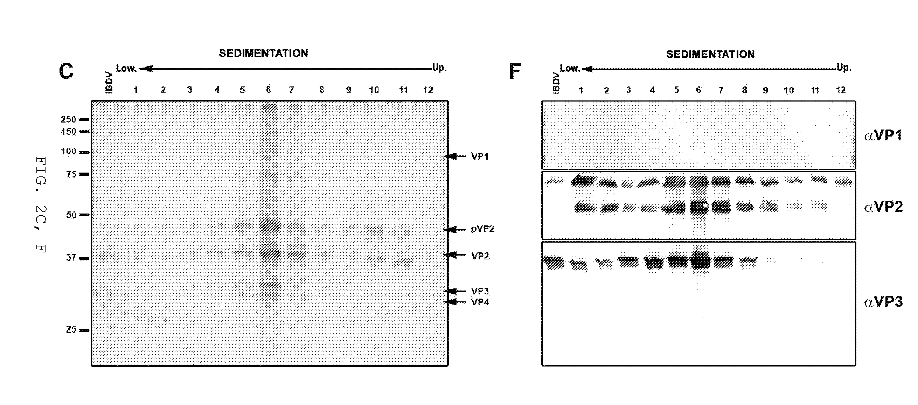

Figure 2. Biochemical analysis of purified assemblies from the coinfection of qm7 cells with rVV VT7/Poly, VT7/VP1 in the presence and absence of VT7/TReGS-A or VT7/TReGS-A-GFP. Analysis by means of SDS-PAGE and (A-C) Coomassie blue staining or (D-F) electrotransfer and immunodetection with anti-VP1 (top), anti-VP2 (middle) or anti-VP3 (bottom) polyclonal sera of fractions of the sucrose gradient after being concentrated by ultracentrifugation. The samples analyzed were: (A and D) VT7/Poly + VT7/VP1, (B and E) VT7/Poly + VT7/VP1 + VT7/TReGS-A and (C and F) VT7/Poly + VT7/VP1 + VT7/TReGS-A-GFP. Purified IBDV virions (right lane of each gel) were used as a positive control. (A-C) The molecular weight markers are indicated at the left of each gel and the positions of the VP1, pVP2, VP2, VP3 and VP4 proteins are indicated at the right. (D-E) The polyclonal serum with which the membrane has been immunodetected is indicated at the right of each gel.

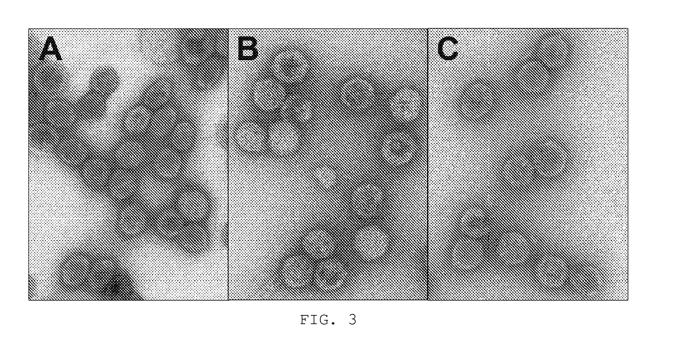

Figure 3. Analysis by electron microscopy of VLPs assembled from the coinfection of qm7 cells with rVV VT7/Poly, VT7/VP1 in the presence and absence of VT7/TReGS-A or VT7/TReGS-A-GFP. (A) VT7/Poly + VT7/VP1, (B) VT7/Poly + VT7/VP1 + VT7/TReGS-A and (C) VT7/TReGS-A-GFP. The images shown correspond to the migration maximum (fraction 6) of each of the coinfections.

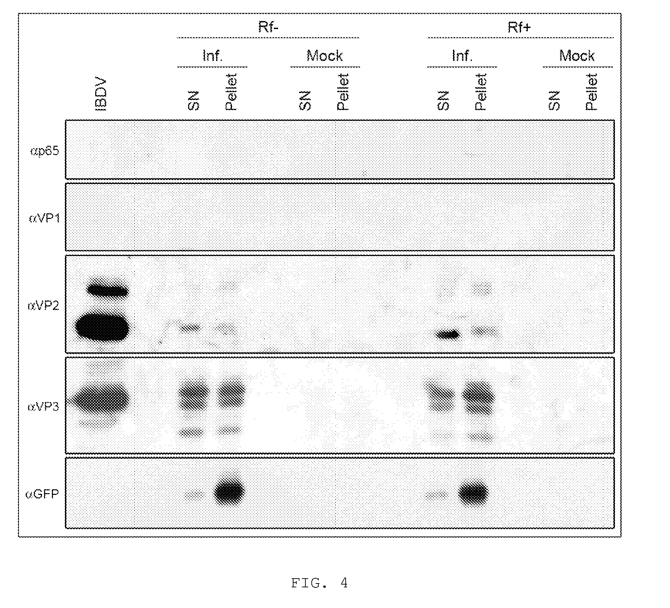

Figure 4. Biochemical analysis of the transduction of QM7 cells with extracts from the VT7/Poly + VT7/VP1 + VT7/TReGS-A-GFP coinfection. Cultures of QM7 cells transduced both with extracellular (SN) and intracellular (Pellet) extracts of cells coinfected with rVV VT7/Poly + VT7/VP1 + VT7/TReGS-A-GFP were analyzed by means of SDS-PAGE, electrotransfer to nitrocellulose membranes and immunodetection. The transductions were performed in the presence (Rf+) and absence of rifampicin (Rf-). Purified IBDV virions (left lane of each gel) were used as a control. The monoclonal antibody (anti-p65 and anti-GFP) or polyclonal serum (anti-VP1, anti-VP2 and anti-VP3) used to immunodetect each membrane is indicated at the left.

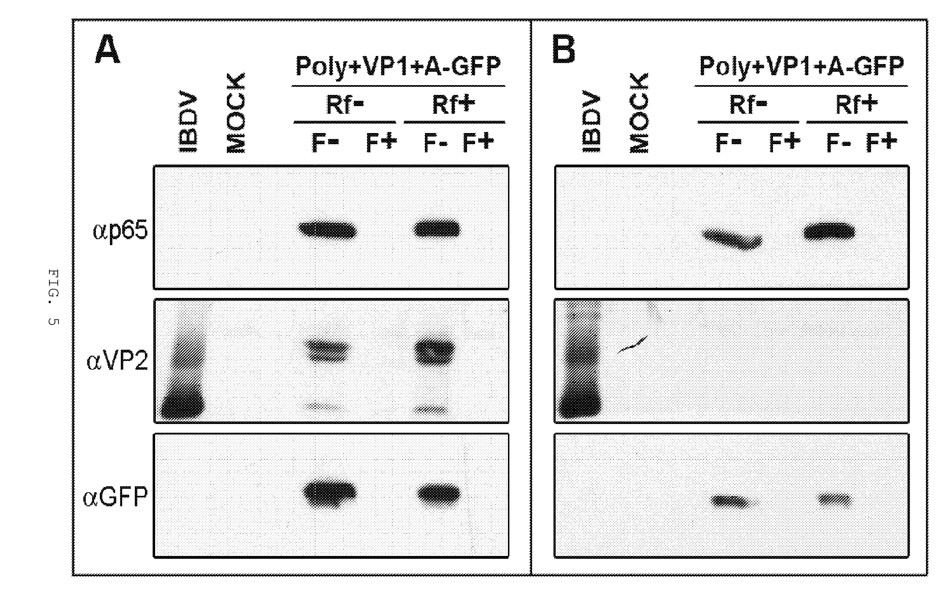

Figure 5. Biochemical analysis of cells reinfected with supernatants of transduced cells. The presence of vaccinia virus in the supernatants of transduced cells, with extracellular (A) or intracellular (B) extracts, was analyzed by means of SDS-PAGE of extracts of cells contacted therewith, followed by anti-p65 (top), anti-VP2 (middle) or anti-GFP (bottom) immunodetection and electrotransfer. It is indicated if the previous transduction was performed in the absence (Rf-) or presence (Rf+) of rifampicin, as well as if, once the extracts were prepared, they were filtered (F+) or not (F-). Purified IBDV virions (IBDV) and non-infected cells (MOCK) were used as a control.

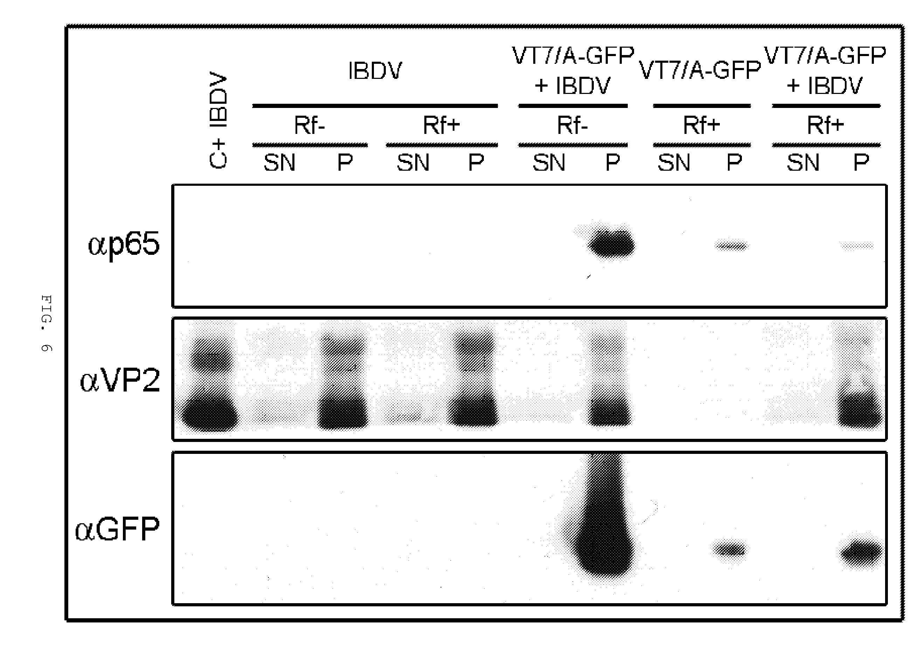

Figure 6. Biochemical characterization of the IBDV + VT7/TReGS-A-GFP coinfection. Analysis by means of SDS-PAGE, electrotransfer and immunodetection of extracellular (SN) or intracellullar (P) extracts of cultures of QM7 cells infected with IBDV, with rVV VT7/TReGS-A-GFP (VT7/A-GFP) or coinfected with both viruses (VT7/A-GFP + IBDV) in the absence (Rf-) or presence (Rf+) of rifampicin. Purified IBDV virions (C+ IBDV) were used as a control. The antibody with which each membrane was immunodetected is indicated at the left of each gel.

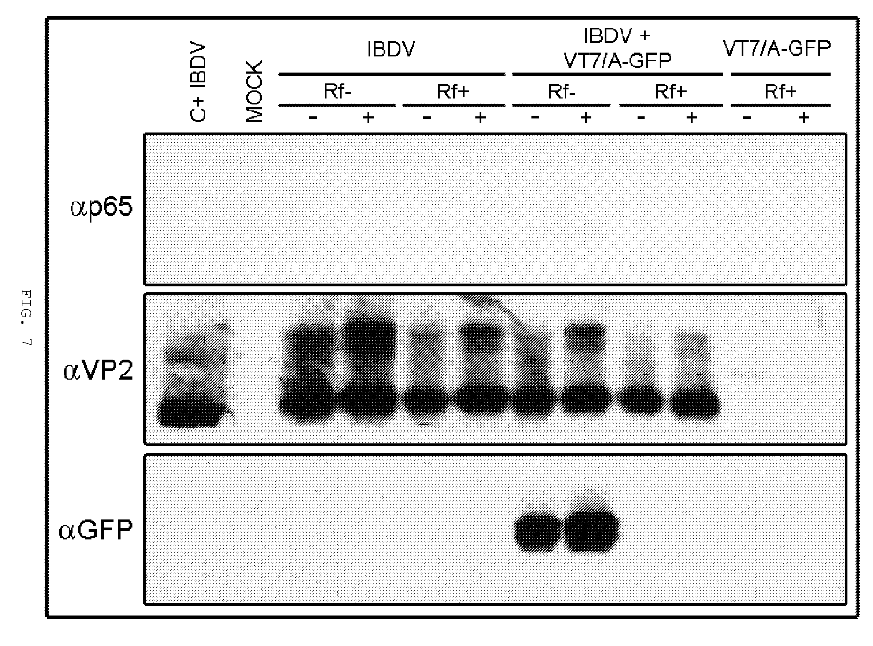

Figure 7. Biochemical characterization of cells infected with extracts of the IBDV + VT7/TReGS-A-GFP coinfection. Analysis by means of SDS-PAGE, electrotransfer and immunodetection of cells infected in the presence (Rf+) and absence (Rf-) of rifampicin with intracellular extracts of cultures of QM7 cells infected with IBDV, with rVV VT7/TReGS-A-GFP (VT7/A-GFP) or coinfected with both viruses (VT7/A-GFP + IBDV) in the absence (-) or presence (+) of rifampicin. Purified IBDV virions (C+ IBDV) and non-infected cells (MOCK) were used as controls.

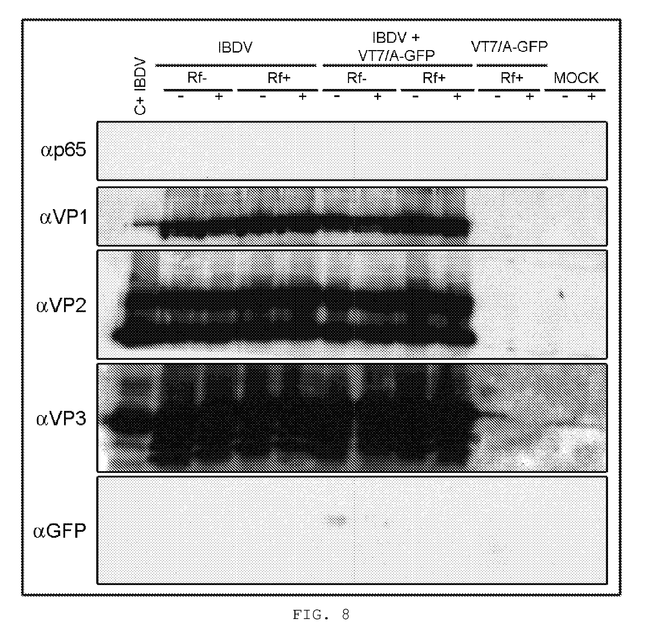

Figure 8. Biochemical characterization of cells treated with supernatants of cells incubated with extracts of cells coinfected with IBDV + VT7/TReGS-A-GFP. Analysis by means of SDS-PAGE, electrotransfer and immunodetection of cells treated with the supernatant of cells infected in the presence (Rf+) and absence (Rf-) of rifampicin with intracellular extracts of cell cultures infected with IBDV, rVV VT7/TReGS-A-GFP (VT7/A-GFP) or coinfected with both viruses (VT7/A-GFP + IBDV) in the absence (-) or presence (+) of rifampicin. Purified IBDV virions (C+ IBDV) and non-infected cells (MOCK) in the presence (+) or absence (-) of rifampicin were used as controls.

### Detailed Description of the Invention

The authors of the present invention have surprisingly shown that the presence in cells of the capsid proteins and the RNA polymerase of a birnavirus and of a gene encoding a protein of therapeutic interest flanked by the non-translated regions of a genomic segment of said birnavirus gives rise to the production of virus-like particles comprising said RNA polymerase and said gene of interest in the form of double-stranded RNA. Said virus-like particles are transcriptionally active since the subsequent infection of target cells with the virus-like particles results in the transcription of the double-stranded RNA by the RNA-dependent RNA polymerase of the birnavirus, giving rise to the expression of the gene of therapeutic interest.

### Polynucleotide of the invention

Thus, in a first aspect, the invention relates to a polynucleotide comprising in the 5' to 3' direction
(a) a first sequence comprising a non-translated region of a segment of the genomic RNA of a birnavirus or a functionally equivalent variant thereof,
(b) a second sequence encoding a heterologous protein and
(c) a third sequence comprising a non-translated region of a segment of the genomic RNA of said birnavirus
or a functionally equivalent variant thereof wherein the first and/or the third sequence allow or allows the encapsidation of said segment inside the viral particle.

Sequence (i) of the polynucleotide of the invention corresponds to the non-translated region of a segment of genomic RNA of a birnavirus or a functionally equivalent variant thereof. The polynucleotide of the invention can be single-stranded DNA, double-stranded DNA, single-stranded RNA and double-stranded RNA. In the event that the polynucleotide of the invention is single-stranded DNA, sequence (a) is the DNA sequence complementary to the sequence of the (-) strand present in the non-translated 5' region of a segment of the birnavirus genome. In the event that the polynucleotide of the invention is double-stranded DNA, sequence (a) corresponds to the non-translated 5' region of a segment of the birnavirus genome but wherein the uracil residues are thymidine residues. In the event that the polynucleotide is single-stranded RNA, sequence (a) comprises the sequence of the (+) strand corresponding to the non-translated 5' region of a segment of the birnavirus genome. In the event that the polynucleotide of the invention is double-stranded RNA, sequence (a) corresponds to the non-translated 5' region of a segment of the birnavirus double-stranded RNA genome.

The non-translated sequences of the birnavirus genome are known for the person skilled in the art. Thus, the invention contemplates the use of a non-translated 5' region from segment A or from segment B of the infectious bursal disease virus (IBDV) or of the infectious pancreatic necrosis virus (IPNV) genome. In a preferred embodiment, sequence (i) in the polynucleotide of the invention is from the non-translated 5' region of segment A of the IBDV genome (SEQ ID NO:1).

The invention contemplates the use of functionally equivalent variants of the non-translated sequence of the genome of a birnavirus. In the context of the present invention, a "functionally equivalent variant" of the non-translated 5' region of a segment of the birnavirus genome is understood as any sequence having additions, substitutions, deletions or combinations thereof in its nucleotide sequence and/or which has been chemically modified with respect to said sequence and which substantially maintains the function of the non-translated regulatory sequences, particularly the capacity to promote the replication of the sequences located in the 3' direction mediated by the RNA-dependent RNA polymerase, the capacity to promote the translation of the sequences located in the 3' direction and/or the capacity to promote the encapsidation of nucleic acids containing said regulatory sequences in the virus-like particles of the birnavirus. Suitable assays for determining the capacity of a determined sequence to promote the transcription or the expression of sequences located in the 3' direction are, for example, the assay described by Nagarajan and Kibenge (J. Virol. Methods., 1998 72:51-58) as well as the assay described in Example 2 attached to the present description. Preferably, the variants of the non-translated 5' region of the birnavirus show said capacity to regulate the transcriptional expression of the nucleotide sequence operatively coupled in the 3' position at least by 60%, preferably by 70%, advantageously by 80%, more preferably by 90%, more preferably by 95%, even more preferably by 97% and even more preferably by 98%, advantageously by 99%.

The variants of the sequence of the proximal promoter identified in SEQ ID NO:1 can be obtained by means of various methods known in the state of the art. Said methods include but are not limited to: the isolation from a natural source, in the event that said variations in the nucleotide sequences occur naturally, and the obtaining by mutagenesis, which can be oligonucleotide-mediated mutagenesis (or directed mutagenesis), PCR mutagenesis and cassette mutagenesis of a previously prepared variant or non-variant.

In a particular embodiment, the method chosen for preparing said variants, which have mutations of substitution and/or of deletion of one or more nucleotides, is oligonucleotide-mediated mutagenesis. This technique is well known in the state of the art (Adelman JP et al, (1983) DNA;2:183-93.). In oligonucleotide-mediated mutagenesis, the DNA sequence of the native promoter is altered by means of the hybridization of an oligonucleotide, encoding the desired mutation, with a template DNA. Said template DNA consists of a single-stranded DNA of a plasmid or a bacteriophage which contains the native or unaltered sequence. After the hybridization, a DNA polymerase enzyme is used to completely synthesize the second complementary strand of the template which will thus incorporate the selected mutation in the DNA of the native sequence. Said oligonucleotides usually have a length of at least 25 nucleotides. An optimized oligonucleotide should have between 12 and 15 nucleotides completely complementary to the template DNA on both sides of the mutation, such that it is assured that the oligonucleotide will suitably hybridize to the template of single-stranded DNA. The nucleotides are synthesized using techniques known in the state of the art. Alternatively, it is possible to introduce mutations by means of error-prone PCR, as has been described by Ausubel, F.M. et al., (Current Protocols in Molecular Biology, John Wiley & Sons Inc; ringbou edition, 2003).

In addition, from the sequence of the non-translated 5' region of a segment of the genome of a birnavirus, non-translated 5' regions can be obtained from the genomes in other birnavirus species as a result of the existing general knowledge of molecular biology techniques. All these nucleotide sequences homologous to the one described in the present invention and which substantially maintain the capacity to regulate the transcriptional expression of the nucleotide sequence operatively coupled in the 3' position of said sequence can be used in the present invention. In the context of the present invention, homologous sequences are understood as those nucleotide sequences having a degree of similarity of at least 80% with the nucleotide sequences of the promoter of the present invention, preferably, those having a degree of similarity of at least 85%, more preferably, those having a degree of similarity of at least 90% with the nucleotide sequences of the promoter of the present invention, and even more preferably those having a degree of similarity of at least 95%. The algorithms for analyzing sequences are known in the state of the art, such as BLAST for example, described in Altschul et al. (1990) (J Mol Biol. Oct 5;215(3):403-10).

Within the context of the present invention, those nucleotide sequences which hybridize with the nucleotide sequence under severe hybridization conditions with the non-translated 5' regions of segments A or B of the birnaviruses known to date are also considered as "variants of the non-encoded 5' regions". The conditions under which completely complementary nucleic acid strands hybridize are referred to as "very severe hybridization conditions" or "sequence-specific hybridization conditions". Nevertheless, substantially complementary stable nucleic acid double strands can be obtained under less severe hybridization conditions, generically referred to as "severe hybridization conditions", in which case the tolerated degree of mismatch can be adjusted by means of the suitable adjustment of the hybridization conditions. The person skilled in the art can empirically determine the stability of a duplex by taking into account various variables, such as the length and concentration of base pairs of the probes, the ionic strength and the incidence of the mismatched base pairs, following the guidelines of the state of the art (see, for example, Sambrook et al., Molecular Cloning: A Laboratory Manual, Cold Spring Harbor Laboratory, Cold Spring Harbor, N. Y. (1989); and Wetmur, Critical Reviews in Biochem. and Mol. Biol. 26 (3/4):227-259 (1991)). By way of illustration, in a particular embodiment, the severe hybridization conditions comprise, for example, a concentration comprised between 0.15 M and 0.9 M of NaCl and a temperature comprised between 20°C and 65°C. In another particular embodiment, the very severe hybridization conditions comprise, for example, a concentration comprised between 0.02 M and 0.15 M of NaCl and a temperature comprised between 50°C and 70°C.

Sequence (b) of the polynucleotide of the invention corresponds to a sequence encoding a heterologous protein. In the context of the present invention, heterologous protein is understood as a protein which is not naturally encoded by the genome of a birnavirus. Heterologous protein sequences which can be incorporated in the polynucleotides of the present invention include sequences encoding heterologous proteins which are easily detectable, which allows using polynucleotides of the invention for studying infection processes by means of viewing cells expressing the detectable protein. Detectable heterologous polypeptides the encoding sequences of which can be incorporated in the present invention include but are not limited to luciferase, (green/red) fluorescent protein and variants thereof, such as EGFP (enhanced green fluorescent protein), RFP (red fluorescent protein, such as DsRed or DsRed2), CFP (cyan fluorescent protein), BFP (blue fluorescent protein), YFP (yellow fluorescent protein), β-galactosidase or chloramphenicol acetyltransferase, and the like.

Alternatively or additionally, the heterologous polypeptide can be a polypeptide of therapeutic interest such that the virus-like particles generated according to the methods of the present invention can be used for the *in vitro* expression of said polypeptide or for the treatment of diseases requiring the expression of said polypeptide. Examples of sequences of therapeutic interest which can be incorporated in the polynucleotides of the invention include but are not limited to genes or cDNAs encoding erythropoietin (EPO), leptins, corticotropin-releasing hormone (CRH), growth hormone-releasing hormone (GHRH), gonadotropin-releasing hormone (GnRH), thyrotropin-releasing hormone (TRH), prolactin-releasing hormone (PRH), melatonin-releasing hormone (MRH), prolactin-inhibiting hormone (PIH), somatostatin, adrenocorticotropic hormone (ACTH), somatotropin or growth hormone (GH), luteinizing hormone (LH), follicle-stimulating hormone (FSH), thyrotropin (TSH or thyroid-stimulating hormone), prolactin, oxytocin, antidiuretic hormone (ADH or vasopressin), melatonin, Müllerian inhibiting factor, calcitonin, parathyroid hormone, gastrin, cholecystokinin (CCK), secretin, insulin-like growth factor type I (IGF-I), insulin-like growth factor type II (IGF-II), atrial natriuretic peptide (ANP), human chorionic gonadotropin (HCG), insulin, glucagon, somatostatin, pancreatic polypeptide (PP), leptin, neuropeptide Y, renin, angiotensin I, angiotensin II, factor VIII, factor IX, tissue factor, factor VII, factor X, thrombin, factor V, factor XI, factor XIII, interleukin 1 (IL-1), interleukin 2 (IL-2), tumor necrosis factor alpha (TNF-α), interleukin 6 (IL-6), interleukin 8 (IL-8 and chemokines), interleukin 12 (IL-12), interleukin 16 (IL-16), interleukin 15 (IL-15), interleukin 24 (IL-24), interferons alpha, beta, gamma, CD3, ICAM-1, LFA-1, LFA-3, chemokines including RANTES 1α, MIP-1α, MIP-1β, nerve growth factor (NGF), platelet-derived growth factor (PDGF), transforming growth factor beta (TGF-beta), bone morphogenetic proteins (BMPs), fibroblast growth factors (FGF and KGF), epidermal growth factor (EGF and related factors), vascular endothelial growth factor (VEGF), granulocyte colony-stimulating factor (G-CSF), glial growth factor, keratinocyte growth factor, endothelial growth factor, alpha 1-antitrypsin, tumor necrosis factor, granulocyte-macrophage colony-stimulating factor (GM-CSF), cardiotrophin-1 (CT-1), oncostatin M (OSM), amphiregulin (AR), cyclosporine, fibrinogen, lactoferrin, tissue-type plasminogen activator (tPA), chymotrypsin, immunoglobins, hirudin, superoxide dismutase, imiglucerase, β-Glucocerebrosidase, alglucosidase-α, α-L-iduronidase, iduronate-2-sulfatase, galsulfase, human α-galactosidase A, α-1 proteinase inhibitor, lactase, pancreatic enzymes (lipase, amylase, protease), adenosine deaminase, immunoglobulins, albumin, Botulinum toxins type A and B, collagenase, human deoxyribonuclease I, hyaluronidase, papain, L-asparaginase, lepirudin, streptokinase, cell transforming factor beta (TNF-**β**) inhibitor peptides such as those described in WO0331155 WO20051924 and WO0393293 the content of which is incorporated in the present invention by reference, expression cassettes suitable for the transcription of interference RNA molecules (shRNA, siRNA, miRNA, RNA of modified U1 ribonucleoproteins).
Sequence (c) of the polynucleotide of the invention corresponds to the non-translated region of the genome of a birnavirus or to a functionally equivalent variant thereof. As in the case of sequence (a), sequence (c) can be single-stranded DNA, double-stranded DNA, single-stranded RNA and double-stranded RNA. In the event that the polynucleotide of the invention is single-stranded DNA, sequence (c) is the DNA sequence complementary to the sequence of the (-) strand present in the non-translated 3' region of a segment of the birnavirus genome. In the event that the polynucleotide of the invention is double-stranded DNA, sequence (c) corresponds to the non-translated 3' region of a segment of the birnavirus genome but wherein the uracil residues are thymidine residues. In the event that the polynucleotide of the invention is single-stranded RNA, sequence (c) comprises the sequence of the (+) strand corresponding to the non-translated 3' region of a segment of the birnavirus genome. In the event that the polynucleotide of the invention is double-stranded RNA, sequence (c) corresponds to the non-translated 3' region of a segment of the birnavirus double-stranded RNA genome.

As with the non-translated 5' region of sequence (i), sequence (iii) can be from segment A or from segment B of a birnavirus or can correspond to variants resulting from the insertion, elimination or substitution of one or more nucleotides, variants which hybridize under severe conditions and variants corresponding to homologs of other birnaviruses provided that they maintain substantially unaltered their capacity to promote the transcription mediated by viral RNA-polymerase or to promote the translation of the sequences located in the 3' position with respect to said region. In a preferred embodiment, sequence (iii) is from the sequence of the non-translated 3' region of the (+) strand of segment A of IBDV (SEQ ID NO:2).

### Gene constructs, vectors and host cells

As defined above, the polynucleotide of the invention can be a DNA molecule or an RNA molecule. Although RNAs can be introduced inside a cell both *in vitro* and *in vivo* using the suitable technology, the high sensitivity thereof to RNases makes it preferable for the polynucleotide to be administered in the form of DNA, either in the form of naked DNA or forming part of a viral particle. In this case, the DNA must be transcribed to give rise to a single-stranded RNA, for which said DNA molecule is typically under the operative control of a transcription regulatory sequence.

Thus, in another aspect, the invention relates to a polynucleotide according to the invention wherein said polynucleotide is DNA and sequence (a) is operatively coupled to a transcription regulatory region (hereinafter, expression cassette of the invention).

As used in the present invention, the term "operatively coupled" relates to an ordering of elements wherein each of said elements is arranged such that it performs its usual function. Thus, a gene is operatively coupled to a determined promoter when the promoter is capable of activating the transcription of said gene in the presence of the suitable enzymes. The promoter does not have to be contiguous to the sequence of the gene provided that it maintains the transcription-activating function. Thus, even when non-translated transduction regulatory sequences appear between the promoter and the end of the encoding sequence of the gene, the gene will be considered to be under operative control if its transcription is activated by said promoter.

The promoters which can be used to regulate the transcription of the polynucleotide of the invention can be constitutive promoters, i.e., promoters which direct the transcription in a basal manner or inducible promoters in which the transcriptional activity requires an external signal. Suitable constitutive promoters for regulating the transcription are, among others, the CMV promoter, the SV40 promoter, the DHFR promoter, the mouse mammary tumor virus (MMTV) promoter, the elongation factor 1a (EFla) promoter, the albumin promoter, the ApoA1 promoter, the keratin promoter, the CD3 promoter, the immunoglobulin heavy or light chain promoter, the neurofilament promoter, the neuron-specific enolase promoter, the L7 promoter, the CD2 promoter, the myosin light chain kinase promoter, the HOX gene promoter, the thymidine kinase promoter, the RNA Polymerase II promoter, the MyoD gene promoter, the phosphoglycerokinase (PGK) gene promoter, the low-density lipoprotein (LDL) promoter, the actin gene promoter. In a preferred embodiment, the promoter regulating the expression of the transactivator is the PGK gene promoter. In a preferred embodiment, the promoter regulating the transcription of the polynucleotide of the invention is the T7 phage RNA polymerase promoter (SEQ ID NO:3) .

The inducible promoters which can be used in the context of the present invention are preferably those which respond to an inducing agent, which show nil or negligible basal expression in the absence of inducing agent and which are capable of promoting the activation of the gene located in the 3' position. Depending on the type of inducing agent, the inducible promoters are classified as Tet on/off promoters (Gossen, M. and H. Bujard (1992) Proc.Natl.Acad.Sci.USA, 89:5547-5551; Gossen, M. et al., 1995, Science 268:1766-1769; Rossi, F.M.V. and H.M. Blau, 1998, Curr. Opin. Biotechnol. 9:451-456); Pip on/off promoters (US6287813); antiprogestin-dependent promoters (US2004132086), ecdysone-dependent promoters (Christopherson et al., 1992, Proc.Natl.Acad.Sci.USA, 89:6314-6318; No et al., 1996, Proc.Natl.Acad.Sci.USA, 93:3346-3351, Suhr et al., 1998, Proc.Natl.Acad.Sci.USA, 95:7999-8004 and WO9738117) a metallothionein-dependent promoter (WO8604920 and rapamycin-dependent promoters (Rivera et al., 1996, Nat.Med. 2:1028-32).

Additionally, the expression cassette of the invention can contain additional expression-regulating elements such as start and stop signals, cleavage sites, polyadenylation signal, origin of replication, transcriptional activators (enhancers), transcriptional silencers (silencers), etc. Generally, said elements, as well as the vectors used to construct the expression cassettes and the recombinant vectors according to the invention are chosen depending on the host cells which are to be used.

The polynucleotide of the invention can additionally contain the transcription terminator sequence, such as the terminator of the bovine or human growth hormone gene, the terminator of the SV40 early genes, the terminators of the beta globin genes. In a preferred embodiment, the terminator corresponds to the terminator of the T7 phage RNA polymerase (SEQ ID NO:4).

In the event that the polynucleotide of the invention is a DNA molecule and comprises additional regions in the 3' position (for example, polyadenylation sequences or transcriptional terminators), the polynucleotide of the invention can additionally comprise a cleavage sequence in the 3' position with respect to the non-translated sequence (c) the function of which is to be eliminated after the generation of RNA from the DNA polynucleotide. In a preferred embodiment, the cleavage sequence corresponds to the DNA encoding the hepatitis δ virus ribozyme (SEQ ID NO:5). The hepatitis δ virus ribozyme is capable of being self-processed such that, once the polynucleotide of the invention has been transcribed, the hepatitis δ virus ribozyme acts on the RNA strand, causing its elimination from the RNA strand. It is thus possible to exactly control the sequence of the terminal 3' region, length of the RNA molecule, since it is enough to include the sequence encoding the hepatitis δ virus ribozyme immediately in the 3' position with respect to the nucleotide which is to form the terminal nucleotide of the RNA molecule. In a preferred embodiment, the sequence encoding the self-processable sequence is in the 3' position with respect to sequence (c) comprising a non-translated region of a segment of the genomic RNA of said birnavirus.

In another aspect, the invention relates to vectors comprising the polynucleotide of the invention. Vectors suitable for the insertion of said polynucleotide are vectors derived from expression vectors in prokaryotes such as pUC18, pUC19, Bluescript and derivatives thereof, mp18, mp19, pBR322, pMB9, ColEl, pCR1, RP4, phages and shuttle vectors such as pSA3 and pAT28, expression vectors in yeasts such as vectors of the type of 2-micron plasmids, integration plasmids, YEP vectors, centromeric plasmids and the like, expression vectors in insect cells such as vectors of the pAC series and of the pVL series, expression vectors in plants such as vectors of the pIBI, pEarleyGate, pAVA, pCAMBIA, pGSA, pGWB, pMDC, pMY, pORE series and the like and expression vectors in higher eukaryotic cells based on viral vectors (adenoviruses, adeno-associated viruses as well as retroviruses and, particularly, lentiviruses) as well as non-viral vectors such as pSilencer 4.1-CMV (Ambion), pcDNA3, pcDNA3.1/hyg pHCMV/Zeo, pCR3.1, pEFl/His, pIND/GS, pRc/HCMV2, pSV40/Zeo2, pTRACER-HCMV, pUB6/V5-His, pVAX1, pZeoSV2, pCI, pSVL and pKSV-10, pBPV-1, pML2d and pTDT1.

In another aspect, the invention relates to a host cell comprising a polynucleotide of the invention or a vector of the invention.

### Method for obtaining transcriptionally active virus-like particles

The polynucleotides of the invention can be used to generate transcriptionally active virus-like particles by means of introducing said polynucleotides in a cell together with (i) one or several polynucleotides encoding the structural proteins necessary for the encapsidation of an RNA virus requiring an RNA-dependent RNA polymerase for its replication, wherein said RNA virus is of the same species as the RNA virus from which the non-translated sequences forming part of the polynucleotide of the invention are derived and (ii) a polynucleotide comprising the sequence encoding the RNA polymerase of the RNA virus from which the non-translated sequence forming part of the first polynucleotide is derived.

Without wishing to be bound by any theory, it is believed that the presence of the three polynucleotides in a cell results in (i) the expression of the different structural proteins of the RNA virus, (ii) the expression of the RNA polymerase of said RNA virus from the polynucleotide encoding it and (iii) the transcription of the polynucleotide of the invention to give rise to a single-stranded RNA which is subsequently used as a template by the RNA-dependent RNA polymerase of the RNA virus, thus giving rise to a double-stranded RNA. The structural proteins of the virus form virus-like particles which include the RNA polymerase (by means of the interaction of said RNA polymerase with the RNA virus VP3 protein) and the double-stranded RNA.

Thus, Example 2 of the present invention shows how, in the particular case of a birnavirus (IBDV), the introduction in a cell of the polynucleotide encoding the polyprotein of the birnavirus gives rise to the expression of the different structural proteins of the IBDV virus (VP2, VP3 and VP4), the introduction of a second polynucleotide encoding the RNA polymerase gives rise to the expression of said RNA polymerase (VP1). The presence in said cell of a third polynucleotide comprising the sequence of a gene of interest flanked by the non-translated 5' and 3' regions derived from segment A of the IBDV genome results in the production of transcriptionally active virus-like particles, i.e., comprising RNA polymerase and the modified birnavirus genome. When these particles are contacted with target cells, they release said recombinant genomic segment which in turn results in the expression of said gene of interest mediated by the RNA polymerase forming part of said particles.

Therefore, in another aspect, the invention relates to a method (hereinafter, first method of the invention) for obtaining transcriptionally active virus-like particles which comprises the steps of:
(i) contacting a cell with a first polynucleotide under conditions suitable for the entry of said polynucleotide in the cell, wherein said polynucleotide comprises in the 5' to 3' direction
   (a) a first sequence comprising a non-translated region of a segment of the genomic RNA of an RNA virus requiring an RNA polymerase for its replication or a functionally equivalent variant thereof,
   (b) a second sequence encoding a heterologous product of therapeutic interest and
   (c) a third sequence comprising a non-translated region of a segment of the genomic RNA of said RNA virus or a functionally equivalent variant thereof
(ii) contacting said cell with one or several additional polynucleotides under conditions suitable for the entry of said polynucleotide or said polynucleotides in the cell and wherein said polynucleotide or said polynucleotides comprise sequences encoding the RNA polymerase of said RNA virus from which the non-translated sequence forming part of the first polynucleotide is derived sequences encoding all the proteins necessary for the encapsidation of said RNA virus,
(iii) maintaining the cells under conditions suitable for the expression of the polynucleotides introduced in the cell in steps (i) and (ii) and
(iv) recovering the virus-like particles from the culture obtained in (iii)
wherein steps (i) and (ii) are carried out in any order or simultaneously.

Step (i) of the first method of the invention comprises contacting a cell with a polynucleotide under conditions suitable for the entry of said polynucleotide in the cell, wherein said polynucleotide comprises
(a) a first sequence comprising a non-translated region of a segment of the genomic RNA of an RNA virus requiring an RNA polymerase for its replication or a functionally equivalent variant thereof,
(b) a second sequence encoding a heterologous product of therapeutic interest and
(c) a third sequence comprising a non-translated region of a segment of the genomic RNA of said RNA virus or a functionally equivalent variant thereof

An RNA virus requiring an RNA polymerase for its replication is understood as a virus the genome of which is formed by an RNA molecule and which, unlike other RNA viruses the genome of which first undergoes a reverse transcription to give rise to a DNA which is used as a template for the replication, is replicated as a result of an RNA-dependent RNA polymerase which is encoded by the genome of the virus itself. RNA viruses the replication of which requires an RNA-dependent RNA polymerase include but are not limited to the viruses of the Birnaviridae family, the viruses of the Flaviviridae family, the viruses of the Cystoviridae family, the viruses of the Reoviridae family, the viruses of the Coronaviridae family, the viruses of the Togaviridae family, arteriviruses, the viruses of the Astroviridae family, the viruses of the Caliciviridae family, the viruses of the Picornaviridae family, the viruses of the Potyviridae family, the viruses of the Filoviridae family, the viruses of the Paramyxoviridae family, the viruses of the Rhabdoviridae family, the viruses of the Arenaviridae family and the viruses of the Bunyaviridae family. The person skilled in the art will understand that the non-translated 5' and 3' sequences of the different RNA viruses can be obtained from the sequences of said viruses described in public access databases. In a preferred embodiment, the polynucleotide used in step (i) includes the non-translated 5' and 3' sequences of a virus of the birnaviridae family. Even more preferably, the birnavirus used is IBDV, in which case the non-translated 5' and 3' sequences can be from segment A or from segment B of the genome of said virus.

The conditions suitable for the entry of the polynucleotide in the cell are well known for the person skilled in the art and include the microinjection of a composition comprising the polynucleotide or, alternatively, contacting the cell with a coprecipitate of the polynucleotide and calcium phosphate or calcium chloride or, alternatively, using transfection techniques such as DEAE-dextran, lipofection, electroporation as well as an entire series of transfection kits based on the previous techniques and which are commercially available. Alternatively, the polynucleotide can form part of a viral vector, in which case the conditions suitable for the entry of the polynucleotide in the cell include contacting the cell with said viral vector. Viral vectors suitable for the introduction of the polynucleotide in the cell include but are not limited to adenoviral vectors, adeno-associated vectors, retroviral vectors, lentiviral vectors, vectors based on herpes simplex or vectors based on alphaviruses. In a preferred embodiment, the polynucleotide is introduced in the cell using a vaccinia vector.

The efficiency of the transfection will depend on a series of factors including the type of cell, the number of passages, the state of confluence as well as the transfection conditions (time, form of preparation of the liposomes or of the precipitates, etc.). All these parameters can be determined and adjusted by means of routine experimentation.

Step (ii) comprises contacting the cell used in step (i) with one or several polynucleotides comprising sequences encoding both the RNA polymerase of the RNA virus and all the structural and non-structural proteins of said virus which are necessary for the formation of virus-like particles under conditions suitable for the entry of said polynucleotide or polynucleotides in the cell wherein said sequences are from an RNA virus of the same species as the virus which the non-translated sequences used in the polynucleotide used in step (i) are from.

In the particular case in which IBDV is used, the sequences necessary for the generation of virus-like particles include the sequences encoding the structural capsid proteins (VP2, VP3 and VP4). Therefore, it is possible to contact the cell used in step (i) with polynucleotides encoding each of said structural proteins. However, the person skilled in the art will understand that use can be made of the genomic organization itself of the birnaviruses wherein all the proteins necessary for the formation of the capsids are synthesized in the form of a polyprotein comprising both the aforementioned structural capsid proteins and the non-structural capsid proteins (VP5 and the non-structural NS protease) but which are necessary for processing the polyprotein. In that case, step (ii) would comprise contacting the cell with a polynucleotide comprising the sequence encoding said polyprotein. This sequence appears in segment A of the birnavirus genome, therefore, in a preferred embodiment, step (ii) comprises contacting the cell with a polynucleotide comprising the sequence of which corresponds to the sequence of segment A of the genome of a birnavirus with the exception of the non-translated regions in the 5' and 3' position. In an even more preferred embodiment, the polynucleotide encoding the polyprotein is from segment A of the IBDV genome. In an even more preferred embodiment, the polynucleotide comprises the sequence defined in SEQ ID NO:6.

In a preferred embodiment, the polynucleotide encoding the capsid protein of the birnavirus can be modified by means of the presence of an encoding sequence for a heterologous amino acid sequence with the capacity to bind to a ligand. Virus-like particles will thus be obtained in which the capsid will be modified by the presence of multiple copies of said ligand, which will allow the formation of conjugates between the virus-like particles and a compound of interest which is capable of binding specifically to said encoding sequence. This modification of the virus-like particles will allow addressing the particles to a target organ or cell of interest having on its surface molecules with the capacity to bind specifically to said heterologous sequence.

The modification of the polynucleotide encoding the structural protein or proteins can be carried out such that the heterologous insert appears in a single viral protein or in several viral proteins. In a preferred embodiment, the sequence which is modified is the sequence encoding the VP2 structural protein such that a fusion protein formed by the VP2 birnaviral protein and the heterologous peptide is obtained.

As used herein, the term "IBDV VP2 (or pVP2) protein" generally relates to a protein the amino acid sequence of which comprises, or is formed by, the amino acid sequence of the IBDV pVP2 of any IBDV strain belonging to any of the known serotypes (1 or 2) [by way of illustration see the review by van den Berg TP et al. (2000), Rev Sci Tech. 19:509-43] and includes any of the different forms of the VP2 proteins representative of any IBDV strain, according to the definition provided by Sánchez and Rodriguez (1999) as well as to proteins substantially homologous to said IBDV pVP2 proteins, i.e., proteins having a good alignment with the sequence of a determined IBDV pVP2, for example, proteins the amino acid sequences of which have a degree of identity, with respect to said IBDV pVP2 proteins, of at least 60%, preferably of at least 80%, more preferably of at least 90% and even more preferably of at least 95%. Sequences homologous to a sequence of the IBDV pVP2 protein can be easily identified by a person skilled in the art with the aid of a computer program suitable for comparing sequences, for example, the BLAST program (Altschul *et al.* (1997)). In a particular embodiment, the IBDV pVP2 protein is the Soroa strain IBDV pVP2 protein the full-length amino acid sequence of which is deposited in the NCBI with accession number AAD30136.

As used herein, the term "peptide insert" [i.e., heterologous peptide comprising an amino acid sequence with the capacity to bind to a ligand] does not involve any particular size or any other structural technical feature different from that of said peptide insert (which comprises a specific binding domain) being capable of binding to a ligand (which comprises the region of interaction corresponding to said specific binding domain present in the peptide insert); nevertheless, in a particular embodiment, for its use in the present invention, said peptide insert must not adversely affect the assembling capacity of the structural protein with which it is fused. The size of the peptide insert can vary within a wide range, for example, between 1 and 50 amino acid residues; advantageously, said peptide insert has less than 40 amino acid residues, preferably less than 30 amino acid residues, more preferably less than 20 amino acid residues, for example, between 1 and 15 amino acids.

Therefore, virtually any heterologous peptide with the capacity to bind to a ligand can be used in the present invention. By way of non-limiting illustration, said peptide can be a member of a specific binding pair, for example, an amino acid tag (e.g., a histidine tag (his-tag), an arginine tag (arg-tag), etc.), a peptide epitope which can be recognized by an antibody (e.g., c-myc-tag, etc.), a biotin acceptor peptide (BAP), a linear domain of interaction with another protein or proteins (e.g. SH3 proteins, signal transduction proteins, etc.), a post-translational modification sequence (e.g. myristoylation, methylation, phosphorylation, etc.), a sequence of transport to a cell compartment, etc.

In a particular embodiment, said peptide with the capacity to bind to a ligand is a BAP; as used herein, the term "BAP" relates to a peptide with the capacity to bind to biotin or to an analog thereof (e.g., 2-iminobiotin, etc.); illustrative examples of BAP are mentioned in EP 711303 (entirely incorporated in this description by reference) and include peptides comprising the following amino acid sequence (SEQ ID NO:7):
Leu Xaa1 Xaa2 Ile Xaa3 Xaa4 Xaa5 Xaa6 Lys Xaa7 Xaa8 Xaa9 Xaa10 wherein Xaa1 is any one amino acid; Xaa2 is an amino acid different from Leu, Val, Ile, Trp, Phe, or Tyr; Xaa3 is Phe or Leu; Xaa4 is Glu or Asp; Xaa5 is Ala, Gly, Ser, or Thr; Xaa6 is GIn or Met; Xaa7 is Ile, Met, or Val; Xaa8 is Glu, Leu, Val, Tyr, or Ile; Xaa9 is Trp, Tyr, Val, Phe, Leu, or Ile; and Xaa10 is any amino acid different from Asp or Glu.

In a specific embodiment, said peptide with the capacity to bind to a ligand is a BAP with 15 amino acids having the amino acid sequence:

### GLNDIFEAQKIEWHE (SEQ ID NO:8)

The peptide insert can be found in any position with respect to the capsid protein to which it is fused. Thus, the invention contemplates the use of polynucleotides encoding a fusion protein comprising the structural protein the C-terminus of which is fused to the N-terminus of the peptide insert and polynucleotides encoding a fusion protein comprising the structural protein the N-terminus of which is fused to the C-terminus of the peptide insert.

In a preferred embodiment, the heterologous insert is inserted inside the amino acid sequence of the capsid protein. The introduction of said peptide insert in said capsid protein must be performed such that it does not substantially alter the self-assembly capacity of said capsid protein; and, furthermore, said peptide insert must be located in a region of said capsid protein accessible to the solvent in the macromolecular structures formed after the self-assembly of the capsid protein.

In this particular embodiment, given that the peptide insert is inserted inside the amino acid sequence of the capsid protein, the fusion protein maintains the amino and carboxyl termini of said capsid protein and the peptide insert is included inside the amino acid sequence of said capsid protein, for example, between two contiguous amino acids of said capsid protein or, alternatively, replacing one or more amino acids of said capsid protein. By way of illustration, in a particular embodiment, said peptide insert is located between 2 contiguous amino acids of the capsid protein, i.e., between the "x" residue and the "x+1" residue of the amino acid sequence of said capsid protein; whereas, in another particular embodiment, due to the strategy followed for the introduction of the peptide insert one or more residues located in the regions adjacent to the point of insertion can be lost. Furthermore, given that the peptide insert must be located in a region of said capsid protein accessible to the solvent in the virus-like particles formed according to the method of the invention, the peptide insert must be inserted in a suitable region. Particularly suitable regions of the structural protein for introducing the peptide insert are the loops present in said capsid proteins, particularly those loops which, in the macromolecular structures that they form, are exposed to the solvent. Illustrative examples of loops present in capsid proteins which can be used while putting the present invention into practice include the P_{HI} loop of the IBDV pVP2.

To choose the suitable site of insertion of the peptide insert in the birnaviral capsid protein it is convenient to know the atomic structure of the capsid protein to be genetically modified. The atomic structure of a structural protein can be determined by means of using X-ray diffraction. The use of suitable computer programs, such as the UCSF Chimera program (http://www.cgl.ucsf.edu/chimera/) for example, allows locating the regions of the protein under study which can potentially be genetically modified. These merely theoretical predictions initially serve to: i) discard regions that are not exposed to the solvent; and ii) identify regions the modification of which could cause a serious alteration in the secondary structure of the protein. Once the atomic structure of the capsid protein is known, a strategy can be designed to incorporate the peptide insert in the suitable site, for example, in a loop of said capsid protein which is exposed to the solvent when said structural protein is assembled with the rest of the structural proteins of the birnavirus and forms the macromolecular structure.

In a particular embodiment, said structural protein is IBDV pVP2, or a functional variant thereof, such as the protein identified as IBDV pVP2_452 (SEQ ID NO:9). In this case, for the purpose of designing the strategy for introducing the peptide insert in the suitable region, an in-depth study of the atomic structure of said IBDV pVP2 452, which has already been described (Garriga et *al.,* 2006) (Protein Data Bank accession no. 2GSY) was conducted by means of using the UCSF Chimera computer program (http://www.cgl.ucsf.edu/chimera/). After this analysis, a strategy was designed for incorporation, in the region corresponding to the P_{HI} loop, located in the region exposed to the solvent of said IBDV pVP2_452 in the macromolecular structures formed after the assembly thereof (Figure 2). Therefore, in a specific embodiment, the SAP is IBDV pVP2, or a functional variant thereof, such as the protein identified as IBDV pVP2_452 (SEQ ID NO:9), and said peptide insert is located inside the P_{HI} loop of said IBDV pVP2 or functional variant thereof (IBDV pVP2_452) either between 2 contiguous amino acids or replacing one or more of the amino acids of said P_{HI} loop.

In another specific embodiment, the capsid protein is IBDV pVP2, or a functional variant thereof, such as the protein identified as IBDV pVP2_452 (SEQ ID NO:9), and said peptide insert comprises the amino acid sequence of a BAP. In an even more specific embodiment, said BAP comprises the amino acid sequence shown in SEQ ID NO:8.

In another specific embodiment, the capsid protein is IBDV pVP2, or a functional variant thereof, such as the protein identified as IBDV pVP2_452 (SEQ ID NO:9) , said peptide insert comprises the amino acid sequence of a BAP and said peptide insert is located inside the P_{HI} loop of said IBDV pVP2 or functional variant thereof (IBDV pVP2_452) either between 2 contiguous amino acids or replacing one or more amino acids of said P_{HI} loop. In an even more specific embodiment, said BAP comprises the amino acid sequence shown in SEQ ID NO:8 and the insertion of said peptide insert in said capsid protein (IBDV pVP2, or a functional variant thereof, such as the protein identified as IBDV pVP2_452) is performed between residues 319 and 321 of the amino acid sequence of said capsid protein, which entails the elimination of residue 320 (Gln).

In another particular embodiment, said peptide insert is bound to the amino or carboxyl terminus of said structural protein. Therefore, in a particular embodiment, said peptide insert is bound to the amino terminus of said capsid protein, whereas, in another particular embodiment, said peptide insert is bound to the carboxyl terminus of said capsid protein. In another particular embodiment, the possibility is contemplated that the capsid protein comprises two peptide inserts bound to said protein, wherein each of said peptide inserts is bound to each of the amino and carboxyl termini of said capsid protein.

In a particular embodiment, the heterologous peptide is bound to the amino or carboxyl terminus of said capsid protein. In a specific embodiment, said BAP comprises the amino acid sequence shown in SEQ ID NO:8. In another more particular embodiment, said capsid protein is IBDV pVP2, or a functional variant thereof, such as IBDV pVP2_452.

Likewise, step (ii) comprises contacting the cell with a polynucleotide encoding the RNA-dependent RNA polymerase of an RNA virus. In the particular case of using IBDV, the RNA polymerase is encoded by segment B of the birnavirus genome. Therefore, it is possible to use in step (ii) a polynucleotide the sequence of which is complementary to one of the strands of the double-stranded RNA forming segment B of the birnavirus genome with the exception of the non-encoding regions in the 5' position and in the 3' position with respect to the sequence encoding the RNA polymerase. The sequence encoding the RNA polymerase can form part of the same polynucleotide which encodes the structural proteins or the polyprotein of the birnavirus, in which case the sequence encoding the RNA polymerase can be preceded by an internal ribosome entry sequence or IRES, such that from a polycistronic RNA, it is possible to obtain a translation product corresponding to the RNA polymerase. IRESs suitable for their use in the present invention include but are not limited to eukaryotic IRESs such as those appearing in the RNAs encoding proteins such as FGF, connexins, the p27, Bcl-2, HSP 101, HSP 70 protein, the c-myc, L-myc, n-myc proto-oncogenes or in the Mnt transcriptional repressor, as well as viral IRESs such as those present in the poliomyelitis virus, in the encephalomyocarditis virus, the GBV-A, GBV-B, GBV-C viruses, hepatitis C virus, bovine viral diarrhea virus, equine rhinitis A and B viruses (ERAV and ERBV), the retroelements ZAM, Idefix and gypsy or of HIV.

Alternatively, it is possible for the polynucleotide encoding the structural polyproteins and the polynucleotide encoding the RNA polymerase to be a single monocistronic polynucleotide encoding a polyprotein comprising both the structural proteins of the birnavirus and the RNA polymerase. In this case, it is possible to include a sequence encoding an autoprotease between the encoding sequences for each of the proteins or at least between the sequence encoding the capsid proteins and the region encoding the RNA polymerase. Autoproteases act in *cis* on the fusion protein eliminating the sequences from the protease and from the translation-enhancing sequence, giving rise to a protein the N-terminus of which only has an additional proline. By way of non-limiting illustration, said nucleotide sequence encoding an (auto)protease acting in cis between the sequence of said autoprotease and the sequence encoded by the polynucleotides forming the vector of the invention is from a virus, for example, a picornavirus, an alphavirus, etc. In a particular embodiment, said sequence comprises the nucleotide sequence encoding the 2A region of the polyprotein of the foot-and-moth disease virus (FMDV) or FMDV autoprotease 2A as has been described, among others, in EP736099, the entire content of which is included by reference.

The introduction in the cells of the polynucleotides encoding the structural proteins and the RNA polymerase can be carried out transiently or stably by means of using vectors comprising said polynucleotides and a selection marker such that, by means of selecting the cells in the presence of an agent which is toxic for those cells which do not express the selection marker, only those cells which have incorporated the vector in their genome will be capable of surviving. Selection markers suitable for their use in the present invention include but are not limited to the neomycin resistance gene, which confers resistance to the G418 aminoglycoside, the hygromycin phosphotransferase gene, which confers resistance to hygromycin, the ODC gene, which confers resistance to the ornithine decarboxylase inhibitor 2-(difluoromethyl)-DL-ornithine (DFMO), the dihydrofolate reductase gene, which confers resistance to methotrexate, the puromycin-N-acetyl transferase gene, which confers resistance to puromycin, the ble gene, which confers resistance to zeocin, the adenosine deaminase gene, which confers resistance to 9-beta-D-xylofuranosyl adenine, the cytosine deaminase gene, which allows the cells to grow in the presence of N-(phosphonacetyl)-L-aspartate, thymidine kinase, which allows the cells to grow in the presence of aminopterin, the xanthine-guanine phosphoribosyltransferase gene, which allows the cells to grow in the presence of xanthine and the absence of guanine, the E.coli trpB gene, which allows the cells to grow in the presence of indole instead of tryptophan, the *E.coli* hisD gene, which allows the cells to use histidinol instead of histidine. The selection gene is incorporated in a plasmid which can additionally include a promoter suitable for the expression of said gene in eukaryotic cells (for example, the CMV or SV40 promoters), an optimized translation initiation site (for example, a site which follows so-called Kozak's rules or an IRES), a polyadenylation site such as, for example, the polyadenylation site of SV40 or of phosphoglycerate kinase, introns such as, for example, the intron of the beta-globulin gene.

Evidently, if the expression of the structural proteins or of the RNA polymerase of the birnavirus results in a cytopathic effect, the transient transfection of the cells is preferable or, if stable transfection is used, it is preferable for the expression of said proteins to be under the control of an inducible promoter such that the expression of the birnaviral proteins occurs only after the addition to the cells of an agent capable of promoting the activation of said promoter. Inducible promoters suitable for regulating the expression of birnaviral proteins have been described previously.

In a preferred embodiment, the RNA polymerase is provided to the cell forming part of a polynucleotide different from the one encoding the structural proteins and, preferably, the sequence encoding the RNA polymerase is from IBDV. In an even more preferred embodiment, the polynucleotide encoding the RNA polymerase comprises the sequence defined in SEQ ID NO:10.

In the same way as step (i), the introduction of the polynucleotides encoding the RNA polymerase and the structural proteins of the birnavirus is carried out using standard transfection techniques (if the polynucleotide is free or forms part of a vector) or infection techniques (if the polynucleotide has been incorporated in a viral vector).

Step (iii) comprises maintaining the cells under conditions suitable for the expression of the polynucleotides introduced in steps (i) and (ii). In the event that the polynucleotide introduced in step (i) is DNA, the transcription thereof is necessary to generate single-stranded RNA which is subsequently converted into double-stranded RNA by the mediation of the RNA polymerase introduced in step (ii). The transcription of the polynucleotide introduced in step (i) requires the presence of a transcription promoter region in the 5' position with respect to the first non-translated region wherein said promoter region must be suitable for the expression of sequences located in the 3' position in the cells used for the production of the virus-like particles. Promoters suitable for the expression of the polynucleotide of the invention have been described previously. In a preferred embodiment, the promoter is the T7 phage RNA polymerase promoter, in which case the cell must be modified such that it expresses said RNA polymerase. In a preferred embodiment, the polynucleotide introduced in step (i) forms part of a recombinant vaccinia virus comprising the sequence encoding the T7 phage RNA polymerase as well as the sequence of the polynucleotide of the invention obtained using the methodology described by Kriajevska et al. (J.Gene.Virol., 1993, 74:47-53). In this way, the T7 phage RNA polymerase which recognizes the promoter region located in the 5' region with respect to the polynucleotide of the invention is synthesized in the cell from a single vector, thus generating an RNA molecule comprising the non-translated 5' and 3' regions of a birnavirus flanking a region encoding a protein of therapeutic interest.

In the same way, the expression of the polynucleotides introduced in step (ii) requires transcription mediated by cellular RNA polymerase or, alternatively, by a viral RNA polymerase introduced in the cell simultaneously with the polynucleotides of the invention. In a preferred embodiment, the sequence encoding the polyprotein of the birnavirus and the sequence encoding the RNA polymerase of the birnavirus is under the control of a T7 promoter forming part of respective recombinant vaccinia vectors additionally comprising the sequence encoding the T7 phage RNA polymerase.

The person skilled in the art will understand that the objective of steps (i) and (ii) of the first method of the invention is to provide the cell with all the components necessary for the formation of the virus-like particles, therefore they can be carried out in any order or simultaneously. In a preferred embodiment, steps (i) and (ii) are carried out simultaneously by means of contacting the cells with recombinant vaccinia vectors comprising the polynucleotides of the invention, the polynucleotide encoding the polyprotein of the birnavirus and the polynucleotide encoding the RNA polymerase under the control of the T7 phage promoter and additionally comprising the sequence encoding the T7 phage RNA polymerase. Alternatively, it is also possible to simultaneously or sequentially introduce plasmids in the cells comprising the necessary polynucleotides under the control of the T7 phage RNA polymerase promoter and subsequently infect the cells with a recombinant vaccinia virus comprising the sequence encoding the T7 phage RNA polymerase.

Finally, the first method of the invention includes a step (iv) which comprises recovering the virus-like particles from the cell culture used in the previous steps. The particles can be recovered from the cells or from the supernatant of the culture medium. In the first case, the step of recovering requires the separation of the cells from the culture medium by any method known by the persons skilled in the art (trypsinization and centrifugation or filtration) and the rupture of said cells in an inert solution (freezing/thawing cycles, homogenization, sonication, cavitation, use of detergents and the like). Subsequently, it is possible to recover the particles from the cell homogenate or from the supernatant of the culture medium using well known methods such as density gradient ultracentrifugation (for example, using CsCl or sucrose) as has been described by Lombardo et al. (J. Virol., 1999, 73:6973-6983) and by Nick, H., et al. (J. Virol., 1976, 18:227-234), by means of affinity chromatography using anti-VP2 antibodies, by means of filtration using a series of fiberglass filters with a final pore size of 0.2 µm, as has been described in US2005214316.

### Virus-like particles of the invention

The first method of the invention allows obtaining transcriptionally active virus-like particles comprising an RNA polymerase of an RNA virus and a double-stranded recombinant RNA genome formed by the non-encoding regions of the genome of said RNA virus flanking a sequence encoding a gene of interest, wherein said recombinant genome and RNA polymerase are included in a viral capsid. Therefore, in another aspect, the invention relates to virus-like particles obtained by means of the first method of the invention. The nature of the particles will depend on the nature of the polynucleotides encoding the RNA polymerase and the structural proteins. In a preferred embodiment, said virus-like particles comprise the capsid of a birnavirus including:
(i) a variable number of copies of the RNA polymerase of said birnavirus and
(ii) a variable number of copies of a single-stranded RNA molecule comprising, in the 5' to 3' direction:
   (a) a first sequence comprising the non-translated region of a segment of the genomic RNA of the birnavirus or a functionally equivalent variant thereof,
   (b) a second sequence encoding a heterologous protein and
   (c) a third sequence comprising a non-translated region of a segment of the genomic RNA of said birnavirus or a functionally equivalent variant thereof

In a preferred embodiment, said polynucleotides are from a birnavirus and, more particularly, from IBDV, therefore the virus-like particles of the invention will be formed by an IBDV capsid and would include the RNA polymerase of IBDV and a variable number of copies of polynucleotide of the invention wherein the non-translated 5' and 3' regions are from genomic segment A of IBDV or from genomic segment B of IBDV.

As can be seen, the virus-like particle of the invention is a macromolecular structure resulting from the assembly of a determined number of structural proteins. The number of units of said structural proteins which must be assembled to form the virus-like particle of the invention depends on the specific virus. In the particular case of IBDV, the virus-like particles have a structure with icosahedral symmetry (with a triangulation number T=1), formed by 60 monomers of said protein organized into 20 identical trimers, with a diameter of 22 nm and a free internal volume of approximately 500 Å³, Each of the trimers is formed by the protein identified as IBDV pVP2_452 (SEQ ID NO:9) and is stabilized by the presence of a calcium atom which interacts with 6 Asp residues from the 3 molecules which form it. The structure of said virus-like particle is stabilized by means of a wide range of non-covalent interactions established between adjacent trimers.

In the event that the virus-like particles have been obtained using a variant of the capsid protein modified by means of the fusion of said capsid protein to a heterologous insert, these virus-like particles will have in the capsid as many copies of said insert as monomers of the structural protein which are forming the capsid. This insert will allow the formation of complexes between the virus-like particle and a product of interest. This product of interest can interact directly with the peptide insert or can be modified by means of the presence of a ligand showing affinity for the peptide insert such that the product of interest interacts with the peptide insert through said ligand.

As used herein, the term "product of interest" includes any molecule with biological activity in its broadest sense, i.e., any substance which, when administered to a subject, interacts with its receptor in the site of action and exerts a determined effect. The product of interest can be of a peptide (protein) nature or a non-peptide nature, for example, a protein, an antibody or a functional fragment thereof, a hormone, an enzyme, a lipid, a sugar, a non-peptide drug, a nucleic acid, etc.

Illustrative non-limiting examples of products of interest include any type of bioactive molecules such as synthetic molecules (e.g., acetylsalicylic acid, paracetamol, tranexamic acid, metoprolol, etc.), natural products (e.g., taxol, taxane derivatives, paclitaxel, epibatidine, atropine, etc.), oligonucleotides (e.g., augmerosen), aptamers (e.g., pegaptanib), proteins (e.g., interleukins, interferons, erythropoietins, coagulation factors, insulins, etc.), antibodies (e.g., erbitux, rituxan, herceptin, avastin, remicade, humira, xolair, etc.), peptides (e.g., calcitonins, vasopressin analogs (e.g., arginine vasopressin, desmopressin acetate, etc.), insulin polypeptides (e.g., humulin, insulin aspart, etc.), growth hormones (e.g., somatotropin, sermorelin, recombinant growth factor, etc.), gonadotropin polypeptides (e.g., urofollitropin, recombinant follicle-stimulating hormone, etc.), gonadotropins (e.g., chorionic gonadotropins), thyrotropic hormone analogs, somatostatin analogs (e.g., octreotide, lanreotide acetate, etc.), parathyroid hormone polypeptides (e.g., teriparatide acetate, recombinant parathyroid hormone (1-84), etc.), alpha-atrial natriuretic peptide, brain natriuretic peptide (e.g., nesiritide, etc.), thyroid hormone (e.g., thyrotropin, etc.), luteinizing hormone polypeptides (e.g., lutropin alfa, etc.), glucagon-like peptide-1 (GLP1) analogs (e.g., exenatide, etc.), corticotropin polypeptides (e.g., corticotropin, etc.), corticotropin release factor, gastrin analogs, peptides for radiation treatment (e.g., lanreotide, etc.), peptide vaccines (e.g., malaria, cancer, HIV, HVB, multiple sclerosis, etc.), hormones, enzymes, lipids, sugars, nucleic acids (DNA, RNA, PNA, etc.), e.g., oligonucleotides, polynucleotides, genes, interfering RNA, etc.

In a particular embodiment, said product of interest is of a peptide nature; in this case, its size can vary within a wide range, from a few amino acids to hundreds of amino acids. Said peptide product of interest can be virtually any peptide (as used herein, the term peptide includes peptides and proteins without distinction), regardless of its origin (eukaryotic, prokaryotic, viral, etc.), which can be recombinantly expressed, for example, a peptide antigen, such as a viral, bacterial, microbial, tumor antigen, etc., against which an immune response is to be induced in a subject (such as an animal, including humans); an enzyme, such as an enzyme intended to supplement a function in which an organism is deficient; a peptide drug intended to prevent or treat a pathological condition in a subject; an antibody or a functional fragment thereof; etc. Therefore, in a particular embodiment, said peptide product of interest is a peptide useful in vaccination, therapy or diagnosis, such as an epitope or antigenic determinant capable of inducing an immune response in a subject (e.g., animals, including humans) against diseases caused by viruses, bacteria, parasites or any other type of microorganism, or against tumor diseases, or is a peptide useful in therapy (prevention and/or treatment) of other pathologies, for example, heparin, a cell growth factor, an interleukin, an interferon, a protein of interaction with a membrane receptor, a peptide of interaction with a specific cell marker, etc.; in a specific embodiment, said product of interest comprises the tissue factor, or a functional variant thereof, or yeast-derived microvesicles containing the tissue factor or a functional variant thereof; as is known, tissue factor, or a functional variant thereof, which is lipidized, as well as said yeast-derived microvesicles containing the tissue factor or a functional variant thereof, are useful in the treatment of hemorrhages.

In another particular embodiment, said product of interest is a product of a non-peptide nature. Said product of interest of a non-peptide nature can be virtually any compound of a non-peptide nature, for example, a polynucleotide, a non-peptide antigen, such as allergens, viral, bacterial, microbial, tumor antigen, etc., against which an immune response is to be induced in a subject (such as an animal, including humans); a non-peptide drug intended to prevent or treat a pathological condition in a subject; a lipid with the capacity to interact with a molecule of interest; a sugar with the capacity to interact with a molecule of interest; etc. Therefore, in a particular embodiment, said non-peptide product of interest is a compound useful in vaccination, therapy or diagnosis, such as a non-peptide antigenic determinant capable of inducing an immune response in a subject (e.g., animals, including humans) against diseases caused by viruses, bacteria, parasites or any other type of microorganism, or against tumor diseases, or is a compound useful in the therapy (prevention and/or treatment) of pathologies of another type.

Alternatively, the product of interest can interact with the heterologous insert present in the capsid of the virus-like particle through a ligand.

As used herein, the term "ligand" relates to a molecule which is capable of interacting specifically and binding to the peptide insert present in the virus-like particles of the invention. The product of interest can be of a peptide (protein) nature or a non-peptide nature.

As used herein, the expression "capable of interacting specifically" or "specific interaction" describes the interaction between a first species and a second species characterized in that the nature of the binding is such that a an antibody, a receptor or a nucleic acid binding protein binds to its corresponding binding partner but does not substantially bind to other species. Likewise, as used herein, the term "binding" or "binding to" means the physical association between a first species and a second species, for example, the binding of a ligand by a receptor, the binding of an antigen by an antibody, the binding of a nucleic acid by a nucleic acid binding protein, etc.

The term "ligand" does not involve any particular size or another structural technical feature different from that of said ligand being capable of interacting and binding to the peptide insert present in the virus-like particle of the invention; therefore, said ligand contains a region of interaction with the specific binding domain which is present in the peptide insert contained in the virus-like particle of the invention. By way of illustration, the "peptide insert"-"ligand" pair acts as a specific binding pair, e.g., of the type of biotin-avidin, epitope-antibody or functional fragment thereof (e.g., a Fab or F(ab')₂ fragment, an scFv (single-chain antibody), a chimeric antibody, etc.), etc., in which each of said peptide insert and ligand form the members of said specific binding pair, each of them containing the binding domains or the corresponding regions of interaction; thus, depending on the peptide insert present in the virus-like particle of the invention the ligand which must be present in the product of interest will be chosen, and, vice versa, depending on the ligand which is to be bound to the virus-like particle of the invention, the peptide insert which must be present in said virus-like particle will be chosen.

In a particular embodiment, the binding of the ligand to the peptide insert is performed directly. In this case, for its use in the present invention, a product of interest with the capacity to bind to said peptide insert present in the virus-like particle of the invention will be used, such that said peptide insert and product of interest form a specific binding pair. By way of non-limiting illustration, when said peptide insert comprises an amino acid tag (e.g., a histidine tag (his-tag), an arginine tag (arg-tag), etc.); likewise when said peptide insert comprises a peptide epitope which can be recognized by an antibody (e.g., c-myc-tag, etc.), the product of interest is an antibody or a functional fragment thereof; etc.

Alternatively, the binding of said ligand to the peptide insert can be performed indirectly, i.e., by means of using a "linker", or molecule comprising, on one hand, a first region of interaction with the binding domain contained in the peptide insert present in the virus-like particle and, on the other hand, a second region of interaction (binding domain) with a region of interaction present in the ligand. Thus, the product of interest binds to the virus-like particle through a structure of the type: peptide insert-linker-ligand-product of interest. Illustrative non-limiting examples of this embodiment include the use of avidin or an analog thereof (linker) when the peptide insert present in the virus-like particle comprises a BAP; etc. As used herein, the term "avidin" includes any avidin, of a eukaryotic or prokaryotic origin, and its variants which maintain the capacity to bind to biotin; in a particular embodiment, the ligand present in the conjugate of the invention comprises a monomeric variant of avidin (mAV).

Therefore, in a particular embodiment, the invention provides a virus-like particle wherein the capsid protein comprises a heterologous peptide insert wherein said heterologous peptide comprises an amino acid sequence with the capacity to bind to a ligand, wherein said structural protein is IBDV pVP2, or a functional variant thereof. In a particular embodiment, said functional variant of IBDV pVP2 is the protein identified as IBDV pVP2_452 (SEQ ID NO:9) . In another specific embodiment, said peptide insert comprises a BAP. In an even more specific embodiment, said BAP comprises the amino acid sequence shown in SEQ ID NO:8.

In another embodiment, the peptide insert is located inside the P_{HI} loop of said IBDV pVP2 or functional variant thereof (IBDV pVP2_452), either between 2 contiguous amino acids or replacing one or more amino acids of said P_{HI} loop. In a specific embodiment, said peptide insert comprises a BAP; in an even more specific embodiment, said peptide insert comprises a BAP comprising the amino acid sequence shown in SEQ ID NO:8 and the insertion of said peptide insert in said capsid protein (IBDV pVP2, or a functional variant thereof, such as the protein identified as IBDV pVP2_452) is located in the P_{HI} loop of said identified protein.

The virus-like particle may or may not contain a product of interest bound to the peptide insert present in said virus-like particle through a ligand with the capacity to bind to said peptide insert, or alternatively, through a dual linker, i.e., with two different binding domains, one with the capacity to bind to said peptide insert and another one with the capacity to bind to said ligand to which the product of interest is bound.

### Method for obtaining recombinant infective virions

The authors of the present invention have shown that cells which comprise a polynucleotide formed by the non-translated 5' and 3' regions of the genome of an RNA virus requiring an RNA-dependent RNA polymerase for its replication and wherein said sequences flank a sequence encoding a gene of interest and which are infected with an RNA virus allow generating infective viruses which, in addition to the viral genome, comprise an additional genomic segment comprising a sequence of interest.

Without wishing to be bound by any theory, in the case of birnaviruses, it is believed that infective virions are produced as a result of the capacity of the birnavirus capsids to accumulate a variable number of genomic segments wherein that number can be greater than 2. As a result of that property, it is possible to isolate infectious particles from cells infected with IBDV which have more than one segment A or more than one segment B. If the infected cells comprise a recombinant birnaviral segment formed by a gene of interest flanked by the non-translated sequences of segment A or of segment B, said segment can be incorporated in the capsids together with a segment A and a segment B, thus giving rise to birnaviruses which retain the entire functionality thereof and which additionally comprise a gene of interest which will be expressed in the cells infected by the birnavirus. Thus, Example 4 of the present invention demonstrates how the infection with IBDV of cells previously transfected with a polynucleotide comprising the non-translated 5' and 3' regions of segment A of the IBDV genome and a sequence encoding a gene of interest gives rise to the formation of infective virions which are capable of infecting cells, giving rise to the expression of the protein of interest in said cells.

Thus, in another aspect, the invention relates to a method (hereinafter second method of the invention) for obtaining recombinant virions containing a heterologous genomic segment which comprises the steps of:
(i) contacting a cell with a polynucleotide under conditions suitable for the entry of said polynucleotide in the cell, wherein said polynucleotide comprises in the 5' to 3' direction
   (a) a first sequence comprising a non-translated region of a segment of the genomic RNA of an RNA virus requiring an RNA polymerase for its replication or a functionally equivalent variant thereof,
   (b) a second sequence encoding a product of therapeutic interest and
   (c) a third sequence comprising a non-translated region of a segment of the genomic RNA of said RNA virus or a functionally equivalent variant thereof
(ii) contacting said cell with an RNA virus from which the non-translated **regions** used in the polynucleotide used in step (i) are derived,
(iii) maintaining the cells under conditions suitable for the expression of the polynucleotide introduced in the cell in step (i) and for the expression of the genes of the RNA virus introduced in step (ii) and
(iv) recovering the recombinant virions from the culture obtained in step (iii)
wherein steps (i) and (ii) are carried out in any order or simultaneously.

Step (i) of the second method of the invention is essentially carried out in the same way as step (i) of the first method of the invention. In a preferred embodiment, the polynucleotide encoding the product of interest is under the operative control of a constitutive or inducible promoter. In an even more preferred embodiment, said promoter is the T7 RNA polymerase promoter, in which case it is necessary for the cell to express said RNA polymerase. Thus, in a preferred embodiment, the gene encoding the protein of interest is under the control of the T7 RNA polymerase promoter, forming part of a recombinant vaccinia virus which also comprises the gene encoding the T7 RNA polymerase.

Step (ii) comprises contacting the cells used in step (i) with an RNA virus, wherein said virus is of the same species as the virus which the non-translated 5' and 3' regions used for the generation of the polynucleotide used in step (i) are from. Thus, in the event that the polynucleotide used in step (i) contains the non-translated 5' and 3' regions of IBDV, step (ii) involves contacting said cells with IBDV. Evidently, the cells used in the second method of the invention must be permissive cells for the infection of the RNA virus. In the event that the method involves using IBDV, the invention contemplates using cells capable of being infected by IBDV in a native form (for example, QM7 cells), as well as cells selected for serving as a support for the infection by IBDV (for example, the chicken embryo fibroblasts described in EP1035203).

Step (iii) comprises maintaining the cells under conditions suitable for the expression of the viral proteins and for the replication of the polynucleotide introduced in step (i). Evidently, the replication of the polynucleotide introduced in step (i) is only possible by means of the activity of the RNA-dependent RNA polymerase which forms part of the virus itself, so it is necessary for a successful infection of the cells to have occurred in order for RNA polymerase activity to appear in the cells and in order for sufficient copies of the polynucleotide introduced in step (i) as well as of the polynucleotide or polynucleotides forming the genome of the RNA virus to be generated.

Finally, step (iv) includes recovering the infective virions from the cell culture used. In the same way as step (iv) of the first method of the invention, the virions can be recovered from the culture medium or from homogenates of the cells, in which case a prior step of lysis and homogenization of the cells and of centrifugation for removing the insoluble matter is necessary. The purification of the virus from the culture medium or from the cell homogenates is essentially carried out as has been described for the purification of transcriptionally active virus-like particles.

The second method of the invention allows generating recombinant infective virions which can be used as carriers suitable for addressing a gene of interest to a determined target cell. Thus, in another aspect, the invention relates to recombinant infective virions obtained by means of the second method of the invention. In a preferred embodiment, these virions are obtained according to a method in which the non-translated 5' and 3' sequences of the polynucleotide used in step (i) are from IBDV and the virus used in step (ii) is IBDV, such that the resulting virions comprise:
(i) a capsid formed by IBDV VP2, VP3 and VP4,
(ii) a variable number of copies of the RNA polymerase of IBDV,
(iii) at least one genomic segment A of IBDV,
(iv) at least one genomic segments B of IBDV and
(v) at least one genomic segment comprising
   a. a first sequence comprising a non-translated region of a segment of the genomic RNA of IBDV (A or B) or a functionally equivalent variant thereof,
   b. a second sequence encoding a heterologous product and
   c. a third sequence comprising a non-translated region of a segment of the genomic RNA of IBDV (A or B) or a functionally equivalent variant thereof

### In vitro uses of the virus-like particles and of the recombinant virions of the invention

The invention contemplates using the virus-like particles and the recombinant virions of the invention in an *in vitro* method for expressing the heterologous gene present in said particles or virions in a target cell. Thus, in another aspect, the invention relates to an *in vitro* method for expressing a protein of therapeutic interest in a cell which comprises contacting a virus-like particle of the invention or a recombinant virion of the invention with said cell.

To that end, in a first step, the method for expressing a polypeptide of interest in a cell comprises contacting said cell with a preparation of virus-like particles or of recombinant virions of the invention wherein the heterologous gene encodes the protein of interest and wherein the contacting is carried out under conditions suitable for the entry of the expression vector or of the genetic material contained in the viral particle in at least one cell of the culture.

As is evident for a person skilled in the art, the method can only be put into practice if the cell in which the gene is to be expressed is permissive for the infection by the RNA virus from which the particles or virions are derived, i.e., cells expressing the receptors of the RNA virus. However, in the event that the protein of interest is to be expressed in a cell which is not infected under natural conditions by the RNA virus, it is possible to use strategies known for the person skilled in the art such as the modification of the sequence of the capsid proteins to generate binding domains for the binding to proteins which appear on the surface of target cells. Alternatively, it is possible to introduce in the capsid protein reactive domains which can be derivatized with proteins of interest having binding sites on the surface of the target cell such as, for example, by means of the introduction of myc, FLAG, HA, Tat-PTD, hexahistidine, RGD epitopes, etc., as has been described in EP1849799 in the case of the SV40 VI protein, the introduction of the V5, HA and myc epitope in the case of the U1 protein of the tobacco mosaic virus (TMV) coat, the introduction of HIV-1 epitopes in the VP2 and VP3 proteins of the infectious pancreatic necrosis virus (IPNV), the introduction of CCR5 extracellular loop in the case of the L1 protein of the papillomavirus. The peptide insert capable of generating a target cell binding site in the virus-like particle or in the virion can be located in an internal region of the polypeptide forming the capsid, preferably in a region which, after the acquisition of the tertiary structure by the capsid protein, appears on the outside of the particle or of the virion. Alternatively, the peptide insert can be located at the N-terminus or at C-termini of the capsid protein.

In a second step, the culture obtained in the first step is maintained under conditions suitable for the expression in the cells of the gene of interest. During the performance of this second step, the culture is regularly evaluated until it is detected that the expression of the protein has reached the desired levels. This can be carried out by means of the detection in the culture of immunoreactive material using a specific antibody for the protein which is to be purified, mainly by means of a Western type blot method (J. Bacteriol. 1988; 170:3847).

Finally, in a third step, the protein of interest is recovered from the culture. The way to put this step into practice will depend on whether the protein is a secretable protein, in which case it will be recovered from the culture medium, or on whether the protein is an intracellular protein, in which case it will be necessary to separate the cells from the culture medium, break the cells and purify the protein from the cell lysate.

In the event that the polypeptide of interest is intracellularly expressed, step (iii) of the method of the invention requires separating the cells from the supernatant of the culture, usually by means of centrifugation or filtration, followed by the lysis of the cells. The host cells can be lysed using any conventional method including chemical/enzymatic cell lysis, mechanical lysis, lysis by thermal cycling, lysis by boiling, electrochemical lysis, lysis by electroporation and ultrasonic lysis. Once the cells are lysed, the sample is treated for the purpose of removing cell remains and unbroken cells, normally by means of centrifugation until a cell-free lysate containing the polypeptide of interest is obtained.

The cell-free lysate obtained as defined in the previous paragraph (if the polypeptide of interest is intracellularly expressed) or the supernatant of the cell culture (if the polypeptide of interest is secreted) is then subjected to one or more steps of protein purification for the purpose of isolating the polypeptide of interest. Suitable protein purification methods include but are not limited to size fractionation using molecular exclusion chromatography; ion exchange chromatography; affinity chromatography using, for example, monoclonal antibodies addressed to the polypeptide of interest, adsorption chromatography using non-specific supports, such as hydroxyapatite, silica, alumina, selective precipitation and the like. The fractions obtained during the previous purification processes are then assayed to detect the presence of the polypeptide of interest. The identification of the polypeptide of interest after the fractionation can be established by using several methods known in the art, including but not limited to SDS-PAGE assays, Western type blot and mass spectrometry for detecting the non-specific binding of antibodies including but not limited to radioimmunoassays, ELISA (Enzyme-linked immunosorbent assay), sandwich type immunoassays, immunoradiometric assays, gel diffusion precipitation reactions, immunodiffusion assays, *in* situ immunoassays, Western type blots, precipitation reactions, fluorescence polarization immunoassay (FPIA), nephelometric immunoassay (NIA), agglutination assays, complement fixation assays, immunofluorescence assays, protein A assays and immunoelectrophoresis assays and the like. Such methods are described in, for example, Clinical Immunology (Stites and Terr, eds., 7th ed. 1991), Methods in Cell Biology: Antibodies in Cell Biology, volume 37 (Asai, ed. 1993); and Harlow and Lane (Eds) Antibodies - A Laboratory Manual, (1988), Cold Spring Harbor Laboratory, N.Y.

In the event that the protein incorporates a detection/purification label (i.e., a sequence encoding a peptide with a known sequence and which is not present in native host cells which allows detecting and/or purifying the antigenic peptide), the protein can be purified from the medium or from the cell lysate by means of affinity for commercial molecules showing a high affinity for said labels. Suitable detection/purification labels include hexahistidines (metal chelate residue), affinity for hexa-hat glutathione GST (glutathione S-transferase), calmodulin binding peptide (CBP), streptomycin label, cellulose binding domain, maltose binding protein, S-peptide label, chitin binding label, immunoreactive epitopes, epitope labels, E2tag, HA epitope label, Myc epitope, FLAG epitope, AU1 and AU5 epitopes, Glu-Glu epitope, KT3 epitope, IRS epitope, Btag epitope, protein kinase C epitope, VSV epitope or any other label provided that the label does not affect the stability of the protein.

### Medical uses of the virus-like particles and of the recombinant virions of the invention

Both the transcriptionally active virus-like particles and the recombinant virions developed in the present invention can be used in different therapeutic uses. Thus, in another aspect, the invention relates to a virus-like particle of the invention or to a recombinant virion of the invention for its use in medicine. In another aspect, the invention relates to a pharmaceutical composition comprising a transcriptionally active virus-like particle or a recombinant virion according to the invention together with a pharmaceutically acceptable excipient. In a particular embodiment, said pharmaceutical composition is a (mono- or polyvalent) vaccine comprising a therapeutically effective amount of particles or of virions of the invention together with, optionally, one or more pharmaceutically acceptable adjuvants and/or excipients. Said vaccine can be used to protect animals (including humans) against diseases caused by pathogenic agents (for example, viruses, bacteria, parasites, etc.) or against tumor diseases. In another particular embodiment, said pharmaceutical composition is a composition intended for its use in gene therapy.

In the sense used in this description, the expression "therapeutically effective amount" relates to the amount of virus-like particles or recombinant virions of the invention, calculated to cause the desired effect and it will generally be determined, among other causes, by the typical characteristics of the particles or of the virions of the invention used and the therapeutic effect to be achieved.

The pharmaceutically acceptable excipients and adjuvants which can be used in the pharmaceutical compositions provided by this invention are known by the persons skilled in the art and are typically used in the preparation of pharmaceutical compositions. The pharmaceutical composition of the invention can be presented in any suitable dosage form, typically in the form of a suspension, and can be administered by any suitable route, for example, parenterally, etc., for which it will include the pharmaceutically acceptable excipients necessary for the formulation of the desired dosage form. The formulations can be of a single dose and can be prepared according to classic galenic methods. The excipients, carriers and auxiliary substances used must be pharmaceutically and pharmacologically tolerable, it must be possible to combine them with other components of the formulation and they must not exert any adverse effect on the treated subject. A review of the different pharmaceutical dosage forms of medicinal products and of the excipients necessary for obtaining them can be found, for example, in the "Tratado de Farmacia Galénica", C. Faulí i Trillo, 1993, Luzán 5, S.A. Ediciones, Madrid. The pharmaceutical composition object of the invention is prepared in the form of an aqueous solution or suspension, in a pharmaceutically acceptable diluent, such as saline, phosphate-buffered saline (PBS), or any other pharmaceutically acceptable diluent. Likewise, in a particular embodiment, the vaccine provided by this invention is given parenterally, for example, intraperitoneally, subcutaneously, etc.

By way of illustration, the dose of active ingredient (virus-like particles or recombinant virions of the invention) to be administered to a subject will be a therapeutically effective amount and can vary within a broad range. The pharmaceutical composition provided by this invention can be administered one or more times a day for preventive or therapeutic purposes. The dose of active ingredient to be administered will depend on a number of factors, including the characteristics of the product to be administered, such as, for example, its activity and biological half life, the concentration of the product in the pharmaceutical composition, the clinical condition of the subject, the severity of the pathology, the chosen pharmaceutical dosage form, etc.

The pharmaceutical composition provided by this invention can contain either a single type of virus-like particles or recombinant virions of the invention or two or more different virus-like particles of the invention together with one or more pharmaceutically acceptable carriers or excipients, either two or more recombinant virions of the invention or a combination of virus-like particles or recombinant virions of the invention.

If desired, the pharmaceutical composition provided by this invention can be used together with other drugs for the purpose of increasing the efficiency of the pharmaceutical composition provided by this invention, a combination therapy thus being generated. Said additional drugs can form part of the same pharmaceutical composition or, alternatively, they can be provided as a separate pharmaceutical composition for its administration at the same time (simultaneous administration) as the pharmaceutical composition of the invention or at different times (sequential administration) with respect to the administration of the pharmaceutical composition provided by this invention.

In addition, the virus-like particles or the recombinant virions of the invention can be contained in an inert medium, i.e., in a medium which does not substantially affect the stability thereof, for example, in a aqueous medium, such as saline, phosphate-buffered saline (PBS), etc. Therefore, in another aspect, the invention relates to a composition comprising a virus-like particle or a recombinant virion of the invention and an inert medium.

In a preferred embodiment, the virus-like particles of the invention or the recombinant virions of the invention are used for the development of a vaccine. Therefore, in another aspect, the invention relates to a vaccine comprising a virus-like particle of the invention or a recombinant virion of the invention. Evidently, the vaccines object of the invention can be used for the treatment of diseases caused by the viruses from which the virus-like particles of the invention or the recombinant virions of the invention are derived. For example, in the event that the virus-like particles of the invention or the recombinant virions are from IBDV, the vaccines can be used for the treatment of infectious bursal disease. These vaccines are efficient for generating an immune response in chickens, turkeys, ducks, ostriches, etc.,

Alternatively, it is possible to use the vaccines of the invention for the treatment of diseases that are not directly related to the viruses from which the particles and virions of the infection are derived. To that end, the particles and virions include a polynucleotide encoding an antigen against which immunity is to be generated. Thus, the administration of the particles and virions to a patient will cause the generation of an immune response against said antigen. It is also possible to prepare bifunctional vaccines in which an immune response is generated against the capsid proteins of the virus-like particle or of the recombinant virion and against the polypeptide encoded by the recombinant genome of said virus-like particle or of the recombinant virion. Thus, by means of choosing the suitable polynucleotide, the invention contemplates the use of the vaccines for the treatment of the infestations by ascarids, of aspergillosis, of botulism, of infestations by *Capillaria obsignata,* of infestations by *Heterakis gallinae,* of infections by mites of the type of *Trombicula splendens, Trombicula alfreddugesi,* and *Neoschongastia americana americanam,* the treatment of coccidiosis, infectious coryza, erysipelas, infections by E. *coli,* the fatty liver hemorrhagic syndrome, the treatment of the infections by *Pasteurella multocida,* avian cholera, avian influenza, fowl pox, salmonellosis, infections by *Syngamus* trachea, hexamitiasis, lymphoid leukosis, Marek's disease, moniliasis, mycoplasmosis, mycotoxicosis, necrotic enteritis, Newcastle disease, omphalitis, pullorum, bronchitis, ulcerative enteritis, avian encephalomyelitis, airsacculitis, bursal disease (gumboro) and the like.

In a preferred embodiment, the invention comprises the use of the virus-like particles of the invention or of the recombinant virions of the infection for the treatment of diseases in chickens. The virus-like particles of the invention or the recombinant virions of the invention can also be used as vaccines for inducing an immune response against the virus which has served as the basis for the generation of the particles or of the virions and wherein the heterologous gene encodes a polypeptide acting as an enhancer of said immune response. Examples of genes encoding polypeptides with immunostimulatory capacity include immunostimulatory cytokines such as IL-12, IL-2, IL-15, IL-18, IL-24, GM-CSF, TNF-α, ligand CD40, IFN-α, IFN-β, IFN-γ.

Evidently, the immune response of an individual to a particle or virion of the invention will only be possible if the particles or virions are capable of accessing the intracellular medium for the expression of the heterologous gene to occur. Therefore, if particles or virions comprising the native proteins of the RNA virus are used, they will only be useful in those organisms which are infected by said viruses in their natural state. Thus, the particles or virions derived from IBDV will exclusively be useful for the treatment of fowls. However, the invention contemplates particles or virions modified on their outside by means of the introduction of domains or regions allowing the interaction and subsequent internalization of the particles or virions in all those cells expressing receptors for said ligands. Thus, as has been previously described with respect to the *in vitro* methods of the invention, it is also applicable for the purpose of obtaining particles showing tropism for cells different from those which are infected by the particles or virions formed by the native viral proteins. Ligands which allow carrying the particles and virions of the invention to a target tissue include but are not limited to peptides, polypeptides, antibodies and multimeric proteins. Specific ligands include members of the TNF superfamily such as tumor necrosis factors (TNF-, TNF-b, capable of binding to the TNF receptors (APRIL, etc) lymphotoxins (LT-a, LT-b), Fas ligands which bind to the Fas antigen, CD40 and derivatives which bind to the CD40 receptor in B cells, CD30 and derivatives which bind to the CD30 receptor on neoplastic cells, CD27, NGF, OX_40, transferrin which binds to the transferrin receptor located in tumor cells, activated T cells, neural tissue cells, ApoB which binds to the LDL receptor in liver cells, alpha-2-macroglobulin, capable of binding to the LRP receptor in liver cells, alpha-I acid glycoprotein, which binds to the receptor of the asialoglycoproteins of the liver, peptides comprising mannose, which bind to the mannose receptor in macrophages, peptides containing the sialyl-Lewis-X antigen which bind to the ELAM-1 receptor in activated endothelial cells, CD34 which binds to the CD34 receptor in hematopoietic progenitor cells, ICAM-I which binds to LFA-1 (CD11b/CD18) or to the Mac-I (CD11a/CD18) receptor in macrophages, M-CSF which binds to the c-fms receptor in the macrophages of the spleen and of the bone marrow, the circumsporozoite protein which binds to the Plasmodium receptor in hepatocytes, VLA-3 which binds to the VCAM-I receptor in activated endothelial cells, HIV gp120 and the class II histocompatibility antigen which binds to the CD4 receptor on the surface of T cells, the region of binding to the LDL receptor of apolipoprotein E (ApoE), IGF-1 and IGF-II which bind to their respective receptors, interleukins 1 to 14 which bind to their respective receptors, folic acid which binds to folate receptors, peptides of the F3 family which bind to the surface of the angiogenic endothelium in tumor cells through the nucleolin of the cell surface (Christian et al., 2003, J. Cell Biol., 163:871-878), peptides of the LyP-1 family which bind to lymphatic and tumor cells (Laakonen, P. et *al.,* 2004, 101:9381-9386), peptides of the NGR family which bind to cells of the angiogenic endothelium in tumors through aminopeptidase N (Arap, W. et al., 1998, Science, 377-380), peptides of the RGR-4C family which bind to cells of the angiogenic endothelium in tumors through the αᵥβ3 and αᵥβ5 integrins (Arap, W. et al., 1998, Science, 279:377-380) and the like.

The person skilled in the art will understand that the aforementioned ligands can be incorporated in the surface of the particles or virions by means of the insertion of the sequence encoding them at the terminus or in a region of the polynucleotide encoding the structural proteins such that, after the expression, said ligand acquires its native conformation and appears exposed at the outside of the particle or virions. Alternatively, it is possible to modify the sequence of the capsid proteins by means of the inclusion of a sequence allowing its subsequent *in vitro* or *in vivo* binding with a suitably modified ligand of those mentioned above. For example, the invention contemplates the modification of the capsid with a biotin acceptor peptide or BAP which is biotinylated *in vitro* or *in vivo* by means of contacting said capsid comprising the BAP with a biotin ligase (for example, BirA) using the technology described by Predonzani et al. (BMC Biotechnology, 2008, 8:41) or in European patent application EP08380046.6. The biotinylated virions or particles can be contacted with a ligand of those mentioned above conjugated to streptavidin to give rise to complexes in which a particle or virion is conjugated to specific ligands of a determined cell.

The invention is illustrated below based on the following examples which are provided as a non-limiting illustration of the scope of the invention.

### Examples

### 1. Materials and Methods

### 1.1 Biological material

### 1.1.1 Prokaryotic cells

The E. coli strain DH5α (Raleigh et *al.* 2002) was used for the amplification of recombinant plasmids.

### 1.1.2 Eukaryotic cells

For the generation, amplification and expression of rVV, cultures of *Cercopithecus aethiops* (African green monkey) BSC40 cells (ATCC, CRL-2761), derived from the BSC1 line (ATCC, CCL-26) were used. For the amplification of IBDV, the QM7 cell line derived from muscle fibroblasts of *Coturnix japonica* (Japanese quail) (ATCC, CRL-1962) was used.

Both the cultures of QM7 cells and of BSC40 cells were grown and maintained in Dulbecco's Modified Eagle Medium (Dulbecco and Freeman 1959) supplemented with 10% FCS, penicillin (100 U/ml), streptomycin (100 µg/ml), gentamicin (50 µg/ml), fungizone (0.5 µg/ml) and non-essential amino acids.

### 1.1.3 Plasmids

### pT-ReGS A

The chemical synthesis of the recombinant gene referred to as Cas1, which is formed by the T7 phage promoter (nucleotides 25 to 41), the 5'UTR region of segment A of the IBDV virus (nucleotides 42 to 137, a fragment of the ORFs of said segment, specifically the initial ATGs both of ORF 1A (VP5) and of ORF 2A (Poly), an intermediate region, and the terminator of ORF 2A (Poly) (the entire set was referred to as Cas1 and is comprised between positions 138 to 573), the 3'UTR region of segment A of the IBDV virus (nucleotides 574 to 665), the hepatitis δ virus ribozyme sequence and the phage T7 terminator (nucleotides 666 to 891), all flanked by the Not I targets, was commissioned. The plasmid sequence is included in SEQ ID NO:10.

This recombinant gene was introduced in the pUC57 plasmid (Fermentas, Ref: #SD0171; GenBank: Y14837) by means of digestion with EcoRV, giving rise to the pCas1 plasmid (Figure 3) (SEQ ID NO:11) .

The pVOTE2 plasmid (Ward et al., 1995, Proc.Natl.Acad.Sci.USA, 92:6773-7) was used to introduce therein the Cas1 recombinant gene as an intermediate step for generating the corresponding recombinant vaccinia viruses.

The pHA-Cas1 plasmid (Figure 5 and SEQ ID NO:12) was generated by means of ligation of the pVOTE2 vector (previously described) digested with Not I and an insert extracted from the pCas1 plasmid by means of digestion also with Not I and which coincides with the previously described one.

The IBDV virus polyprotein (segment A) was cloned into the pFastBac1 plasmid to give rise to the pFastBac-Poly plasmid (Figure 7) described in Martinez-Torrecuadrada et al. (Virology, 2000, 278:322-331).

The pT-ReGS-A plasmid was generated starting from the pHA-Cas1 plasmid, which was cut with the SphI and PshAI enzymes to open the plasmid and was ligated with the fragment from the restriction of the pFast Bac-Poly plasmid (SEQ ID NO:13) with the sphI and pshAI enzymes. A cassette formed by the Vaccinia selection gene, GPT, in addition to the Early / Late Promoter thereof (Figure 8), was subsequently cloned into this vector. To that end, a fragment of the pVOTE plasmid was extracted by means of PCR both from the GPT gene and the E/L promoter including the ClaI targets and using the primers:
01 5'→ GCGCATCGATCATTTCGCGGGATCGAGATCC; (SEQ ID NO:14)
01 3' →GCGCATCGATAGAAAAATTAGCGACCGGAGATTG (SEQ ID NO:15)

The resulting fragment (SEQ ID NO:16) was cloned into a slightly modified pVOTE2 plasmid containing segment A of the IBDV virus, from 5'UTR to 3'UTR, the T7 phage promoter and the hepatitis δ virus ribozyme, the T7 phage terminator and the truncated sequences of the hemagglutinin gene. The resulting plasmid was referred to as pT-ReGS-A (SEQ ID NO:17).

### pTReGS B

The chemical synthesis of the recombinant gene referred to as Cas2 (SEQ ID NO: 18), formed by the T7 phage promoter, the 5'UTR region of segment B of the IBDV virus, a fragment of the ORF of said segment, specifically the initial ATG of ORF 1B (VP1), an intermediate region, and the terminator of ORF 1B (VP1) (the entire set was referred to as Cas2), the 3'UTR region of segment B of the IBDV virus, the hepatitis δ virus ribozyme sequence and the T7 phage terminator, all flanked by the Not I targets, was commissioned.

This recombinant gene was synthesized by Genescript Corp. (USA) and was introduced in the pUC57 plasmid (Fermentas, Ref: #SD0171; GenBank: Y14837) by means of digestion with EcoRV giving rise to the pCas2 plasmid (Figure 11) (SEQ ID NO:19).

The pVOTE2 plasmid (Ward et al., 1995, Proc.Natl.Acad.Sci.USA, 92:6773-7) was used to introduce therein the Cas2 recombinant gene as an intermediate step for generating the corresponding recombinant vaccinia viruses.

The pHA-Cas2 plasmid (SEQ ID NO:20) was generated by means of ligation of the pVOTE2 vector (previously described) digested with Not I and an insert extracted from the pCas2 plasmid by means of digestion also with Not I and which coincides with the one described in Figure 10.

The IBDV VP1 protein (segment B) was cloned into the pFastBac1 plasmid (Figure 6) to give rise to the pFastBac_VP1 plasmid, described in Maraver et al. (J.Virol., 2003, 77:6438-6449) .

The fragment extracted from the pFastBac-VP1 plasmid by means of digestion with SalI (SEQ ID NO:21) was cloned into pHA-Cas2 by means of digestion with SalI to open the plasmid and ligation with the fragment from the restriction of the pFast Bac-VP1 plasmid (SEQ ID NO:21) with SalI. The pT-ReGS-B plasmid was generated starting from the pHA-Cas2 plasmid. A cassette formed by the Vaccinia selection gene, GPT, in addition to the Early/Late Promoter thereof (SEQ ID NO 22:), was subsequently cloned into this vector. To that end, a fragment of the pVOTE plasmid was extracted by means of PCR both from the GPT gene and the E/L promoter including the ClaI targets and using the primers:
01 5' → GCGCATCGATCATTTCGCGGGATCGAGATCC; (SEQ ID NO:14)
01 3' → GCGCATCGATAGAAAAATTAGCGACCGGAGATTG (SEQ ID NO:15)

The resulting pT-ReGS-B plasmid (SEQ ID NO:22) is a slightly modified pVOTE2 plasmid containing segment A of the IBDV virus, from 5'UTR to 3'UTR, the T7 phage promoter and the hepatitis δ virus ribozyme, the T7 phage terminator and the truncated sequences of the hemagglutinin gene.

### pT-ReGS-AGFP

This vector was obtained from pT-ReGS-A by means of introducing an ORF of the EGFP for obtaining a vector having the UTRs (both 5' and 3') of segment A and which in turn expresses EGFP. The result is a vector which expresses two proteins:
- a first protein which is a fusion of GFP with the start of the IBDV polyprotein since the GFP was introduced in the ORF 2A (SEQ ID NO:23).
- a second protein which is a fusion of ORF1A and ORF2 (SEQ ID NO:24).

The vector was obtained by means of digestion of pT-ReGS-A with PshAI BglII. In addition, a fragment comprising the encoding region of EGFP was obtained by means of PCR from the pEGFP-C1 plasmid (Reference: Clontech GenBank Accession #: U55763) using the oligos:
01 5': CGCGCCCGGGATGAGCAAGGGCGAGGAGCTG (SEQ ID NO:25)
01 3': GCGCAGATCTGATTATCCGGACTTGTACAGCTCGTC (SEQ ID NO:26)

The PCR product (SEQ ID NO:27) was digested with SmaI and BglII. The resulting fragment was ligated to the pT-ReGS plasmid to give rise to the pT-ReGS-AGFP plasmid (SEQ ID NO:28).

### 1.1.4 Viruses

The Soroa strain (Lombardo et al. 1999) has been used for infections with IBDV. This viral strain, belonging to serotype I, was isolated from an infected chicken in the region of Soroa (Cuba) and adapted to grow in primary cultures of chicken embryo fibroblasts (CEF). Subsequently, the strain was adapted to grow in different mammal cells.

To generate the different recombinant vaccinia viruses (rVV), the rVV VT7LacOI has been used as the parent virus (Ward et al. 1995).

### 1.1.5 Antibodies

Polyclonal rabbit antisera against the VP1, VP2 and VP3 proteins described above (Lombardo et al. 1999; Sánchez and Rodriguez 1999), commercial antibodies against GFP (Roche) and the anti-p65 antibody described by Heljasvaara R et al. (J Virol., 2001, 75:5778-5795) have been used in the present work.

### 1.2 Handling and obtaining recombinant vectors

### 1.2.1 PCR

The reactions were carried out in a final volume of 50 µl with 0.2 mM dNTPs, 200 ng of each primer, 2 ng of template DNA, 20 mM Tris-HC1 pH 8.8, 10 nM KC1, 2 mM MgSO₄ 10 mM (NH)₄SO₂, 0.1% Triton X-100 and 2 U of DNA polymerase Vent (New England Biolabs). The following was used as the amplification protocol: 1 min at 94°C; 30 cycles of 40 sec at 92°C, 40 sec at 60°C and 1 min 30 sec at 75°C; 5 min at 75°C.

### 1.2.2 Ligation reactions of DNA fragments

The reactions were performed in a final volume of 15 µl in a buffer formed by 50 mM Tris-HCl pH 7.8, 10 mM MgCl₂, 10 mM DTT, 1 mM ATP, 25 µg/ml BSA; using 200 ng/ml of each DNA fragment and 200 U of T4 DNA ligase (New England Biolabs). The reactions were carried out for 16 h at 16°C.

### 1.2.3 Enzyme restriction reactions

The reactions were performed with enzymes from the companies New England Biolabs and Fermentas, following the supplier's indications.

### 1.2.4 Obtaining recombinant plasmids

See documents: Gener pT-ReGS A.doc; Gener pT-ReGS A-GFP.doc; Gener pT-ReGS B.doc

### 1.2.5 Obtaining recombinant vaccinia viruses

To generate the rVVs VT7/T-ReGS A, VT7/T-ReGS B and VT7/T-ReGS A-GFP, the plasmids previously described in sections 1.1.3 and 1.2.4. were used following the protocol previously described by Ward et al. 1995; Mulligan et *al.* 1981; Falkner et *al.* 1988; Earl, P. and B. Moss. Current Protocols in Molecular Biology 2003

### 1.3 Infections and expression of proteins

### 1.3.1 Production of rVV stocks

For the amplification of the different rVVs, confluent cultures of BSC40 cells were infected at an MOI of 0.01 pfu/cell of the corresponding rVV in a minimal volume. After 1 h of adsorption, the inoculum was removed and DMEM supplemented with 2% FCS was added. The culture supernatants were collected at 72 h.p.i. according to the cytopathic effect observed.

### 1.3.2 Production of IBDV stocks

For the amplification of IBDV virions, cultures of QM7 cells at a confluence of 50-60% were infected at an MOI of 0.05 pfu/cell of IBDV in a minimal volume. After 1 h of adsorption, the inoculum was removed and DMEM supplemented with 2% FCS was added. The culture supernatants were collected at 72-96 h.p.i. according to the cytopathic effect observed.

### 1.3.3 Titration of viral stocks

The viral titers of the rVV stocks, of the IBDV stocks and of the stocks of the different IBDV populations were determined by means of plaque forming assays. Monolayers of QM7 cells at a confluence of 50-60% in a minimal volume were infected with increasing dilutions of viruses in DMEM. After 1 h of absorption, the inoculum was removed and semisolid agar at a final concentration of 0.5% in DMEM medium supplemented with 2% FCS was added. At 48 h.p.i., the monolayers were fixed with 10% formaldehyde and stained with a 2% crystal violet aqueous solution. The titer was determined by means of lysis plate counting.

### 1.3.4 Infection with rVV

For the different infections performed, cultures of QM7 cells at a confluence of 50-60% were infected at an MOI of 2 pfu/cell of each of the rVVs used in a minimal volume. After 1 hour of adsorption, the inoculum was removed and DMEM supplemented with 2% FCS and 2 mM IPTG were added. Both the infected cells and their culture supernatants were collected at 72 h.p.i. and separated by means of centrifugation at 2500 rpm 5 min.

### 1.3.5 rVV + IBDV coinfection

For rVV+IBDV coinfections, cultures of QM7 cells at a confluence of 50-60% were infected at an MOI of 2 and 5 pfu/cell of each of the rVVs and IBDV, respectively, in a minimal volume. After 1 hour of adsorption, the inoculum was removed and DMEM supplemented with 2% FCS and 2 mM IPTG were added. Furthermore, in the indicated cases, the medium was further supplemented with rifampicin at a final concentration of 100 µg/ml. Both the infected cells and their culture supernatants were collected at 48 h.p.i. and separated by means of centrifugation at 2500 rpm 5 min.

### 1.3.6 Generation of intracellular and extracellular extracts

For the generation of extracellular extracts, the culture supernatants generated as previously described (sections 1.3.4 and 1.3.5) were mixed at a 1:5 (v:v) ratio with PEG 20% 3M NaCl and incubated with stirring overnight at 4°C. They were subsequently centrifuged at 3,000 r.p.m. (1,000xg) for 30 min and the resulting precipitates were resuspended in DMEM.

For the generation of intracellular extracts, the infected cells were resuspended in DMEM and lysed by means of three consecutive freezing-thawing cycles, followed by three 10-sec sonication cycles. Finally, the extracts were clarified by means of centrifugation at 3,000 r.p.m. (1,000xg) for 10 min.

Furthermore, in the indicated cases the intracellular or extracellular extracts were filtered through membranes with a pore size of 0.1 µm by means of using syringe filters (Millipore).

### 1.3.7 Transduction with transcriptionally active VLPs by means of intracellular and extracellular extracts

For the transduction with intracellular and extracellular extracts of cells coinfected with the different rVVs, cultures of QM7 cells at a confluence of 50-60% were incubated with a minimal volume of the corresponding extract (150 µl per well of M24 plate) for 1 h, after which DMEM supplemented with 2% FCS was added. In the indicated cases the medium was further supplemented with rifampicin at a final concentration of 100 µg/ml. At 72 h both the transduced cells and their culture supernatants were collected and separated by means of centrifugation at 2500 rpm 5 min.

### 1.3.8 Infection with recombinant IBDV virions by means of intracellular and extracellular extracts

For the infection with intracellular and extracellular extracts of cells coinfected with the different rVVs, cultures of QM7 cells at a confluence of 50-60% were incubated with a minimal volume of the corresponding extract (300 µl per well of M12 plate) for 1 h, after which DMEM supplemented with 2% FCS and, in the indicated cases, rifampicin at a final concentration of 100 µg/ml were added. At 72 h.p.i. both the transduced cells and their culture supernatants were collected and separated by means of centrifugation at 2500 rpm 5 min.

### 1.3.9 Reinfection with culture supernatants of transductions or infections with intracellular and extracellular extracts

Cultures of QM7 cells at a confluence of 50-60% were incubated with a minimal volume of a 1/10 dilution in DMEM of the culture supernatants generated as described in sections 1.3.7 and 1.3.8. After 1 h of adsorption, DMEM supplemented with 2% FCS and, in the indicated cases, rifampicin at a final concentration of 100 µg/ml were added. At 72 h.p.i. both the cells and their culture supernatants were collected and separated by means of centrifugation at 2500 rpm 5 min.

### 1.3.10 Purification of transcriptionally active VLPs

QM7 cells were coinfected as described in section 1.3.4 and were collected at 72 h.p.i. The cells were washed with PES buffer (50 mM PIPES pH 6.2, 150 mM NaCl, 20 mM CaCl₂) and were lysed in PES buffer supplemented with protease inhibitors (Complete Mini; Roche) by means of three freezing/thawing cycles followed by three 10-sec sonication cycles. The lysate was clarified by means of centrifugation at 3,000 r.p.m. (1,000xg) for 10 min in a 5624 tilting rotor (Hettich Zentrifugen). The supernatant obtained was loaded on a 25% sucrose cushion in PES and ultracentrifuged at 37,000 r.p.m. (170,000xg) for 2 h 30 min in an SW41 rotor (Beckman-Coulter Inc.). The resulting sediment was resuspended in PES buffer, centrifuged in a microfuge at 13,000 r.p.m. (16,000xg) for 1 min and the supernatant loaded on a 25%-50% linear sucrose gradient in PES which was ultracentrifuged at 40,000 r.p.m. (200,000xg) for 45 min in an SW41 rotor (Beckman-Coulter Inc.). The gradient was collected in 12 fractions which were concentrated 20 times by means of ultracentrifugation at 50,000 r.p.m. (240,000xg) for 2 h in an SW55 rotor (Beckman-Coulter Inc.). All the purification steps were performed at 4°C.

### 1.4 Analysis of samples

### 1.4.1 Electrophoretic analysis in denaturing polyacrylamide gels

For the electrophoretic resolution of proteins, the Laemmli electrophoresis method in SDS-polyacrylamide (SDS-PAGE) gels was followed. The analyzed samples were mixed with 5X denaturing buffer (62.5 mM Tris-HCl pH 6.8, 1% SDS, 0.06% bromophenol blue, 25% glycerol and 10 mM DTT), boiled for 3 min at 100°C, cooled 1 min at 4°C and loaded in 11% polyacrylamide gels (37.5:1). Said gels were subsequently electrotransferred to nitrocellulose membranes or stained with a solution of Coomassie blue at 2% in 10% methanol, 10% acetic acid.

### 1.4.2 Electrotransfer and immunodetection (Western Blot)

Once the electrophoretic separation was carried out by means of polyacrylamide gels, the latter were electrotransferred under semi-dry conditions for 45 min at 200 mA to nitrocellulose membranes (Protran, Schleicher & Schuell) after incubating both the gel and the membranes in 48 mM Tris buffer, 39 mM glycine, 0.0375% (w/v) SDS, 20% (v/v) methanol. The membranes were saturated in a blocking solution (5% powdered skimmed milk in phosphate buffer saline (PBS)) for 30 min at room temperature and subsequently incubated with the diluted corresponding primary antibody (anti-VP1 1:500, anti-anti-VP2 1:500; anti-VP3 1:500; anti-GFP 1:1000; anti p65 1:500) in a blocking solution for 1.5 h at room temperature. After three washings of 10 min with PBS, they were incubated for 1 h at room temperature with a goat anti-rabbit or mouse IgG antibody, as appropriate, conjugated with peroxidase (GE Healthcare and Sigma-Aldrich, respectively) diluted 1:5000 in blocking solution. The membrane was washed 3 times with PBS for 10 min and was developed by means of using the commercial ECL chemiluminescent system (GE Healthcare) or by means of adding 4-chloro-1-naphthol in the presence of 0.025% H₂O₂.

### 1.4.3 Electron microscopy

An electron microscopy grid with carbon-collodion previously ionized by means of ion discharge (K100X, Emitech) was deposited on a drop (2-5 µl) of the sample under study. The grid was incubated 3-5 min, washed 2 times in water and the excess liquid removed by means of contacting the edge of the grid with grade 1 filter paper (Whatman). The optimal concentration for transmission electron microscopy of the different IBDV assemblies analyzed was determined empirically by means of the serial dilution thereof in their corresponding buffer.

The grids on which the samples had previously been adsorbed were incubated on a drop of 2% (w/v) uranyl acetate for 1 min and the excess staining agent was removed by contacting the grid with grade 1 filter paper (Whatman). To view the sample, a JEOL 12000 EX II microscope stabilized at 100 kV was used and the images were recorded in electron micrographs (Kodak SO-163) at a nominal magnification of 40,000x, which were developed (Kodak D-19) for 8 min at room temperature.

### Example 1

### Generating transcriptionally active IBDV VLPs

The expression of the IBDV polyprotein by means of recombinant vaccinia viruses (rVVs) gives rise, after the correct folding of the viral polyprotein, to the accumulation of VLPs in the cytoplasm of the infected cells (Ferndndez-Arias et al. 1998, J Gene Virol 79:1047-1054;). Furthermore, the coinfection of this rVV with another one that expresses VP1 gives rise to the formation of VLPs incorporating the viral polymerase (Lombardo et al. 1999, J Virol. 73:6973-6983). For the purpose of generating transcriptionally active IBDV VLPs QM7 cells were coinfected with rVV viruses which express the viral polyprotein, the RNA-dependent RNA polymerase (VP1) and the A-GFP chimeric segment (VT7/Poly, VT7/VP1 and VT7/TReGS-A-GFP, respectively). At 72 h.p.i. the supernatants of the coinfected cells were precipitated with PEG, referred to as extracellular extract, and the cell pellets were lysed and clarified, referred to as intracellular extract. Subsequently, both the extracellular extract and the intracellular extract were filtered through cellulose membranes with a pore size of 0.1 µm for the purpose of removing the vaccinia virus particles.

For the purpose of analyzing the expression of the viral polyprotein and the chimeric form of GFP, the intra- and extracellular extracts were biochemically characterized by means of SDS-PAGE and WB with a polyclonal anti-VP2 serum and monoclonal anti-GFP antibody. Furthermore, the presence of vaccinia virus throughout the process was analyzed by means of SDS-PAGE and WB with a monoclonal anti-p65 antibody, a major structural protein of the vaccinia core, also referred to as P4a (Figure 1). The results obtained show the correct expression and processing of pVP2/VP2 from the IBDV polyprotein, as well as that of the chimeric A-GFP protein, as well as the presence of both proteins in the filtered extracts.

Once the expression was characterized, infections were carried out at a semi-preparative scale with the corresponding recombinant vaccinia and the resulting assemblies were purified by means of successive ultracentrifugations in a sucrose cushion (25%) and in a linear sucrose gradient (25-50%). The assemblies resulting from the rVV VT7/Poly + VT7/VP1 coinfection were purified in parallel as an internal control of the formation of VLPs, in which conditions the formation of VLPs incorporating the VP1 viral polymerase has previously been described (Lombardo et al. 1999, J Virol 73:6973-6983.). Furthermore, to evaluate the possible effect of the coexpression of a segment with the viral UTRs, the same process from the rVV VT7/Poly + VR7/VP1 + VT7/TReGS-A coinfection was carried out.

The fractions of the gradients were concentrated by ultracentrifugation and analyzed by means of SDS-PAGE and subsequent Coomassie blue staining or electrotransfer to nitrocellulose membranes and immunodetection with polyclonal anti-VP1, anti-VP2 and anti-VP3 sera (Figure 2). The analysis of the electrophoretic profile of the gradients shows that all the purifications have a maximum in the migration of assemblies located in the central fractions of the gradient (fractions 5 to 7). Furthermore, the immunodetection of the VP1, VP2 and VP3 viral proteins shows that the three proteins have a migration maximum in said fractions (Figure 2D-F).

The purified fractions of the coinfections with rVV were structurally characterized by means of electron microscopy and negative staining (Figure 3). Both in the VT7/Poly + VT7/VP1 coinfection and in which they have further been infected with VT7/TReGS-A or VT7/TReGS-A-GFP, the major assemblies, which migrate in the central fractions of the gradient, correspond with VLPs of 65-70 nm in diameter that are morphologically similar to the T=13 capsids of the virion (Böttcher et al. 1997, J Virol. 71:325-330.) and to the previously described VLPs (Fernández-Arias et al. 1998, J Gene Virol 79:1047-1054; Lombardo et al. 1999, J Virol. 73:6973-6983).

These results demonstrate that the coexpression of VT7/Poly and VR7/VP1 in the presence of the chimeric construct VT7/TReGS-A-GFP gives rise to the formation of VLPs with a size and morphology similar to the IBDV T=13 capsids and therefore that the coexpression of said chimeric segment does not interfere with the assembly thereof.

### Example 2

### Transduction of QM7 cells with extracts from the VT7/Poly + VT7/VP1 + VT7/TReGS-A-GFP coinfection

To characterize the transcriptional activity of the generated VLPs, cultures of QM7 cells were transduced with intracellular or extracellular extracts of cells coinfected with rVV VT7/Poly + VT7/VP1 + VT7/TReGS-A-GFP generated as described in the previous section and subsequently filtered through cellulose membranes with a pore size of 0.1 µm. Furthermore, said transductions were performed in the presence or absence of rifampicin, an inhibitor of vaccinia virus assembly (Heller et al. 1969), for the purpose of removing possible residual viral particles.

At 72 h the cell cultures transduced were collected and the expression of the different proteins was analyzed by means of SDS-PAGE, electrotransfer to nitrocellulose membranes and immunodetection against the IBDV proteins (VP1, VP2 and VP3), the A-GFP chimeric protein or the vaccinia virus structural protein, p65 (Figure 4). The results obtained show high levels of expression of the chimeric protein containing GFP, as well as medium levels of expression of the major IBDV proteins, VP2 and VP3. These results indicate that the VLPs generated are capable of encapsidating the A-GFP chimeric segment and that said segment is capable of being expressed when said VLPs are used to transfect new cell cultures. However, to eliminate the possibility that the observed expression is from residual vaccinia viruses that have not been removed by means of filtering or blocked by rifampicin, new cultures of QM7 cells were infected with the supernatants of the transduction previously described. At 72 h.p.i., the cells were collected and analyzed by means of SDS-PAGE, electrotransfer to nitrocellulose membranes and immunodetection (Figure 5). The result obtained indicates that while in the cells contacted with supernatants from transductions with extracts without filtering proliferation of vaccinia virus occurs, in the cells contacted with those from transductions with filtered extracts there is no indication (or presence of p65, or cytopathic effect) of viral proliferation. These results imply that the expression of the A-GFP chimeric protein observed in the transduced cells must correspond with transcriptionally active VLPs and not with the presence of rVV therein.

### Example 3

### Generating recombinant IBDV virions

For the purpose of generating IBDV virions which in addition to carrying the two viral segments, A and B, incorporate an exogenous segment, cultures of QM7 cells were coinfected with IBDV and the rVV VT7/TReGS-A-GFP. Furthermore, these coinfections were performed in the presence and absence of rifampicin for the purpose of inhibiting the assembly of new vaccinia virus particles. At 48 h.p.i. the coinfections were collected and the supernatants were precipitated with PEG, referred to as extracellular extract, while the cell pellets were lysed and clarified, referred to as intracellular extract. Said extracts were biochemically characterized by means of SDS-PAGE, electrotransfer to nitrocellulose membranes and immunodetection (Figure 6). The results obtained show that under these conditions, IBDV has similar levels of pVP2/VP2 in the presence and absence of rifampicin whereas the levels of expression of the p65 vaccinia protein clearly drop in the presence of this drug. On the other hand, the coinfection of IBDV with vaccinia virus does not affect the levels of expression of VP2. Finally, an apparently specific increase occurs in the expression of the A-GFP chimeric protein with respect to the one detected in cells infected with VT7/TReGS-A-GFP in the presence of rifampicin. This fact suggests the possibility that the A-GFP recombinant segment is being recognized by the IBDV polymerase, VP1, and potentially encapsidated in the viral particles.

The previously generated extracellular and intracellular extracts were filtered through cellulose membranes with a pore size of 0.1 µm for the purpose of removing the vaccinia virus particles and used to infect cultures of QM7 cells in the presence and absence of rifampicin. At 72 h the cell cultures were collected and the expression of the different proteins was analyzed by means of SDS-PAGE, electrotransfer to nitrocellulose membranes and immunodetection against VP2, the A-GFP chimeric protein and p65 (Figure 7). The results obtained show that in the absence of p65, and therefore of vaccinia virus, high levels of expression of the A-GFP chimeric protein are obtained from the incubation with extracts of cells coinfected with IBDV and VT7/TReGS-A-GFP in the absence of rifampicin.

Nevertheless, to confirm the absence of vaccinia virus, new cultures of QM7 cells were treated with the supernatants of the infection with intracellular extracts and at 72 h.p.i., the cells were collected and analyzed by means of SDS-PAGE, electrotransfer to nitrocellulose membranes and immunodetection (Figure 8). The analysis of these cell extracts allows observing the expression and processing of IBDV proteins (VP1, VP2 and VP3), as well as A-GFP proteins in the absence of p65, used as a marker of the presence of vaccinia virus in the extracts.

## Claims

1. A polynucleotide comprising in the 5' to 3' direction
(a) a first sequence comprising a non-translated region of a segment of the genomic RNA of a birnavirus or a functionally equivalent variant thereof,
(b) a second sequence encoding a heterologous protein and
(c) a third sequence comprising a non-translated region of a segment of the genomic RNA of said birnavirus or a functionally equivalent variant thereof
wherein the first and/or the third sequence allow or allows the encapsidation of said polynucleotide inside a viral particle.

2. A polynucleotide according to claim 1, wherein the birnavirus is IBDV.

3. A polynucleotide according to claim 2, wherein sequence (a) comprises the non-translated 5' region of segment A of the IBDV genome and/or wherein sequence (c) comprises the non-translated 3' region of segment A of the IBDV genome.

4. A method for obtaining transcriptionally active virus-like particles which comprises the steps of:
(i) contacting a cell with a first polynucleotide under conditions suitable for the entry of said polynucleotide in the cell, wherein said polynucleotide comprises in the 5' to 3' direction
(a) a first sequence comprising a non-translated region of a segment of the genomic RNA of an RNA virus requiring an RNA polymerase for its replication or a functionally equivalent variant thereof,
(b) a second sequence encoding a heterologous product of therapeutic interest and
(c) a third sequence comprising a non-translated region of a segment of the genomic RNA of said RNA virus or a functionally equivalent variant thereof
(ii) contacting said cell with one or several additional polynucleotides under conditions suitable for the entry of said polynucleotide or said polynucleotides in the cell and wherein said polynucleotide or said polynucleotides comprise sequences encoding the RNA polymerase of said RNA virus from which the non-translated sequence forming part of the first polynucleotide is derived sequences encoding all the proteins necessary for the encapsidation of said RNA virus,
(iii) maintaining the cells under conditions suitable for the expression of the polynucleotides introduced in the cell in steps (i) and (ii) and
(iv) recovering the virus-like particles from the culture obtained in step (iii)
wherein steps (i) and (ii) are carried out in any order or simultaneously.

5. A method according to claim 4, wherein the birnavirus is IBDV.

6. A virus-like particle obtained by means of a method according to any of claims 1 to 5.

7. A method for obtaining recombinant virions containing a heterologous genomic segment which comprises the steps of:
(i) contacting a cell with a polynucleotide under conditions suitable for the entry of said polynucleotide in the cell, wherein said polynucleotide comprises in the 5' to 3' direction
(a) a first sequence comprising a non-translated region of a segment of the genomic RNA of an RNA virus requiring an RNA polymerase for its replication or a functionally equivalent variant thereof,
(b) a second sequence encoding a heterologous product and
(c) a third sequence comprising a non-translated region of a segment of the genomic RNA of said RNA virus or a functionally equivalent variant thereof
(ii) contacting said cell with an RNA virus from which the non-translated regions used in the polynucleotide used in step (i) are derived,
(iii) maintaining the cells under conditions suitable for the expression of the polynucleotide introduced in the cell in step (i) and for the expression of the genes of the RNA virus introduced in step (ii) and
(iv) recovering the recombinant virions from the culture obtained in step (iii)
wherein steps (i) and (ii) are carried out in any order or simultaneously.

8. A method according to claim 7, wherein the birnavirus is IBDV.

9. A method according to claims 7 or 8, wherein the first sequence of the polynucleotide used in step (i) comprises the non-translated 5' region of segment A of the IBDV genome and/or the third sequence of the polynucleotide used in step (i) comprises the non-translated 3' region of segment A of the IBDV genome.

10. A recombinant virion obtained by means of a method according to any of claims 7 to 9.

11. An *in vitro* method for expressing a protein of therapeutic interest in a cell which comprises contacting a virus-like particle according to any of claims 6 or a recombinant virion according to claim 10 with said cell.

12. A virus-like particle according to any of claims 6 or a recombinant virion according to claim 10 for its use in medicine.

13. A vaccine comprising a virus-like particle according to claim 6 or a recombinant virion according to claim 10.

14. A virus-like particle according to claim 6 or a recombinant virion according to claim 10, wherein the gene of therapeutic interest encodes an antigen of a pathogenic organism.

15. A virus-like particle or a recombinant virion according to claim 14 for its use in the treatment of an infectious disease caused by the pathogenic organism from which the antigen is derived.
